# EUROPEAN PATENT APPLICATION

(11) **EP 4 640 707 A1**
(43) Date of publication of application: **29.10.2025**
(21) Application number: 23906104.7
(22) Date of filing: 22.12.2023
(51) Int. Cl.: C07K 16/28, C07K 19/00, C12N 15/13, C12N 15/63, G01N 33/577, A61K 39/395, A61K 47/68, A61P 35/00

(54) **ANTI-CMET ANTIBODY, ANTIBODY-DRUG CONJUGATE, AND PREPARATION METHOD THEREFOR AND USE THEREOF**

(30) Priority: 23.12.2022 CN 202211663573
(71) Applicant: Medilink Therapeutics (Suzhou) Co., Ltd., Suzhou, Jiangsu 215000 (CN)
(72) Inventor: XIAO, Liang, Suzhou, Jiangsu 215000 (CN); XUE, Tongtong, Suzhou, Jiangsu 215000 (CN); CAI, Jiaqiang, Suzhou, Jiangsu 215000 (CN)
(74) Representative: Mewburn Ellis LLP
(86) International application number: PCT/CN2023/141081
(87) International publication number: WO 2024/131949

(57) **Abstract**

The present disclosure provides an antibody targeting c-Met or an antigen-binding fragment thereof, an antibody-drug conjugate, and a use thereof in treating cancer. The present disclosure further provides a nucleotide, a polynucleotide combination, an expression vector, and an expression vector combination which all encode the c-Met antibody or the antigen-binding fragment thereof, a pharmaceutical composition comprising the c-Met antibody or the antigen-binding fragment thereof, and the antibody-drug conjugate, and a use of the nucleotide, the polynucleotide combination, the expression vector, the expression vector combination, and the pharmaceutical composition in the preparation of a drug for treating or preventing cancer.

## Description

### TECHNICAL FIELD

This application belongs to the field of pharmaceutical technology, and relates to a variety of antibodies, antibody-drug conjugates, and preparation methods thereof, as well as their use in prophylaxis and/or treatment of diseases related to abnormal cell activities, including but not limited to prophylaxis and/or treatment of tumor diseases.

### BACKGROUND ART

cMET, a tyrosine kinase receptor expressed on a cell membrane, binds to a ligand HGF via a Sema domain, thereby resulting in a downstream phosphorylation cascade reaction and ultimately promoting proliferation of cells. Currently, there are mainly three classes of c-Met kinase target inhibitors: HGF and c-Met biological antagonists, HGF and c-Met antibodies, and small molecular c-Met inhibitors. Existing clinical results show that the efficacy of antibodies directly against the HGF and c-Met, or the small molecular c-Met inhibitors is not ideal.

It is difficult to develop therapeutic antibodies against MET since antibodies that compete for HGF binding usually cause MET receptor dimerization and thus serve as agonists (Prat M, et al. J Cell Sci 1998; 111 (Pt 2), 237-247). Onartuzumab is the first anti-c-Met antibody developed and is derived from humanization development of a c-Met agonistic antibody 5D5.

Chemotherapy using cytotoxicity is once a standard therapy for cancer, but cytotoxin molecules with high killing potency would kill normal cells, causing serious toxic side effects. Targeted anti-tumor drugs have become a hot topic in the field of current tumor research for having both targeting ability and anti-tumor activity. However, the targeted drugs often produce large toxic side effects due to target selectivity problems, limiting therapeutic effects of the targeted drugs. Biological macromolecular drugs, such as antibodies or antibody fragments, although have high targeting ability, are limited in therapeutic effect on solid tumors or have no therapeutic effect at all. ADC, a conjugate of an antibody and a small molecular drug, combines the targeting effect of the antibody and the activity of a bioactive molecule, to become a biologic missile with very promising efficacy and safety advantages. The antibody guides the ADC to bind to a target cell, and is then internalized by the cell, and the small molecular drug is released in the cell through enzymatic cleavage under the action of a specific enzyme to treat the disease.

Therefore, it is crucial to screen a monoclonal antibody that has high affinity, specifically recognizes the cMet, and effectively blocks the HGF binding and downstream signaling pathways for subsequent ADC drug development. For a C-Met target, the development of differentiated, betterquality, and safer antibodies or ADC drugs can provide tumor patients with broader and better drug options, and also has a broad market prospect.

### SUMMARY OF THE INVENTION

The present disclosure provides an antibody targeting c-MET or antigen-binding fragment thereof, an antibody-drug conjugate, and use thereof in treatment of cancers. The present disclosure further provides an expression vector, an expression vector combination, a nucleotide and a polynucleotide combination encoding the foregoing c-MET antibody or antigen-binding fragment thereof, a pharmaceutical composition comprising the foregoing c-MET antibody or antigen-binding fragment thereof and the antibody-drug conjugate, applications thereof in the preparation of a medicament for treatment or prophylaxis of cancers.

According to a first aspect, the present disclosure provides an antibody targeting c-MET or antigen-binding fragment thereof, and specifically, the present disclosure provides an anti-c-MET antibody or antigen-binding fragment thereof. In some implementations, the c-MET is human c-MET.

In some implementations, the antibody or antigen-binding fragment thereof comprises 3 complementarity determining regions (HCDRs), the HCDR1, the HCDR2, and the HCDR3, from a heavy chain variable region; and/or 3 complementarity determining regions (LCDRs), the LCDR1, the LCDR2, and the LCDR3, from a light chain variable region. In some implementations, the antibody or antigen-binding fragment thereof comprises the heavy chain variable region and/or the light chain variable region. In some implementations, the heavy chain variable region comprises 3 complementarity determining regions (CDRs), the HCDR1, the HCDR2, and the HCDR3, from the heavy chain variable region. In some implementations, the light chain variable region comprises 3 complementarity determining regions (CDRs), the LCDR1, the LCDR2, and the LCDR3, from the light chain variable region.

In some implementations, the CDRs of the heavy chain variable region and/or the CDRs of the light chain variable region have sequences identical to the sequences of the CDRs of a sequence-defined antibody or have 1, 2, or 3 amino acid substitutions compared to the CDRs of the sequence-defined antibody, wherein the sequence-defined antibody comprises:
(1) the amino acid sequence of a heavy chain variable region set forth in SEQ ID NO: 2, 4, 6, 8, 10, or 12; and/or
(2) the amino acid sequence of a light chain variable region set forth in SEQ ID NO: 1, 3, 5, 7, 9, or 11.

In some implementations, the 3 complementarity determining regions (HCDRs), the HCDR1, the HCDR2, and the HCDR3, from the heavy chain variable region of the present disclosure are selected from:
(i) the three complementarity determining regions, the HCDR1, the HCDR2, and the HCDR3, comprised in the VH set forth in SEQ ID NO: 2, 4, 6, 8, 10, or 12, or
(ii) a sequence comprising at least one and no more than 5, 4, 3, 2, or 1 amino acid change(s) (preferably amino acid substitutions, and preferably conservative substitutions) on the three HCDRs relative to the sequence of any one of (i), wherein
preferably, the HCDRs are determined according to the definition scheme of AbM, Chothia, Kabat, Contact or IMGT.

In some implementations, the 3 complementarity determining regions (LCDRs), the LCDR1, the LCDR2, and the LCDR3, from the light chain variable region of the present disclosure are selected from
(i) the three complementarity determining regions, the LCDR1, the LCDR2, and the LCDR3, comprised in the VL set forth in SEQ ID NO: 1, 3, 5, 7, 9, or 11,
   or
(ii) a sequence comprising at least one and no more than 5, 4, 3, 2, or 1 amino acid change(s) (preferably amino acid substitutions, and preferably conservative substitutions) on the three LCDRs relative to the sequence of any one of (i), wherein
preferably, the LCDRs are determined according to the definition scheme of AbM, Chothia, Kabat, Contact or IMGT.

In some implementations, the anti-c-MET antibody or antigen-binding fragment thereof according to any one of the above comprises the HCDR1, the HCDR2, and the HCDR3 comprised in the heavy chain variable region set forth in SEQ ID NO: 10; and the LCDR1, the LCDR2, and the LCDR3 comprised in the light chain variable region set forth in SEQ ID NO: 9.

In some implementations, the anti-c-MET antibody according to any one of the above comprises the HCDR1, the HCDR2, and the HCDR3 comprised in the heavy chain variable region set forth in SEQ ID NO: 12; and the LCDR1, the LCDR2, and the LCDR3 comprised in the light chain variable region set forth in SEQ ID NO: 11.

In some implementations, the anti-c-MET antibody according to any one of the above comprises the HCDR1, the HCDR2, and the HCDR3 comprised in the heavy chain variable region set forth in SEQ ID NO: 2; and the LCDR1, the LCDR2, and the LCDR3 comprised in the light chain variable region set forth in SEQ ID NO: 1.

In some implementations, the anti-c-MET antibody according to any one of the above comprises the HCDR1, the HCDR2, and the HCDR3 comprised in the heavy chain variable region set forth in SEQ ID NO: 4; and the LCDR1, the LCDR2, and the LCDR3 comprised in the light chain variable region set forth in SEQ ID NO: 3.

In some implementations, the anti-c-MET antibody according to any one of the above comprises the HCDR1, the HCDR2, and the HCDR3 comprised in the heavy chain variable region set forth in SEQ ID NO: 6; and the LCDR1, the LCDR2, and the LCDR3 comprised in the light chain variable region set forth in SEQ ID NO: 5.

In some implementations, the anti-c-MET antibody according to any one of the above comprises the HCDR1, the HCDR2, and the HCDR3 comprised in the heavy chain variable region set forth in SEQ ID NO: 8; and the LCDR1, the LCDR2, and the LCDR3 comprised in the light chain variable region set forth in SEQ ID NO: 7.

In specific implementations, the HCDRs 1-3 and the LCDRs 1-3 are identified according to different assay methods or systems or definition schemes.

In specific implementations, the HCDRs 1-3 and the LCDRs 1-3 are identified according to the definition scheme of AbM, Chothia, Kabat, Contact, or IMGT. In specific implementations, the complementarity determining regions HCDRs 1-3 and LCDRs 1-3 are determined according to the definition scheme of Chothia, Kabat, or IMGT based on the corresponding heavy chain and light chain variable regions, as set forth in sequences and a specific information table thereof.

In some implementations, the HCDR1 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| HCDR1 (definition scheme of IMGT) | HCDR1 (definition scheme of Kabat) | HCDR1 (definition scheme of Chothia) |
|---|---|---|
| SEQ ID NO: 13 | SEQ ID NO: 16 | SEQ ID NO: 25 |
| SEQ ID NO: 26 | SEQ ID NO: 29 | SEQ ID NO: 54 |
| SEQ ID NO: 39 | SEQ ID NO: 42 | SEQ ID NO: 62 |

In some implementations, the HCDR2 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| HCDR2 (definition scheme of IMGT) | HCDR2 (definition scheme of Kabat) | HCDR2 (definition scheme of Chothia) |
|---|---|---|
| SEQ ID NO: 14 | SEQ ID NO: 17 | SEQ ID NO: 53 |
| SEQ ID NO:27 | SEQ ID NO: 30 | SEQ ID NO: 55 |
| SEQ ID NO:40 | SEQ ID NO: 43 | SEQ ID NO: 60 |
| | SEQ ID NO:18 | |
| | SEQ ID NO:31 | |

In some implementations, the HCDR2 determined based on the definition scheme of IMGT, Kabat, or Chothia comprises or consists of the amino acid sequence set forth in SEQ ID NO: 38 or 61, or comprises an amino acid sequence having one, two, or three changes (preferably amino acid substitutions, and preferably conservative substitutions) compared to the amino acid sequence set forth in SEQ ID NO: 38 or 61.

In some implementations, the HCDR3 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| HCDR3 (definition scheme of IMGT) | HCDR3 (definition scheme of Kabat) | HCDR3 (definition scheme of Chothia) |
|---|---|---|
| SEQ ID NO:15 | SEQ ID NO: 19 | SEQ ID NO: 19 |
| SEQ ID NO:28 | SEQ ID NO: 32 | SEQ ID NO: 32 |
| SEQ ID NO:41 | SEQ ID NO: 44 | SEQ ID NO: 44 |

In some implementations, the LCDR1 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| LCDR1 (definition scheme of IMGT) | LCDR1 (definition scheme of Kabat) | LCDR1 (definition scheme of Chothia) |
|---|---|---|
| SEQ ID NO:20 | SEQ ID NO: 23 | SEQ ID NO: 23 |
| SEQ ID NO:33 | SEQ ID NO: 36 | SEQ ID NO: 36 |
| SEQ ID NO:45 | SEQ ID NO: 48 | SEQ ID NO: 48 |

In some implementations, the LCDR2 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| LCDR2 (definition scheme of IMGT) | LCDR2 (definition scheme of Kabat) | LCDR2 (definition scheme of Chothia) |
|---|---|---|
| SEQ ID NO:21 | SEQ ID NO: 24 | SEQ ID NO: 24 |
| SEQ ID NO:34 | SEQ ID NO: 37 | SEQ ID NO: 37 |
| SEQ ID NO:46 | SEQ ID NO: 49 | SEQ ID NO: 49 |

In some implementations, the LCDR3 determined based on the definition scheme of IMGT, Kabat, or Chothia comprises or consists of the amino acid sequences set forth in the following SEQ ID NOs, or comprises amino acid sequences having one, two, or three changes (preferably amino acid substitutions, and preferably conservative substitutions) compared to the amino acid sequences set forth in the following SEQ ID NOs:

| LCDR3 (definition scheme of IMGT) | LCDR3 (definition scheme of Kabat) | LCDR3 (definition scheme of Chothia) |
|---|---|---|
| SEQ ID NO:22 | SEQ ID NO: 22 | SEQ ID NO: 22 |
| SEQ ID NO:35 | SEQ ID NO: 35 | SEQ ID NO: 35 |
| SEQ ID NO:47 | SEQ ID NO: 50 | SEQ ID NO: 50 |

In some implementations, the anti-c-MET antibody according to any one of the above comprises a heavy chain variable region and/or a light chain variable region, wherein:
a. the heavy chain variable region comprises the HCDR1, the HCDR2, and the HCDR3, which comprise the amino acid sequences set forth in SEQ ID NO: 13, SEQ ID NO: 14, and SEQ ID NO: 15, respectively, or consist of the amino acid sequences set forth in SEQ ID NO: 13, SEQ ID NO: 14, and SEQ ID NO: 15, respectively; and the light chain variable region comprises the LCDR1, the LCDR2, and the LCDR3, which comprise the amino acid sequences set forth in SEQ ID NO: 20, SEQ ID NO: 21, and SEQ ID NO: 22, respectively, or consist of the amino acid sequences set forth in SEQ ID NO: 20, SEQ ID NO: 21, and SEQ ID NO: 22, respectively;
preferably, the foregoing HCDRs 1-3 and LCDRs 1-3 are determined according to the definition scheme of IMGT.

In some implementations, the anti-c-MET antibody according to any one of the above comprises a heavy chain variable region and/or a light chain variable region, wherein:
b. the heavy chain variable region comprises the HCDR1, the HCDR2, and the HCDR3, which comprise the amino acid sequences set forth in SEQ ID NO: 16, SEQ ID NO: 38 (QIRLKSLNYATHYAXSVKG, wherein X may be any amino acid, such as E or Q), and SEQ ID NO: 19, respectively, or consist of the amino acid sequences set forth in SEQ ID NO: 16, SEQ ID NO: 38 (QIRLKSLNYATHYAXSVKG, wherein X may be any amino acid, such as E or Q), and SEQ ID NO: 19, respectively; and the light chain variable region comprises the LCDR1, the LCDR2, and the LCDR3, which comprise the amino acid sequences set forth in SEQ ID NO: 23, SEQ ID NO: 24, and SEQ ID NO: 22, respectively, or consist of the amino acid sequences set forth in SEQ ID NO: 23, SEQ ID NO: 24, and SEQ ID NO: 22, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
c. the heavy chain variable region comprises the HCDR1, the HCDR2, and the HCDR3, which comprise the amino acid sequences set forth in SEQ ID NO: 16, SEQ ID NO: 17, and SEQ ID NO: 19, respectively, or consist of the amino acid sequences set forth in SEQ ID NO: 16, SEQ ID NO: 17, and SEQ ID NO: 19, respectively; and the light chain variable region comprises the LCDR1, the LCDR2, and the LCDR3, which comprise the amino acid sequences set forth in SEQ ID NO: 23, SEQ ID NO: 24, and SEQ ID NO: 22, respectively, or consist of the amino acid sequences set forth in SEQ ID NO: 23, SEQ ID NO: 24, and SEQ ID NO: 22, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
d. the heavy chain variable region comprises the HCDR1, the HCDR2, and the HCDR3, which comprise the amino acid sequences set forth in SEQ ID NO: 16, SEQ ID NO: 18, and SEQ ID NO: 19, respectively, or consist of the amino acid sequences set forth in SEQ ID NO: 16, SEQ ID NO: 18, and SEQ ID NO: 19, respectively; and the light chain variable region comprises the LCDR1, the LCDR2, and the LCDR3, which comprise the amino acid sequences set forth in SEQ ID NO: 23, SEQ ID NO: 24, and SEQ ID NO: 22, respectively, or consist of the amino acid sequences set forth in SEQ ID NO: 23, SEQ ID NO: 24, and SEQ ID NO: 22, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
e. the heavy chain variable region comprises the HCDR1, the HCDR2, and the HCDR3, which comprise the amino acid sequences set forth in SEQ ID NO: 25, SEQ ID NO: 53, and SEQ ID NO: 19, respectively, or consist of the amino acid sequences set forth in SEQ ID NO: 25, SEQ ID NO: 53, and SEQ ID NO: 19, respectively; and the light chain variable region comprises the amino acid sequences set forth in SEQ ID NO: 23, SEQ ID NO: 24, and SEQ ID NO: 22, respectively, or consist of the amino acid sequences set forth in SEQ ID NO: 23, SEQ ID NO: 24, and SEQ ID NO: 22, respectively;
preferably, the foregoing HCDRs 1-3 and LCDRs 1-3 are determined according to the definition scheme of Chothia.

In some implementations, the anti-c-MET antibody according to any one of the above comprises a heavy chain variable region and/or a light chain variable region, wherein:
f. the heavy chain variable region comprises the HCDR1, the HCDR2, and the HCDR3, which comprise the amino acid sequences set forth in SEQ ID NO: 26, SEQ ID NO: 27, and SEQ ID NO: 28, respectively, or consist of the amino acid sequences set forth in SEQ ID NO: 26, SEQ ID NO: 27, and SEQ ID NO: 28, respectively; and the light chain variable region comprises the LCDR1, the LCDR2, and the LCDR3, which comprise the amino acid sequences set forth in SEQ ID NO: 33, SEQ ID NO: 34, and SEQ ID NO: 35, respectively, or consist of the amino acid sequences set forth in SEQ ID NO: 33, SEQ ID NO: 34, and SEQ ID NO: 35, respectively;
preferably, the foregoing HCDRs 1-3 and LCDRs 1-3 are determined according to the definition scheme of IMGT.

In some implementations, the anti-c-MET antibody according to any one of the above comprises a heavy chain variable region and/or a light chain variable region, wherein:
g. the heavy chain variable region comprises the HCDR1, the HCDR2, and the HCDR3, which comprise the amino acid sequences set forth in SEQ ID NO: 29, SEQ ID NO: 61 (WIFPGSGNTKYX1X2KFX3G, wherein X1, X2, and X3 may be any amino acid, for example, X1 is I or S; X2 is E or Q, and/or X3 is K or Q), and SEQ ID NO: 32, respectively, or consist of the amino acid sequences set forth in SEQ ID NO: 29, SEQ ID NO: 61 (WIFPGSGNTKYX1X2KFX3G, wherein X1, X2, and X3 may be any amino acid, for example, X1 is I or S; X2 is E or Q, and/or X3 is K or Q), and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively, or consist of the amino acid sequences set forth in SEQ ID NO: 36, SEQ ID NO: 37, and SEQ ID NO: 35, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
h. the heavy chain variable region comprises the HCDR1, the HCDR2, and the HCDR3, which comprise the amino acid sequences set forth in SEQ ID NO: 29, SEQ ID NO: 30, and SEQ ID NO: 32, respectively, or consist of the amino acid sequences set forth in SEQ ID NO: 29, SEQ ID NO: 30, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively, or consist of the amino acid sequences set forth in SEQ ID NO: 36, SEQ ID NO: 37, and SEQ ID NO: 35, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
i. the heavy chain variable region comprises the HCDR1, the HCDR2, and the HCDR3, which comprise the amino acid sequences set forth in SEQ ID NO: 29, SEQ ID NO: 31, and SEQ ID NO: 32, respectively, or consist of the amino acid sequences set forth in SEQ ID NO: 29, SEQ ID NO: 31, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively, or consist of the amino acid sequences set forth in SEQ ID NO: 36, SEQ ID NO: 37, and SEQ ID NO: 35, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
j. the heavy chain variable region comprises the HCDR1, the HCDR2, and the HCDR3, which comprise the amino acid sequences set forth in SEQ ID NO: 54, SEQ ID NO: 55, and SEQ ID NO: 32, respectively, or consist of the amino acid sequences set forth in SEQ ID NO: 54, SEQ ID NO: 55, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively, or consist of the amino acid sequences set forth in SEQ ID NO: 36, SEQ ID NO: 37, and SEQ ID NO: 35, respectively;
preferably, the foregoing HCDRs 1-3 and LCDRs 1-3 are determined according to the definition scheme of Chothia.

In some implementations, the anti-c-MET antibody according to any one of the above comprises a heavy chain variable region and/or a light chain variable region, wherein:
k. the heavy chain variable region comprises the HCDR1, the HCDR2, and the HCDR3, which comprise the amino acid sequences set forth in SEQ ID NO: 39, SEQ ID NO: 40, and SEQ ID NO: 41, respectively, or consist of the amino acid sequences set forth in SEQ ID NO: 39, SEQ ID NO: 40, and SEQ ID NO: 41, respectively; and the light chain variable region comprises the LCDR1, the LCDR2, and the LCDR3, which comprise the amino acid sequences set forth in SEQ ID NO: 45, SEQ ID NO: 46, and SEQ ID NO: 47, respectively, or consist of the amino acid sequences set forth in SEQ ID NO: 45, SEQ ID NO: 46, and SEQ ID NO: 47, respectively;
preferably, the foregoing HCDRs 1-3 and LCDRs 1-3 are determined according to the definition scheme of IMGT.

In some implementations, the anti-c-MET antibody according to any one of the above comprises a heavy chain variable region and/or a light chain variable region, wherein:
l. the heavy chain variable region comprises the HCDR1, the HCDR2, and the HCDR3, which comprise the amino acid sequences set forth in SEQ ID NO: 42, SEQ ID NO: 43, and SEQ ID NO: 44, respectively, or consist of the amino acid sequences set forth in SEQ ID NO: 42, SEQ ID NO: 43, and SEQ ID NO: 44, respectively; and the light chain variable region comprises the LCDR1, the LCDR2, and the LCDR3, which comprise the amino acid sequences set forth in SEQ ID NO: 48, SEQ ID NO: 49, and SEQ ID NO: 50, respectively, or consist of the amino acid sequences set forth in SEQ ID NO: 48, SEQ ID NO: 49, and SEQ ID NO: 50, respectively;
preferably, the foregoing HCDRs 1-3 and LCDRs 1-3 are determined according to the definition scheme of Kabat.

In some implementations, the anti-c-MET antibody according to any one of the above comprises a heavy chain variable region and/or a light chain variable region, wherein:
m. the heavy chain variable region comprises the HCDR1, the HCDR2, and the HCDR3, which comprise the amino acid sequences set forth in SEQ ID NO: 62, SEQ ID NO: 60, and SEQ ID NO: 44, respectively, or consist of the amino acid sequences set forth in SEQ ID NO: 62, SEQ ID NO: 60, and SEQ ID NO: 44, respectively; and the light chain variable region comprises the LCDR1, the LCDR2, and the LCDR3, which comprise the amino acid sequences set forth in SEQ ID NO: 48, SEQ ID NO: 49, and SEQ ID NO: 50, respectively, or consist of the amino acid sequences set forth in SEQ ID NO: 48, SEQ ID NO: 49, and SEQ ID NO: 50, respectively;
preferably, the foregoing HCDRs 1-3 and LCDRs 1-3 are determined according to the definition scheme of Chothia.

In some implementations, the 3 heavy chain CDRs comprised in the anti-c-MET antibody or antigen-binding fragment thereof involved in the present disclosure comprise a total of no more than 5 amino acid changes (for example, amino acid substitutions, and preferably conservative substitutions) compared to each group of the foregoing HCDRs 1-3. In some implementations, the 3 light chain CDRs comprised in the anti-c-MET antibody or antigen-binding fragment thereof involved in the present disclosure comprise a total of no more than 5 amino acid changes (for example, amino acid substitutions, and preferably conservative substitutions) compared to each group of the foregoing LCDRs 1-3.

The anti-c-MET antibody or antigen-binding fragment thereof according to any one of the above comprises the heavy chain variable region and the light chain variable region, wherein 4, 3, 2, or 1 CDR(s) of 6 CDRs of the HCDRs 1-3 and the LCDRs 1-3 comprised in the heavy chain variable region and the light chain variable region is/are subjected to 1, 2, or 3 amino acid substitutions. In some implementations, the substitutions are conservative substitutions.

In some implementations, the anti-c-MET antibody according to any one of the above is a humanized antibody, murine-derived antibody, or chimeric antibody. In some implementations, the antibody of the present disclosure is humanized. Humanization can be realized by substituting one or more amino acid residues, especially the sequence of the frame region, in the heavy chain variable region and light chain variable region of a non-humanized natural antibody, with the residues at the corresponding position of the variable region of a conventional antibody. Methods for humanizing antibodies are known in the art. Typically, humanized substitutions are performed in a manner in which binding-beneficial properties of the antibody are retained. Experiments for determining the biological properties of the humanized antibody, such as a binding affinity, to determine and select appropriate humanized residue mutations or combinations of mutations, are well known in the art.

In some implementations, the anti-c-MET antibody according to any one of the above is a monoclonal antibody. In some implementations, the antigen-binding fragment of the c-MET antibody is selected from Fab, Fab', Fab'-SH, F(ab')2, Fv, or single-chain Fv (scFv).

In some implementations, the heavy chain variable region of the present disclosure
(i) comprises or consists of the amino acid sequence having at least 90%, 91%, 92%, 93%, 94%, 95%, 96%, 97%, 98%, or 99% identity to the amino acid sequence selected from SEQ ID NO: 2, 4, 6, 8, 10, or 12; or
(ii) comprises or consists of the amino acid sequence selected from SEQ ID NO: 2, 4, 6, 8, 10, or 12; or
(iii) comprises or consists of the amino acid sequence having 1 or more (preferably no more than 10, and more preferably no more than 5, 4, 3, 2, 1) amino acid changes (preferably amino acid substitutions, and more preferably amino acid conservative substitutions) compared to the amino acid sequence selected from SEQ ID NO: 2, 4, 6, 8, 10, or 12, and preferably, the amino acid changes do not occur in the CDRs.

In some implementations, the light chain variable region of the present disclosure
(i) comprises or consists of an amino acid sequence having at least 90%, 91%, 92%, 93%, 94%, 95%, 96%, 97%, 98%, or 99% identity to an amino acid sequence selected from SEQ ID NO: 1, 3, 5, 7, 9, or 11; or
(ii) comprises or consists of the amino acid sequence selected from SEQ ID NO: 1, 3, 5, 7, 9, or 11; or
(iii) comprises or consists of the amino acid sequence having 1 or more (preferably no more than 10, and more preferably no more than 5, 4, 3, 2, 1) amino acid changes (preferably amino acid substitutions, and more preferably amino acid conservative substitutions) compared to the amino acid sequence selected from SEQ ID NO: 1, 3, 5, 7, 9, or 11, and preferably, the amino acid changes do not occur in the CDRs.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 10, and/or the light chain variable region has at least 90%, 91%, 92%, 93%, 94%, 95%, 96%, 97%, 98%, 99%, or 100% sequence identity to SEQ ID NO: 9.

In some implementations, the anti-c-MET antibody according to any one of the above comprises the heavy chain variable region and the light chain variable region, wherein the heavy chain variable region comprises or consists of the amino acid sequence set forth in SEQ ID NO: 10; and the light chain variable region comprises or consists of the amino acid sequence set forth in SEQ ID NO: 9.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 12, and/or the light chain variable region has at least 90%, 91%, 92%, 93%, 94%, 95%, 96%, 97%, 98%, 99%, or 100% sequence identity to SEQ ID NO: 11.

In some implementations, the anti-c-MET antibody according to any one of the above comprises the heavy chain variable region and/or the light chain variable region, wherein the heavy chain variable region comprises or consists of the amino acid sequence set forth in SEQ ID NO: 12; and the light chain variable region comprises or consists of the amino acid sequence set forth in SEQ ID NO: 11.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 2, and/or the light chain variable region has at least 90%, 91%, 92%, 93%, 94%, 95%, 96%, 97%, 98%, 99%, or 100% sequence identity to SEQ ID NO: 1.

In some implementations, the anti-c-MET antibody according to any one of the above comprises the heavy chain variable region and/or the light chain variable region, wherein the heavy chain variable region comprises or consists of the amino acid sequence set forth in SEQ ID NO: 2; and the light chain variable region comprises or consists of the amino acid sequence set forth in SEQ ID NO: 1.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 4, and/or the light chain variable region has at least 90%, 91%, 92%, 93%, 94%, 95%, 96%, 97%, 98%, 99%, or 100% sequence identity to SEQ ID NO: 3.

In some implementations, the anti-c-MET antibody according to any one of the above comprises the heavy chain variable region and/or the light chain variable region, wherein the heavy chain variable region comprises or consists of the amino acid sequence set forth in SEQ ID NO: 4; and the light chain variable region comprises or consists of the amino acid sequence set forth in SEQ ID NO: 3.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 6, and/or the light chain variable region has at least 90%, 91%, 92%, 93%, 94%, 95%, 96%, 97%, 98%, 99%, or 100% sequence identity to SEQ ID NO: 5.

In some implementations, the anti-c-MET antibody according to any one of the above comprises the heavy chain variable region and/or the light chain variable region, wherein the heavy chain variable region comprises or consists of the amino acid sequence set forth in SEQ ID NO: 6; and the light chain variable region comprises or consists of the amino acid sequence set forth in SEQ ID NO: 5.

In some implementations, the anti-c-MET antibody according to any one of the above comprises the heavy chain variable region and/or the light chain variable region, wherein the heavy chain variable region has at least 90%, 91%, 92%, 93%, 94%, 95%, 96%, 97%, 98%, 99%, or 100% sequence identity to SEQ ID NO: 8, and/or the light chain variable region has at least 90%, 91%, 92%, 93%, 94%, 95%, 96%, 97%, 98%, 99%, or 100% sequence identity to SEQ ID NO: 7.

In some implementations, the anti-c-MET antibody according to any one of the above comprises the heavy chain variable region and/or the light chain variable region, wherein the heavy chain variable region comprises or consists of the amino acid sequence set forth in SEQ ID NO: 8; and the light chain variable region comprises or consists of the amino acid sequence set forth in SEQ ID NO: 7.

In some implementations, the anti-c-MET antibody according to any one of the above further comprises an antibody heavy chain constant region. In some implementations, the anti-c-MET antibody according to any one of the above further comprises an antibody light chain constant region. In some implementations, the anti-c-MET antibody according to any one of the above further comprises the antibody heavy chain constant region and light chain constant region. In some implementations, the heavy chain constant region is selected from a human IgG1, IgG2, IgG3, or IgG4 constant region. In some implementations, the light chain constant region is selected from a human antibody κ or λ chain constant region.

In some preferred implementations, the antibody heavy chain constant region of the present disclosure:
(i) comprises or consists of the amino acid sequence having at least 85%, 90%, 91%, 92%, 93%, 94%, 95%, 96%, 97%, 98%, or 99% identity to the amino acid sequence of SEQ ID NO: 51;
(ii) comprises or consists of the amino acid sequence of SEQ ID NO: 51; or
(iii) comprises or consists of the amino acid sequence having 1 or more (preferably no more than 20 or 10, and more preferably no more than 5, 4, 3, 2, 1) amino acid changes (preferably amino acid substitutions, and more preferably amino acid conservative substitutions) compared to the amino acid sequence of SEQ ID NO: 51.

In some implementations, the amino acid changes occur in the Fc region.

In some implementations, the antibody light chain constant region of the present disclosure:
(i) comprises or consists of the amino acid sequence having at least 85%, 90%, 91%, 92%, 93%, 94%, 95%, 96%, 97%, 98%, or 99% identity to the amino acid sequence of SEQ ID NO: 52;
(ii) comprises or consists of the amino acid sequence of SEQ ID NO: 52; or
(iii) comprises or consists of the amino acid sequence having 1 or more (preferably no more than 20 or 10, and more preferably no more than 5, 4, 3, 2, 1) amino acid changes (preferably amino acid substitutions, and more preferably amino acid conservative substitutions) compared to the amino acid sequence of SEQ ID NO: 52.

In some implementations, the antibody comprises the heavy chain constant region and the light chain constant region, the heavy chain constant region comprises or consists of the amino acid sequence set forth in SEQ ID NO: 51, and the light chain constant region comprises or consists of the amino acid sequence set forth in SEQ ID NO: 52.

In some implementations, the anti-c-MET antibody according to any one of the above further comprises an antibody heavy chain. In some implementations, the anti-c-MET antibody according to any one of the above further comprises an antibody light chain. In some implementations, the anti-c-MET antibody according to any one of the above further comprises the antibody heavy chain and light chain.

In some preferred implementations, the antibody heavy chain of the present disclosure:
(i) comprises or consists of the amino acid sequence having at least 85%, 90%, 91%, 92%, 93%, 94%, 95%, 96%, 97%, 98%, or 99% identity to the amino acid sequence selected from SEQ ID NO: 56 or 58;
(ii) comprises or consists of the amino acid sequence selected from SEQ ID NO: 56 or 58; or
(iii) comprises or consists of the amino acid sequence having 1 or more (preferably no more than 20 or 10, and more preferably no more than 5, 4, 3, 2, 1) amino acid changes (preferably amino acid substitutions, and more preferably amino acid conservative substitutions) compared to the amino acid sequence selected from SEQ ID NO: 56 or 58.

In some implementations, the antibody light chain of the present disclosure:
(i) comprises or consists of the amino acid sequence having at least 85%, 90%, 91%, 92%, 93%, 94%, 95%, 96%, 97%, 98%, or 99% identity to the amino acid sequence selected from SEQ ID NO: 57 or 59;
(ii) comprises or consists of the amino acid sequence selected from SEQ ID NO: 57 or 59; or
(iii) comprises or consists of the amino acid sequence having 1 or more (preferably no more than 20 or 10, and more preferably no more than 5, 4, 3, 2, 1) amino acid changes (preferably amino acid substitutions, and more preferably amino acid conservative substitutions) compared to the amino acid sequence selected from SEQ ID NO: 57 or 59.

In some implementations, the anti-c-MET antibody according to any one of the above comprises:
a heavy chain having at least 85%, 90%, 91%, 92%, 93%, 94%, 95%, 96%, 97%, 98%, 99%, or 100% sequence identity to SEQ ID NO: 56, and/or a light chain having at least 85%, 90%, 91%, 92%, 93%, 94%, 95%, 96%, 97%, 98%, 99%, or 100% sequence identity to SEQ ID NO: 57.

In some implementations, the anti-c-MET antibody according to any one of the above comprises a heavy chain and a light chain, the heavy chain comprises or consists of the amino acid sequence set forth in SEQ ID NO: 56, and the light chain comprises or consists of the amino acid sequence set forth in SEQ ID NO:57.

In some implementations, the anti-c-MET antibody according to any one of the above comprises:
a heavy chain having at least 85%, 90%, 91%, 92%, 93%, 94%, 95%, 96%, 97%, 98%, 99%, or 100% sequence identity to SEQ ID NO: 58, and/or a light chain having at least 85%, 90%, 91%, 92%, 93%, 94%, 95%, 96%, 97%, 98%, 99%, or 100% sequence identity to SEQ ID NO: 59.

In some implementations, the anti-c-MET antibody according to any one of the above comprises a heavy chain and a light chain, the heavy chain comprises or consists of the amino acid sequence set forth in SEQ ID NO: 58, and the light chain comprises or consists of the amino acid sequence set forth in SEQ ID NO:59.

In some implementations, the present disclosure further provides an anti-c-MET antibody or antigen-binding fragment thereof, wherein the antibody competitively binds to human c-MET with the anti-c-MET antibody according to any one of the above. As defined herein, the antibody that competitively, with a reference antibody, binds to the antigen thereof is an antibody that blocks more than 50%, 60%, 70%, 80%, 90%, or 95% of binding of the reference antibody to the antigen thereof in a competition assay. Conversely, the reference antibody blocks more than 50%, 60%, 70%, 80%, 90%, or 95% of binding of the antibody to the antigen thereof in the competition assay. Various types of competitive binding assays can be used to determine whether one antibody competes with another, such as: a direct or indirect solid-phase radioimmunoassay (RIA), a direct or indirect solid-phase enzyme immunoassay (EIA), a sandwich competition assay, biological optical interferometry (such as Fortebio), or surface plasmon resonance (Biacore).

In some implementations, the anti-c-MET antibody or antigen-binding fragment thereof competitively binds to the human c-MET with a 45A5G10-Hz antibody.

In some implementations, the anti-c-MET antibody or antigen-binding fragment thereof competitively binds to the human c-MET with a 55A10G6-Hz antibody.

In some implementations, the anti-c-MET antibody or antigen-binding fragment thereof competitively binds to the human c-MET simultaneously with the 55A10G6-Hz antibody and the 45A5G10-Hz antibody.

In an implementation of the present disclosure, the amino acid changes described herein comprise substitutions, insertions, or deletions of amino acids. In some implementations, the amino acid changes are conservative changes. For to a polypeptide sequence, the "conservative changes" comprise substitutions, deletions, or additions to the polypeptide sequence that do not substantially change the desired functional activity of the polypeptide sequence.

Preferably, the amino acid changes described herein are amino acid substitutions, and preferably conservative substitutions. The conservative substitution means that an amino acid is substituted with another amino acid within the same class, for example, an acidic amino acid is substituted with another acidic amino acid, a basic amino acid is substituted with another basic amino acid, or a neutral amino acid is substituted with another neutral amino acid. For example, the conservative substitution often results in a substitution of an amino acid with a chemically similar amino acid. Conservative substitution tables providing functionally similar amino acids are well known in the art. The following is a list of 8 groups of amino acids comprising mutually conservative substitutions: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M). In some implementations, the term "conservative sequence change" is used to refer to an amino acid modification that does not significantly affect or change the binding characteristics of the target antigen of the antibody molecule or binding protein molecule of the present disclosure comprising the amino acid sequence. For example, a conservatively modified variant retains at least 80%, 85%, 90%, 95%, 98%, 99% or higher, such as 100-110% or higher, binding affinity to the target antigen relative to the parent antibody or binding protein.

In a preferred implementation, the amino acid change described in the present disclosure occurs in regions except for the CDRs (such as in the FR). More preferably, the amino acid change described in the present disclosure occurs in a region except for the heavy chain variable region and/or except for the light chain variable region.

In certain implementations, the substitution occurs in the CDRs of the antibody. Generally, the obtained variant will have modifications (such as improvements) in certain biological property aspects (such as increased affinity) relative to the parent antibody and/or will have substantially retained certain biological properties of the parent antibody.

In certain implementations, it may be required to create cysteine engineered antibodies, for example, "thioMAbs", in which one or more residues of an antibody are substituted with cysteine residues.

In certain implementations, the antibody provided herein may be further modified to contain other non-protein moieties that are known in the art and readily available. Moieties suitable for derivatization of the antibody comprise but are not limited to water soluble polymers.

In certain implementations, the antibody provided by the present disclosure is a multispecific antibody, for example, a bispecific antibody, a trispecific antibody, or a tetraspecific antibody.

According to a second aspect, the present disclosure provides a multispecific binding molecule, such as a multispecific antibody, comprising the foregoing anti-c-MET antibody or antigen-binding fragment thereof. In certain preferred implementations, the multispecific antibody is a bispecific antibody, a trispecific antibody, or a tetraspecific antibody.

According to a third aspect, in some implementations, the present disclosure further provides a nucleic acid molecule, encoding the nucleic acid of the anti-c-MET antibody or fragment thereof, or any heavy chain or light chain according to any one of the above.

For example, the nucleic acid of the present disclosure comprises a nucleic acid encoding the amino acid sequence set forth in any one selected from SEQ ID NOs: 1-12 and 56-59, or a nucleic acid encoding the amino acid sequence having at least 85%, 90%, 91%, 92%, 93%, 94%, 95%, 96%, 97%, 98%, or 99 % identity to the amino acid sequence set forth in any one of SEQ ID NOs: 1-12 and 56-59. As will be apparent to those skilled in the art, each antibody or polypeptide amino acid sequence can be encoded by a plurality of nucleic acid sequences because of codon degeneracy. The nucleic acid sequence encoding the molecule of the present disclosure can be generated using methods well known in the art, such as by *de novo* solid-phase DNA synthesis, or by PCR amplification.

The heavy chain and/or the light chain of the antibody molecule of the present disclosure can be fused at the N-terminus to a secretory signal peptide, and/or a tag peptide that facilitates purification, for production and purification.

The present disclosure further relates to a vector comprising the nucleic acid, such as an expression vector, such as a eukaryotic expression vector. The vectors comprise, but are not limited to, viruses, plasmids, cosmids, λ phages, or yeast artificial chromosomes (YAC). In an implementation, the vector is a pTT5 vector, such as pTT5-mFc and pTT5-hFc vectors.

The present disclosure further provides a host cell, comprising the nucleic acid molecule or the vector according to any one of the above. In another implementation, the host cell is selected from yeast cells, mammalian cells (such as CHO cells (such as CHO-S or CHO-K) or 293 cells (such as HEK293E or HEK293 cells)), or other cells suitable for preparation of the antibody or fragment thereof. In an implementation, the host cell is prokaryotic, such as a bacterium, such as E. *coli.*

Polynucleotides encoding the polypeptide chain of the antibody of the present invention can be inserted into one or more vectors for further cloning and/or expression in the host cell. Methods which are well known to those skilled in the art can be used to construct expression vectors. Once an expression vector for expression comprising one or more nucleic acid molecules of the present invention has been prepared, the expression vector can be transfected or introduced into a suitable host cell. A variety of techniques can be used to achieve this objective, for example, protoplast fusion, calcium phosphate precipitation, electroporation, retroviral transduction, viral transfection, gene gun, liposome-based transfection, or other conventional techniques.

According to a fourth aspect, the present disclosure provides a method for preparing an anti-c-MET antibody or fragment thereof (preferably an antigen-binding fragment), wherein the method comprises culturing the host cell under conditions suitable for expression of a nucleic acid encoding the antibody, fragment thereof (preferably the antigen-binding fragment), or either or both chains thereof, and optionally isolating the antibody or fragment thereof (preferably the antigen-binding fragment). In a certain implementation, the method further comprises recovering the anti-c-MET antibody or fragment thereof (preferably the antigen-binding fragment) from the host cell.

Antibodies prepared as described herein can be purified by known existing techniques such as high performance liquid chromatography, ion exchange chromatography, gel electrophoresis, affinity chromatography such as Protein A, and size exclusion chromatography. The actual conditions used to purify a particular protein further depend on factors such as net charge, hydrophobicity, and hydrophilicity, and are apparent to those skilled in the art.

According to a fifth aspect, in some implementations, the present disclosure further provides an immunoconjugate (for example, an antibody-drug conjugate) comprising the anti-c-MET antibody or antigen-binding fragment thereof according to any one of above and an effector molecule, wherein the effector molecule is conjugated to the anti-c-MET antibody or antigen-binding fragment thereof; and preferably, the effector molecule is selected from a radioactive isotope, an anti-tumor agent, an immunomodulatory agent, a biological response modifier, a lectin, a cytotoxic drug, a chromophore, a fluorophore, a chemiluminescent compound, an enzyme, a metal ion, and any combination thereof.

According to a sixth aspect, in some implementations, the present disclosure further provides a method of using the antibody or antigen-binding fragment thereof of the present invention for diagnosis and detection and a composition for diagnosis and detection comprising the antibody or antigen-binding fragment thereof.

In certain implementations, any anti-c-MET antibody or antigen-binding fragment thereof provided herein can be used to detect the presence of c-MET in a biological sample.

The term "detection" when used herein comprises quantitative or qualitative detection, exemplary detection methods may involve immunohistochemistry, immunocytochemistry, flow cytometry (for example, FACS), magnetic beads complexed with antibody molecules, ELISA assays methods, and PCR-techniques (for example, RT-PCR). In certain implementations, the biological sample is blood, serum, or other fluid samples of biological origin. In certain implementations, the biological sample comprises a cell or tissue. In some implementations, the biological sample is from tumor tissue or cancer tissue.

In an implementation, an anti-c-MET antibody for use in a method for diagnosis or detection is provided.

In another implementation, a method for detecting the presence of c-MET in the biological sample is provided. In certain implementations, the method comprises detecting the presence of a c-MET protein in the biological sample. In certain implementations, the c-MET is human c-MET or cyno c-MET. In certain implementations, the method comprises contacting the biological sample with the anti-c-MET antibody as described herein under conditions that allow the anti-c-MET antibody to bind to the c-MET, and detecting whether a complex is formed between the anti-c-MET antibody and the c-MET. The formation of the complex indicates the presence of the c-MET. The method may be an *in-vitro* or *in-vivo* method. In an implementation, the anti-c-MET antibody is used to select a subject eligible for therapy with the anti-c-MET antibody, for example, where the c-MET is a biomarker for selection of the subject. In some implementations, the method is performed *in-vitro* or *in-vivo.*

In certain implementations, a labeled antibody or fragment thereof are provided. Labels comprise, but are not limited to, labels or moieties that are detected directly (such as fluorescent labels, chromophoric labels, electron-dense labels, chemiluminescent labels, and radioactive labels), as well as moieties, such as enzymes or ligands, that are detected indirectly, for example, through an enzymatic reaction or molecular interaction.

In some implementations provided herein, the sample is obtained prior to treatment with the antibody or fragment thereof of the present invention. In some implementations, the sample is obtained prior to other treatments. In some implementations, the sample is obtained during or after treatment with other therapies.

In some implementations, the c-MET is detected prior to treatment, for example, prior to initial treatment or prior to a certain treatment following a treatment interval.

In some implementations, a method for treating a disease of the present invention is provided, and the method comprises: detecting the presence of c-MET in a subject (for example, a sample) (for example, a subject sample), thereby determining a c-MET value, comparing the c-MET value to a control value (for example, a value in a normal individual), and if the c-MET value is greater than the control value, administering to the subject a therapeutically effective amount of the antibodies or fragments thereof of the present invention or an antibody-drug conjugate, a pharmaceutical composition, a formulation, a combination product, and the like comprising the antibodies or fragments thereof and optionally in combination with one or more other therapies, thereby treating the disease.

Therefore, in an implementation, the present disclosure further provides a method for immune detection or assay of c-MET, comprising a step of contacting the anti-c-MET antibody according to any one of the above with a subject or a sample from the subject.

According to a seventh aspect, the present disclosure further provides an antibody-drug conjugate, a stereoisomer thereof, a prodrug thereof, a pharmaceutically acceptable salt thereof, a tautomer thereof, or a pharmaceutically acceptable solvate thereof, comprising the antibody or antigen-binding fragment thereof according to any one of the above.

In some implementations, the antibody-drug conjugate has a structure as represented in formula (I): 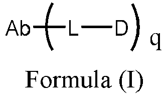 wherein:
Ab is the antibody or antigen-binding fragment thereof according to any one of the above;
D is an active drug unit;
L is a linker covalently attached to the antibody or antigen-binding fragment thereof Ab and the active drug unit D separately; and
q is an integer selected from 1-20.

In some implementations, q is selected from 1-18, 1-16, 1-14, 1-12, 1-10, 1-8, 2-8, or 4-6; for example, is an integer selected from 1-10.

Preferably, q is an integer selected from 1, 2, 3, 4, 5, 6, 7, 8, 9, 10, 11, 12, 13, or 14.

In some implementations, the L is covalently attached to an amino residue or a sulfhydryl residue on the antibody Ab; preferably, the L is covalently attached to the sulfhydryl residue on the antibody Ab; and more preferably, the L is covalently attached to a sulfhydryl residue formed after an interchain disulfide bond on the antibody Ab being opened up.

In some implementations, the L is a cleavable linker or a non-cleavable linker. Preferably, the L is a cleavable linker.

In some implementations, the cleavable linker comprises a peptide unit comprising 2-10 amino acid residues selected from natural amino acid residues, non-natural amino acid residues, or amino acid residues set forth in AA₁ or stereoisomers thereof; and
in some implementations, the peptide unit is a dipeptide, tripeptide, tetrapeptide, pentapeptide, hexapeptide, heptapeptide, octapeptide, nonapeptide, or decapeptide comprising at least 1 (for example, 1, 2, or 3) amino acid residue set forth in AA₁ or stereoisomer thereof.

In some preferred implementations, in some implementations, the peptide unit is a dipeptide, tripeptide, or tetrapeptide comprising 1 amino acid residue set forth in AA₁ or stereoisomer thereof.

In some implementations, the peptide unit consists of the following amino acids:
i. at least 1 (for example, 1, 2, or 3) amino acid residue set forth in AA₁ or stereoisomer thereof, and
ii. at least 1 natural amino acid residue and/or at least 1 non-natural amino acid residue.

The amino acid residue set forth in AA¹ has a structure as follows, 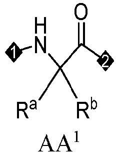 wherein:
either of R^{a} and R^{b} is H, and the other is 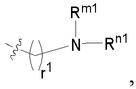 and r¹ is 4;
or R^{a} and R^{b}, taken together with the carbon atom to which they are attached, form a 5-6-membered heterocycle substituted with R⁰;
R^{m1} and Rⁿ¹ are each independently selected from hydrogen, C₁₋₆ alkyl, and C₃₋₆ cycloalkyl;
R⁰ is selected from C₁₋₆ alkyl, C₃₋₆ cycloalkyl, -NR^{m2}Rⁿ², and a 5-6-membered heterocyclyl optionally substituted with C₁₋₆ alkyl; and
R^{m2} and Rⁿ² are each independently selected from hydrogen and C₁₋₆ alkyl.
preferably, the amino acid residue is selected from -Val-, -Ala-, -Gly-, -Cit-, -AA¹-, -Arg-, - Phe-, -Lys-, and -Asn-.

Preferably, the peptide unit is selected from -valine-citrulline- (-Val-Cit-), -valine-alanine- (-Val-Ala-), -valine-lysine- (-Val-Lys-), -valine-arginine- (-Val-Arg-), -phenylalanine-citrulline- (-Phe-Cit-), -phenylalanine-lysine- (-Phe-Lys-), -phenylalanine-arginine- (-Phe-Arg-)-, -alanine-alanine-alanine- (-Ala-Ala-Ala-), -alanine-alanine-asparagine- (-Ala-Ala-Asn-), -valine-AA¹-glycine- (-Val-AA¹-Gly-), -valine-AA¹-alanine- (-Val-AA¹-Ala-), -glycine-glycine-phenylalanineglycine- (-Gly-Gly-Phe-Gly-), and -glycine-glycine-valine-alanine- (-Gly-Gly-Val-Ala-).

In some preferred implementation, the L is 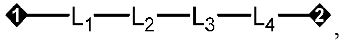 wherein,
L₁ is selected from: 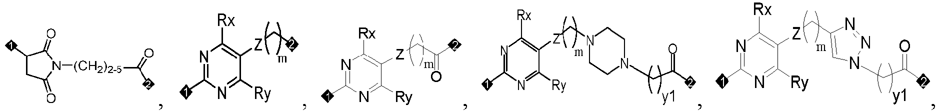 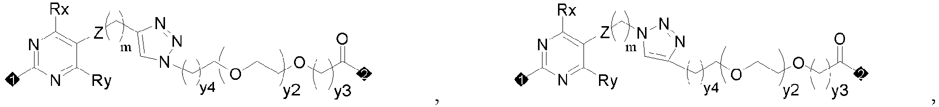 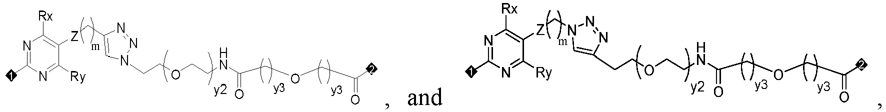 each Z is independently selected from a direct bond, a carbon-carbon triple bond, a carbon-carbon double bond, C₆₋₁₀ aryl, a 5-10-membered heteroaryl, and acylamino (preferably selected from a direct bond, a carbon-carbon triple bond, and a carbon-carbon double bond); Rx and Ry are independently selected from H and C₁₋₄ alkyl; each m is independently selected from 0, 1, 2, 3, 4, 5, and 6; y1 is selected from any integer from 1 to 6 (such as 4, 5, or 6); each y2 is independently selected from any integer from 0 to 15 (such as from 6 to 15); each y3 is independently selected from 1, 2, and 3; each y4 is independently selected from 0 and 1; the position 1 is attached to the antibody or antigen-binding fragment thereof via an S atom, and the position 2 is attached to L₂ or L₃; and
in some implementations, L₁ is selected from 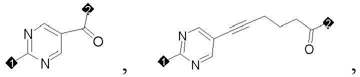 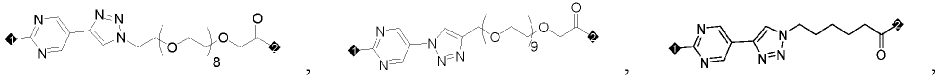 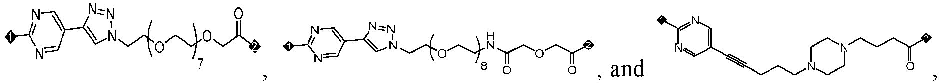 the position 1 is attached to Tb via an S atom, and the position 2 is attached to L₂ or L₃.

In some implementations, L₁ is selected from 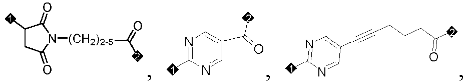 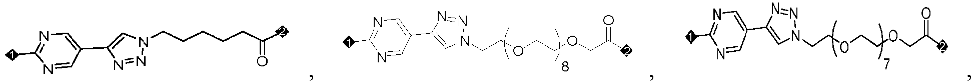 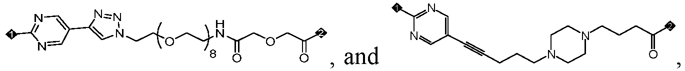 the position 1 is attached to Tb via an S atom, and the position 2 is attached to L₂ or L₃.

In some implementations, L₁ is selected from 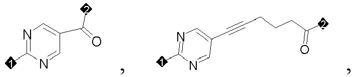 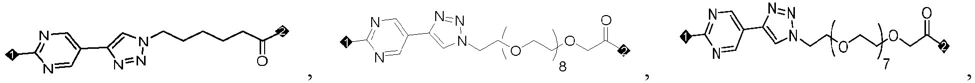 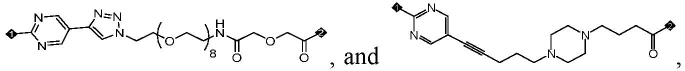 the position 1 is attached to Tb via an S atom, and the position 2 is attached to L₂ or L₃.

In some implementations, L₁ is selected from 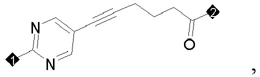 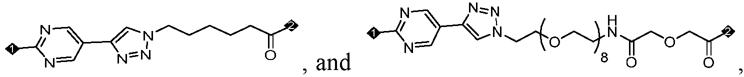 the position 1 is attached to Tb via an S atom, and the position 2 is attached to L₂ or L₃.

In some implementations, L₁ is selected from 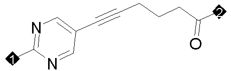 and 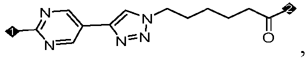 the position 1 is attached to Tb via an S atom, and the position 2 is attached to L₂ or L₃.

L₂ is absent or present, when L₂ is present, L₂ is selected from 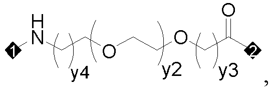 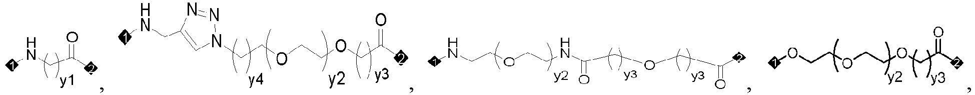 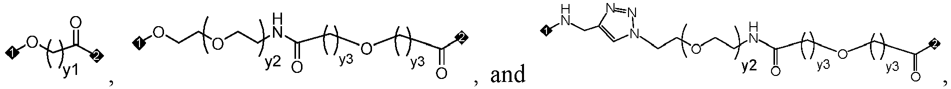 each y1 is selected from any integer from 1 to 6 (such as 4, 5, 6), each y2 is independently selected from any integer from 0 to 10 (such as from 6 to 10), each y3 is independently selected from 1 or 2, each y4 is independently selected from 0 or 1, the position 1 is attached to L₁, and the position 2 is attached to L₃;
in some implementations, L₂ is absent or present, when L₂ is present, L₂ is selected from 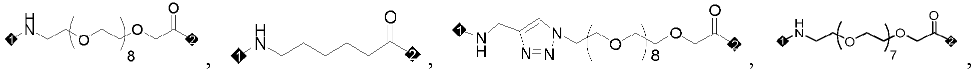 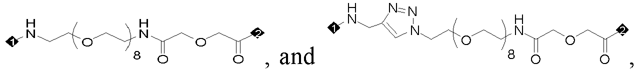 the position 1 is attached to L₁, and the position 2 is attached to L₃.

In some implementations, L₂ is absent or present, when L₂ is present, L₂ is selected from 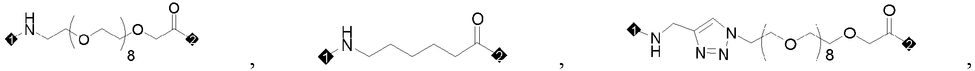 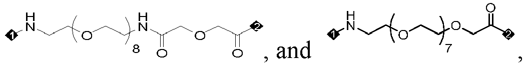 the position 1 is attached to L₁, and the position 2 is attached to L₃.

In some implementations, L₂ is absent or present, when L₂ is present, L₂ is selected from 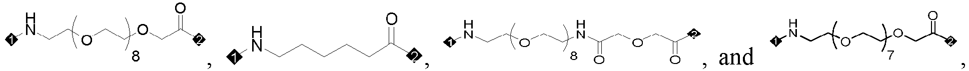 the position 1 is attached to L₁, and the position 2 is attached to L₃.

In some implementations, L₂ is absent.

In some implementations, L₂ is selected from 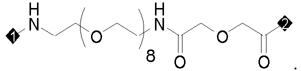
L₃ is selected from 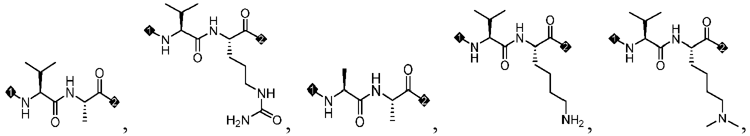 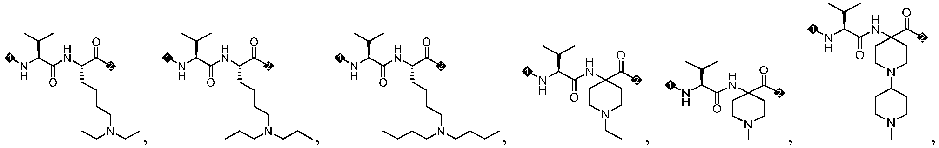 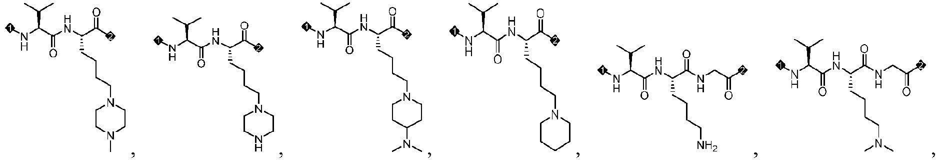 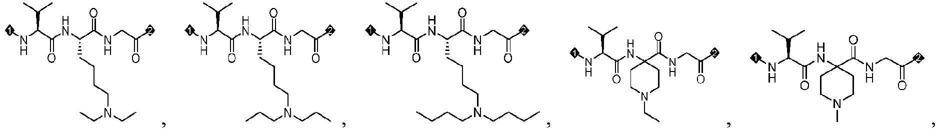 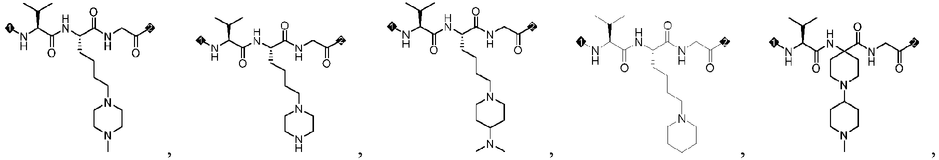 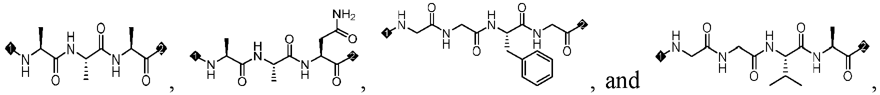 the position 1 is attached to L₁ or L₂, and the position 2 is attached to L₄ or D; and
L₄ is absent or present, when L₄ is present, L₄ is selected from 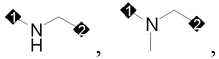 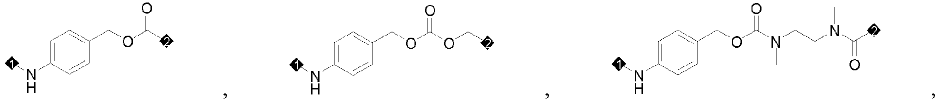 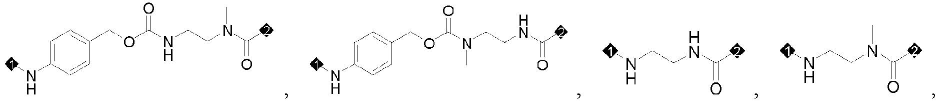 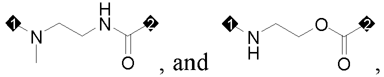 the position 1 is attached to L₃, and the position 2 is attached to D.

In some preferred implementations, the 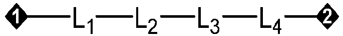 has a structure as follows:

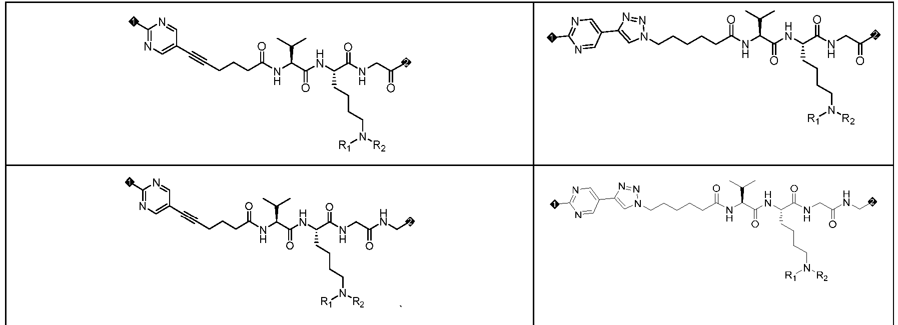

wherein R₁ and R₂ are independently selected from C₁₋₆ alkyl and H; and is preferably C₁₋₄ alkyl; and the position 1 is attached to the antibody or antigen-binding fragment thereof via an S atom, and the position 2 is attached to D.

Preferably, the 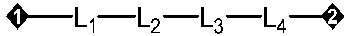 has a structure as follows:

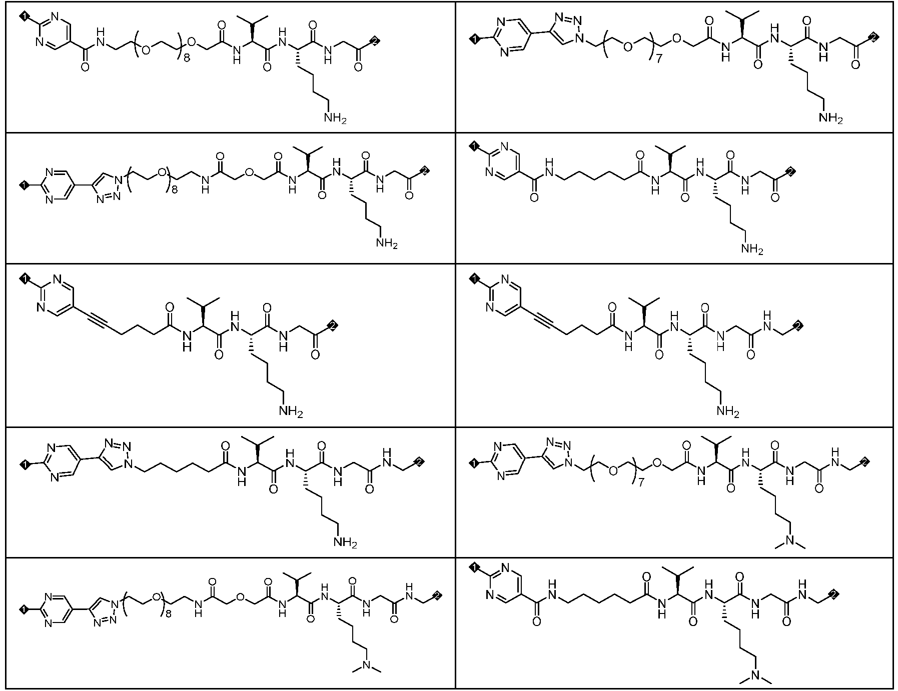

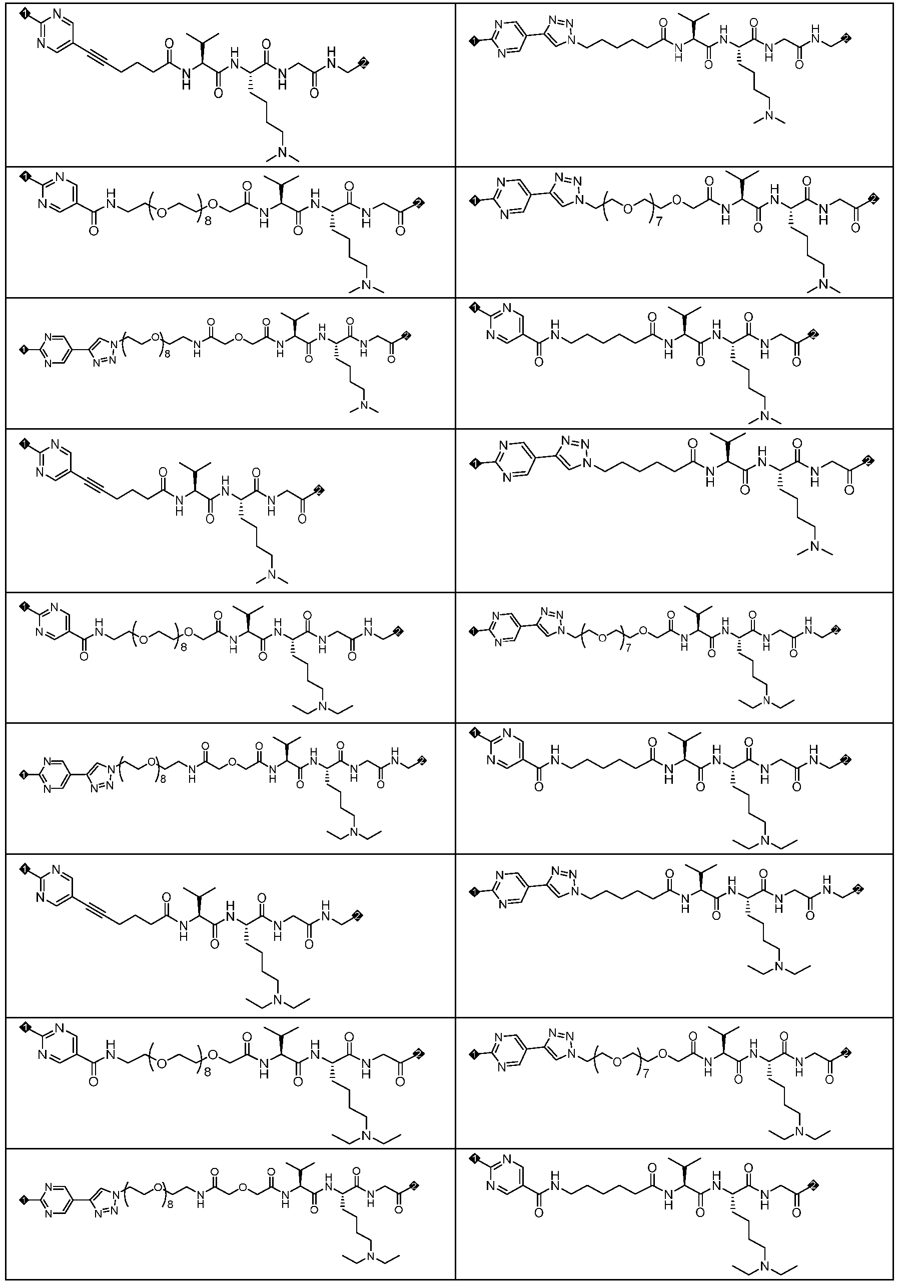

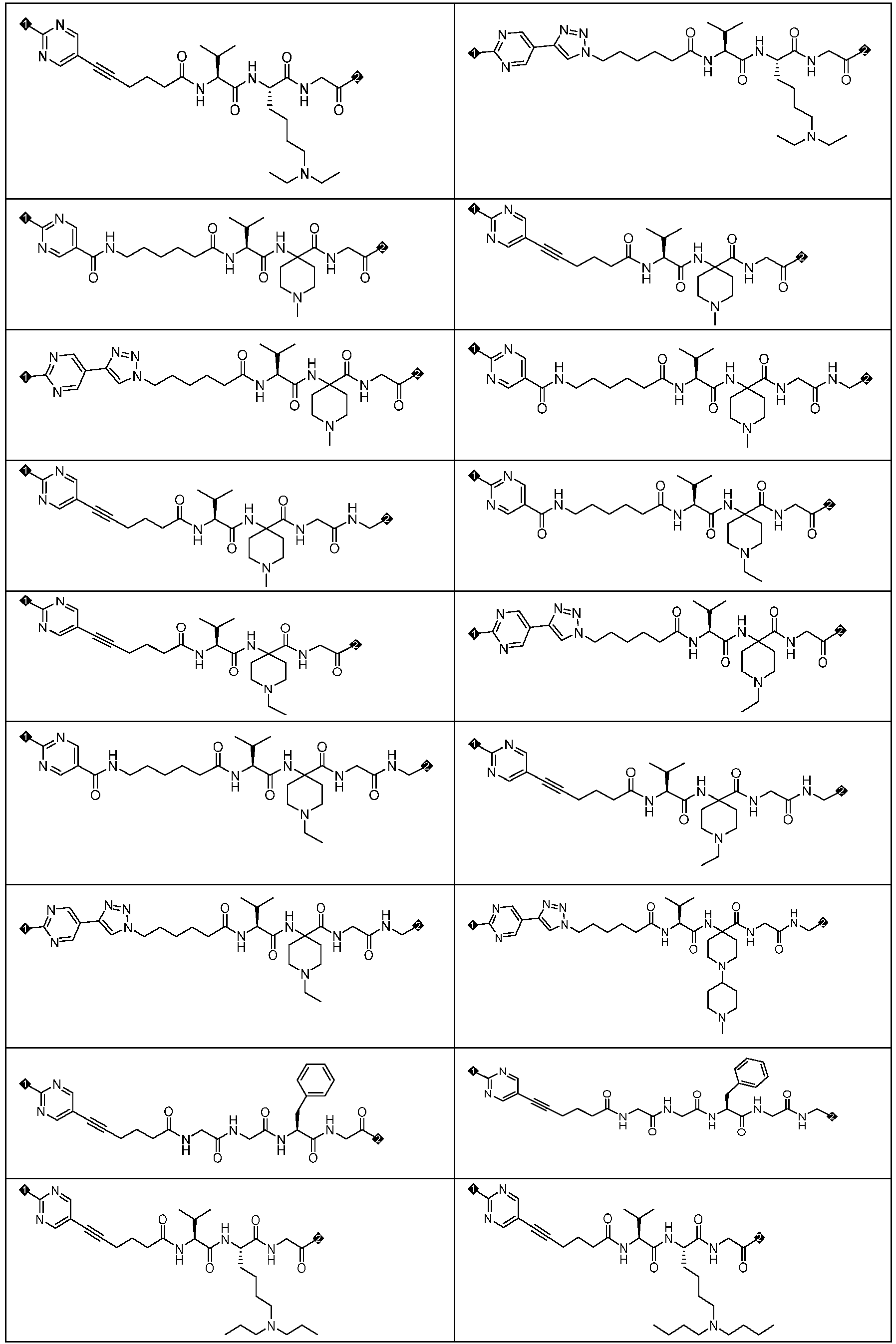

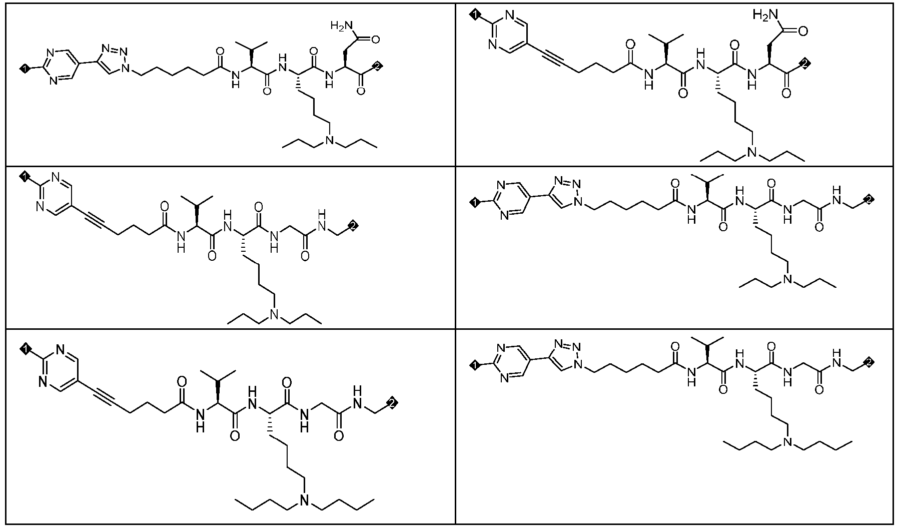

wherein the position 1 is attached to the antibody or antigen-binding fragment thereof via an S atom, and the position 2 is attached to D.

In some implementations, the active drug unit is selected from cytotoxic agents. In some implementations, the active drug unit is selected from a DNA topoisomerase inhibitor (for example, a camptothecin-based biologically active molecule, such as camptothecin, DXD, camptothecin with a substitutent modified or DXD with a substitutent base modified, hydroxycamptothecin, 9-aminocamptothecin, SN-38, irinotecan, topotecan, belotecan, and rubitecan) or a tubulin inhibitor (for example, an MMAF-like tubulin inhibitor, and an MMAE-like tubulin inhibitor).

In some implementations, the antibody-drug conjugate is a compound of Formula (IIA-1) or Formula (IIA-2): 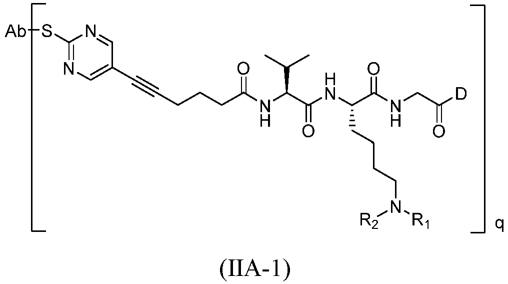 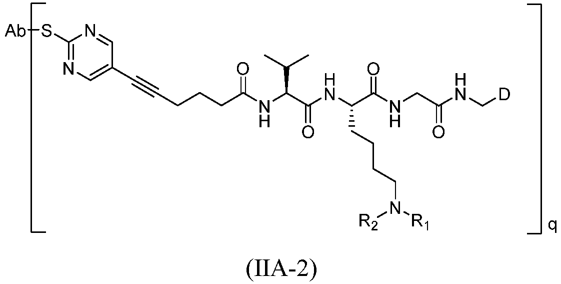 wherein,
Ab is the foregoing anti-c-Met antibody or antigen-binding fragment thereof, or a multispecific antibody;
R₁ and R₂ are independently selected from C₁₋₆ alkyl and H; and is preferably C₁₋₄ alkyl;
D is 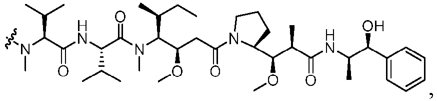 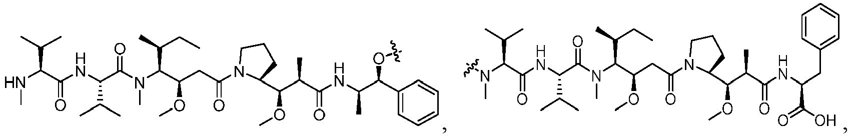 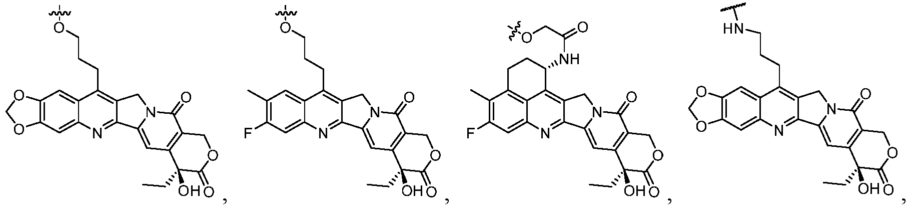 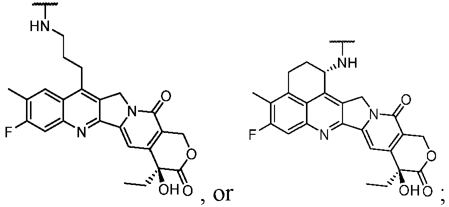 and
q is as defined above. In some preferred implementations, q is 2, 4, 6, or 8.

In some implementations, the antibody-drug conjugate is a compound of Formula (IIB-1) or Formula (IIB-2): 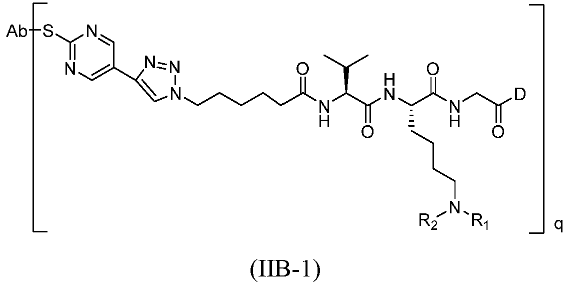 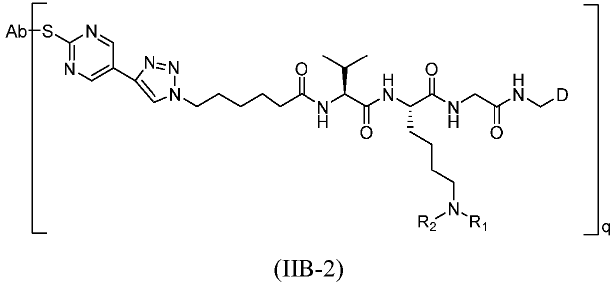 wherein,
Ab is the foregoing anti-c-Met antibody or antigen-binding fragment thereof, for example, a multispecific antibody or antigen-binding fragment thereof;
R₁ and R₂ are independently selected from C₁₋₆ alkyl and H; and is preferably C₁₋₄ alkyl;
D is 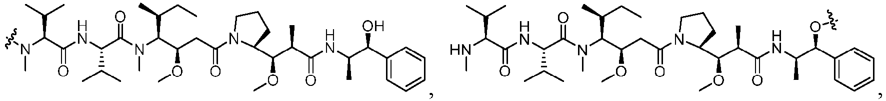 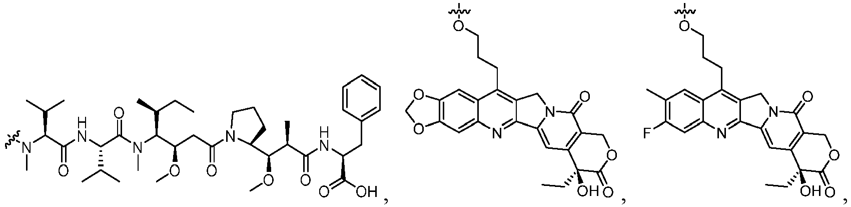 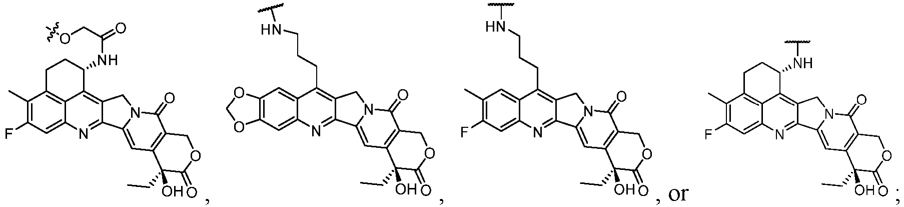 and
q is as defined above. In some preferred implementations, q is 2, 4, 6 or 8.

In some implementations, the antibody-drug conjugate has a structure as follows:

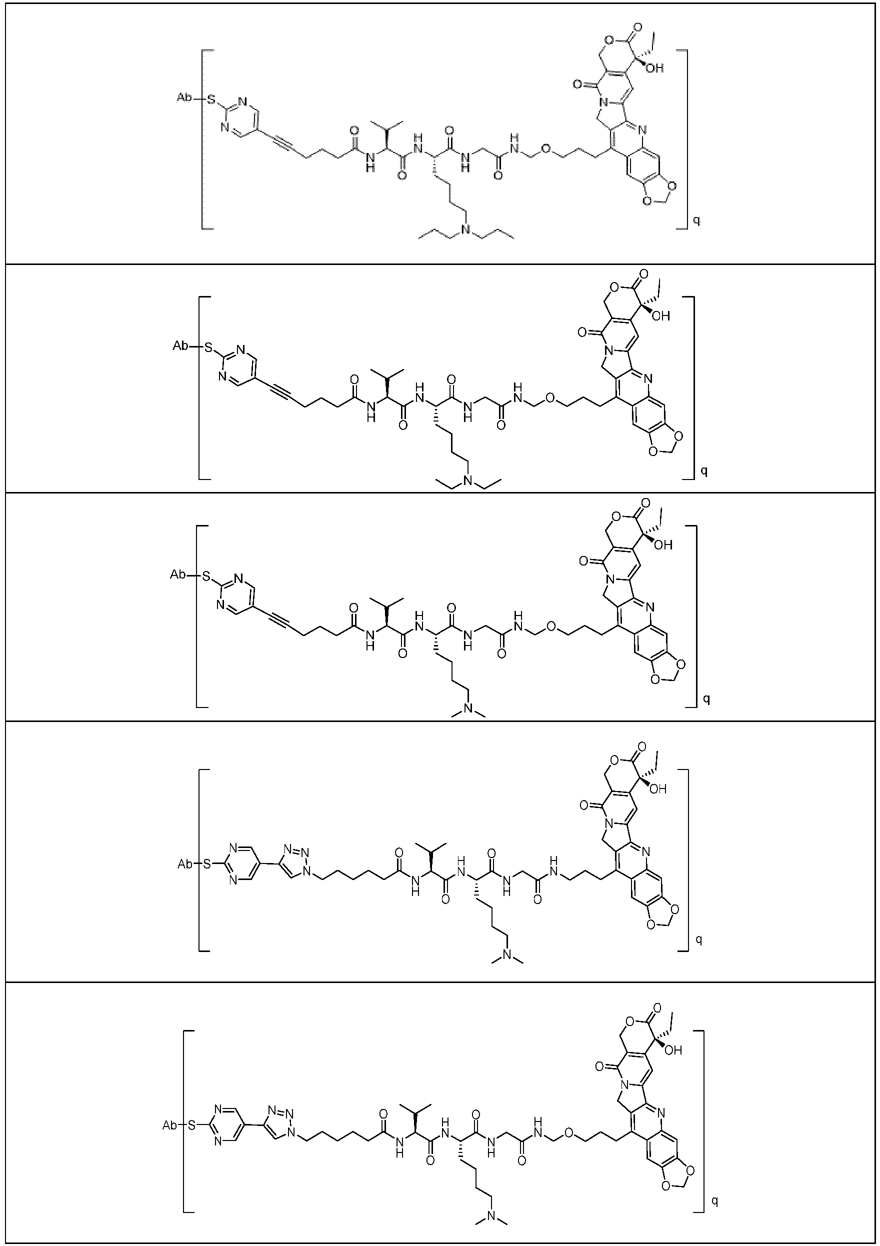

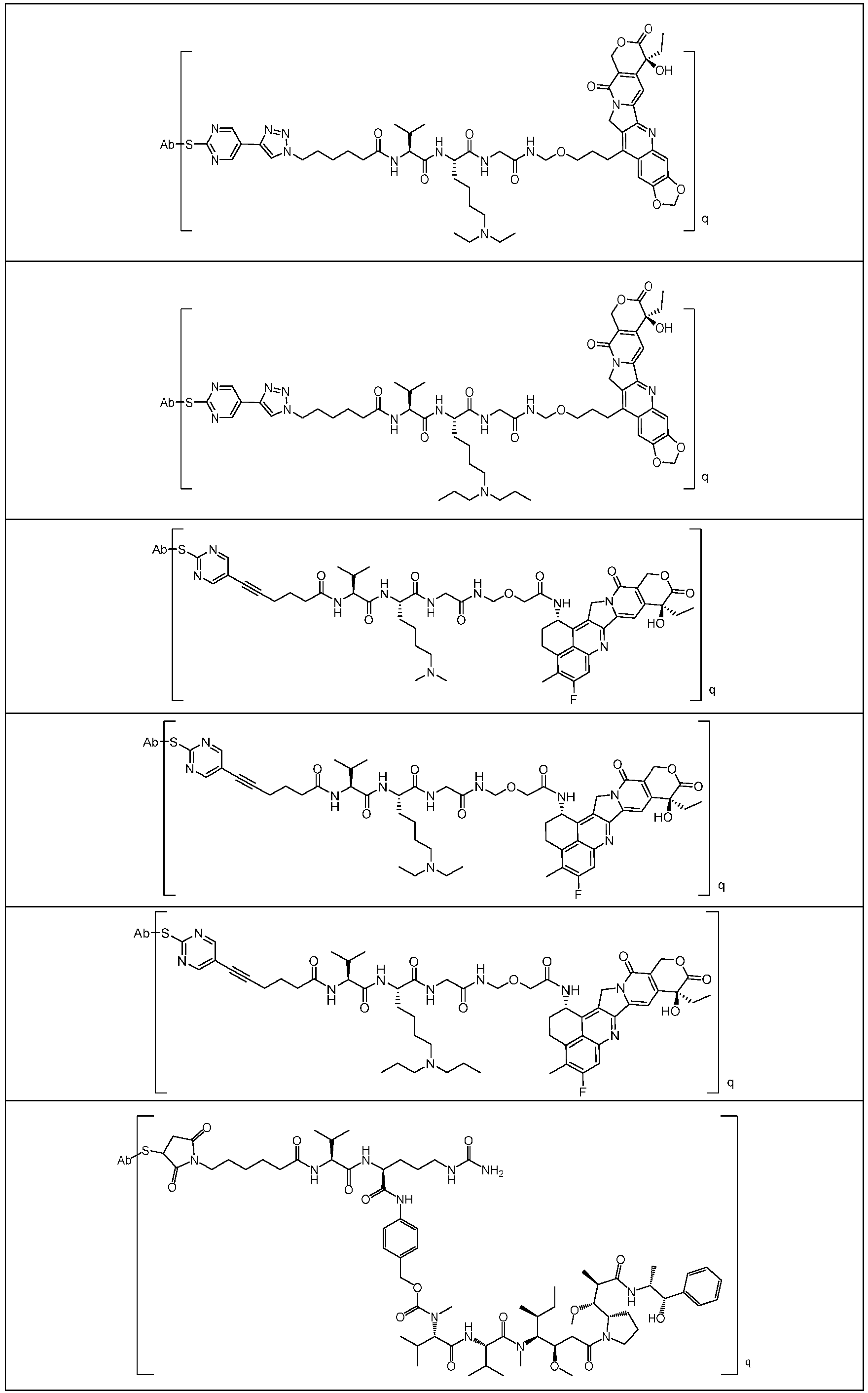

Ab and q are as defined above.

According to an eighth aspect, the present disclosure further provides a preparation method of an antibody-drug conjugate targeting c-MET, a stereoisomer thereof, a prodrug thereof, a pharmaceutically acceptable salt thereof, a tautomer thereof, or a pharmaceutically acceptable solvate thereof, wherein the antibody-drug conjugate has a structure as represented in Formula I, and the method comprises the following steps:
(1) reacting an anti-c-MET antibody or fragment thereof with a reducing reagent in a buffer, to obtain a reduced antibody or fragment thereof; wherein preferably, the reducing reagent is a disulfide bond reducing agent, such as TCEP; and preferably, the buffer has a pH of 6.0-8.0, such as a pH of 6.5, 7.0, 7.5, or 8.0, and more preferably is a phosphate-buffered saline; and
(2) cross-linking a linker-payload (linker-drug conjugate) and the reduced antibody or fragment thereof obtained in step (1) in a mixture of the buffer and an organic solvent, to obtain an antibody-drug conjugate targeting c-MET, wherein the buffer is as defined above, and preferably, the organic solvent is selected from dimethyl sulfoxide.

In some implementations of the present disclosure, the c-MET antibody or fragment thereof is as defined above; and the linker-payload has a structure as represented in the following Formula (IIIA-1), (IIIA-2), (IIIB-1), or (IIIB-2): 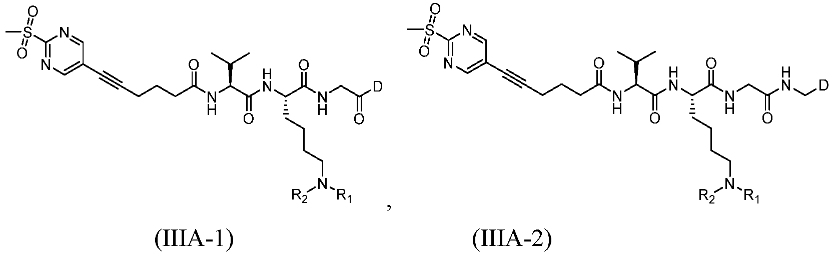 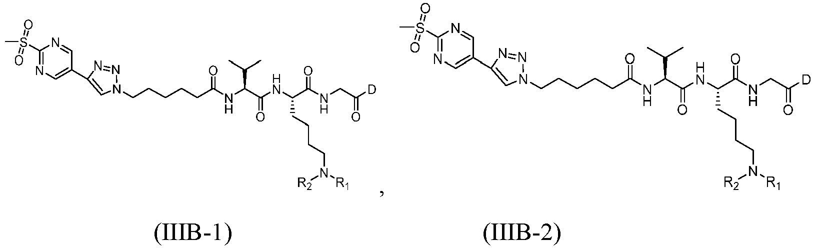 wherein,
R₁ and R₂ are independently selected from C₁₋₆ alkyl and H; and is preferably C₁₋₄ alkyl; and
D is

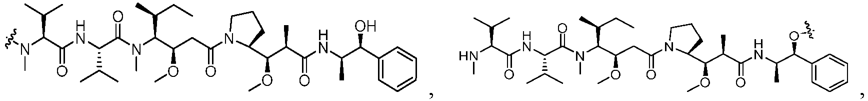

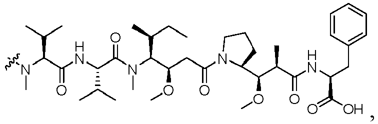

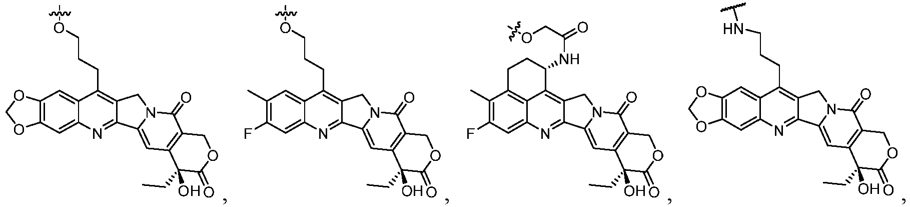

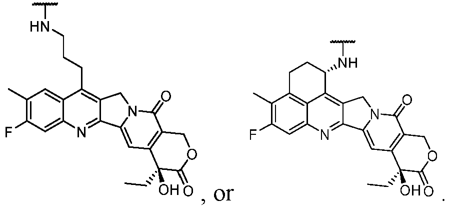

In some implementations, the linker-payload has a structure as represented in following formula:

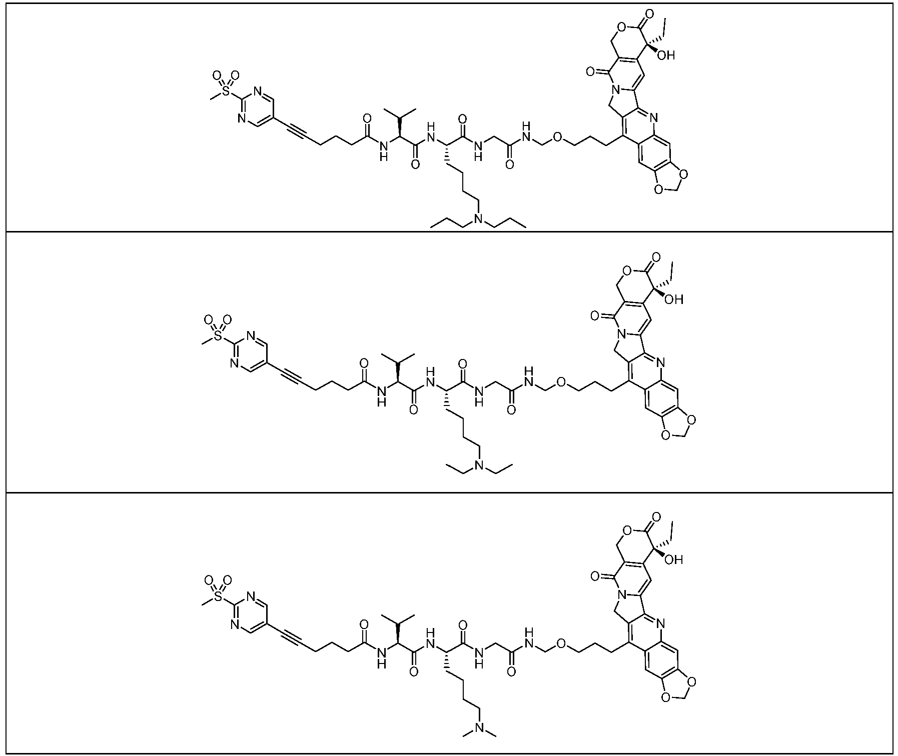

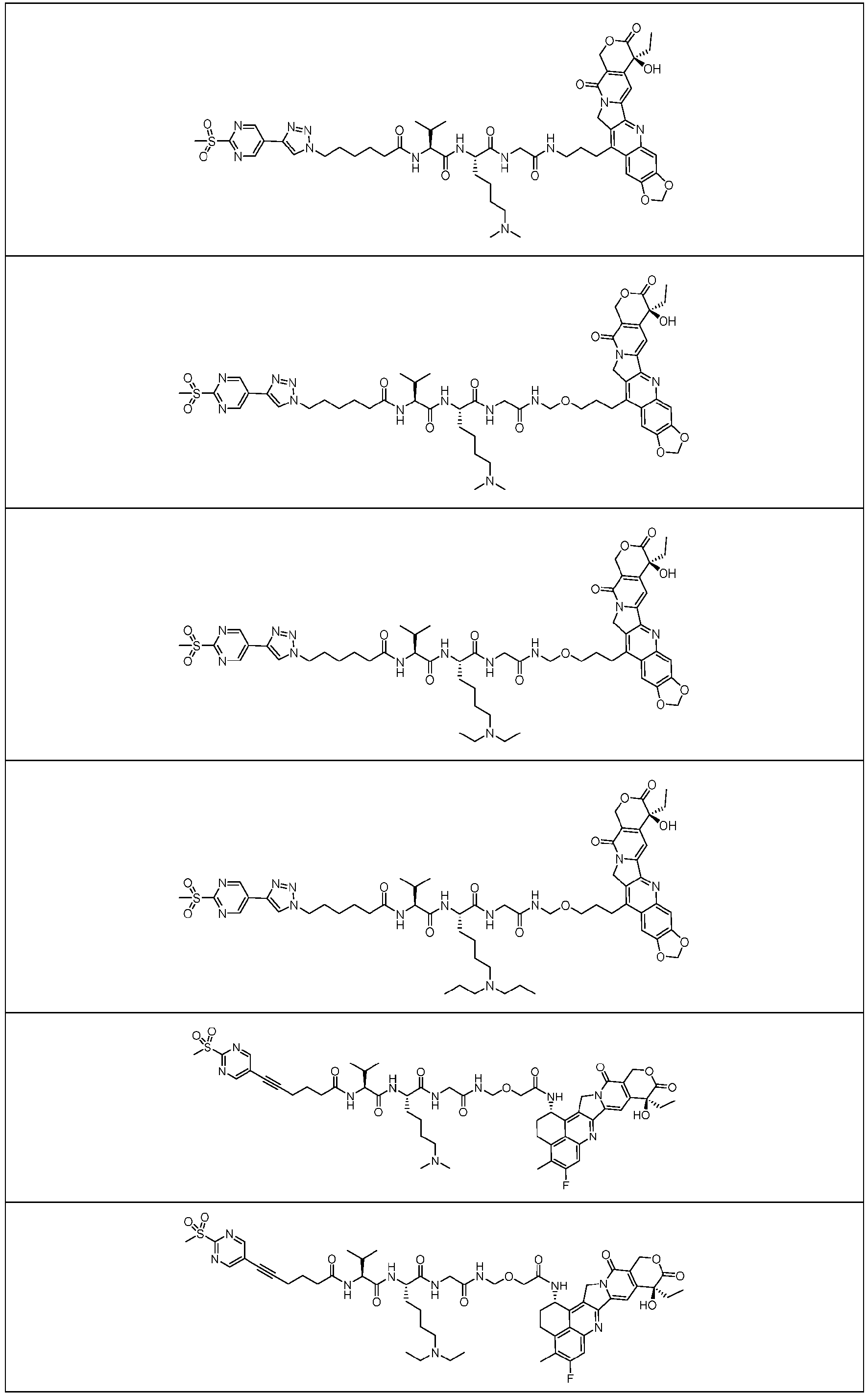

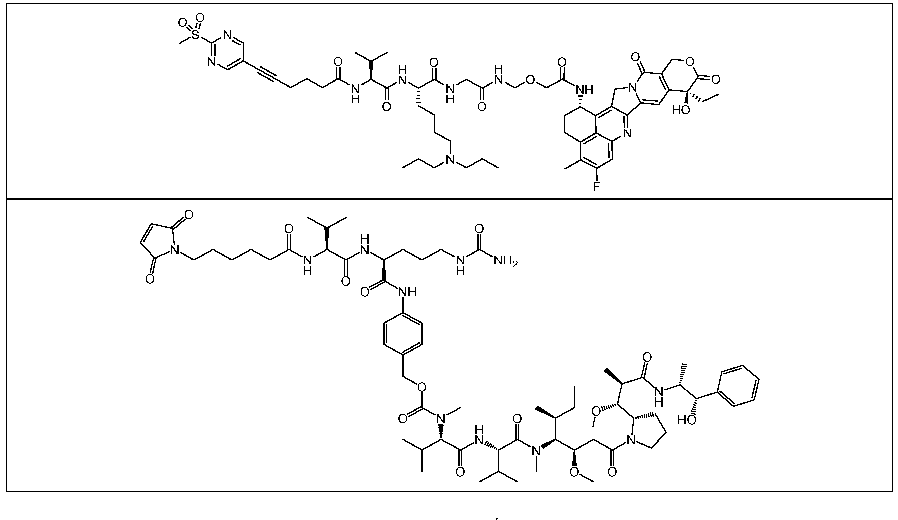

The linker-payload of the present disclosure can be prepared by various methods known in the art, for example, obtained through chemical synthesis techniques. The linker-payload in the above antibody-drug conjugate can be prepared and obtained with reference to WO22022170971, and then is conjugated with the antibody to form an ADC.

According to ninth aspect of the present disclosure, the present disclosure provides a population of antibody-drug conjugates, comprising or consisting of the antibody-drug conjugate, the stereoisomer thereof, the prodrug thereof, the pharmaceutically acceptable salt thereof, the tautomer thereof, or the pharmaceutically acceptable solvate thereof according to the seventh aspect, wherein the antibody-drug conjugate has one, two, or more q values.

In some implementations, when a q value of the antibody-drug conjugate accounts for the majority (such as 80%, 85%, 90%, 95%, 95%, 97%, 98%, 99%) in the population of antibody-drug conjugates, the q value is close to an average DAR.

In some implementations, when the antibody-drug conjugate in the population of antibody-drug conjugates only has one q value, the q value is equivalent to the average DAR.

In some implementations, when the antibody-drug conjugates of the population of antibody-drug conjugates have two or more q values, the antibody-drug conjugate with a specific q value accounts for 50%, 60%, 70%, 80%, 90%, 95%, 96%, 97%, 98%, 99% of all of the antibody-drug conjugates in the composition.

In some implementations, the average drug-to-antibody ratio (average DAR) of the antibody-drug conjugates in the population of antibody-drug conjugates is an integer or decimal number selected from 1-16, preferably 1-10.

In some implementations, the average drug-to-antibody ratio (average DAR) of the population of antibody-drug conjugates is selected from 1.5-2.5, 3.5-4.5, 5.5-6.5, or 7.5-8.5;
in some implementations, the average drug-to-antibody ratio (average DAR) of the population of antibody-drug conjugates is selected from about 2.0, 4.0, 6.0, or 8.0; and
in some implementations, the average drug-to-antibody ratio (average DAR) of the antibody-drug conjugates in the population of antibody-drug conjugates is selected from an integer or decimal number from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, 9, or 9.7.

In some implementations, the population of antibody-drug conjugates comprises ADCs with a distribution of DARs from 1 to 8, for example, 1.5, 2, 4, 6, and 8 (that is, 1.5, 2, 4, 6, and 8 drug-loaded types). It should be noted that degradation products can be generated, such that the mixture can also comprise DARs of 1, 3, 5, and 7. In addition, the population of antibody-drug conjugates can also have an average DAR greater than 8. The antibody-drug conjugate is produced by reduction of interchain disulfides and then conjugation. In some implementations, the antibody-drug conjugate comprises both: the antibody-drug conjugate with a DAR of 4 or lower (that is, 4 or less drug-loaded types) and the antibody-drug conjugate with a DAR of 6 or higher (that is, 6 or more drug-loaded types).

According to a tenth aspect of the present disclosure, the present disclosure provides a pharmaceutical composition, comprising the antibody or antigen-binding fragment thereof according to the first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, the nucleic acid according to the third aspect, the immunoconjugate according to the fifth aspect or the population of antibody-drug conjugates according to the ninth aspect, and optionally one or more pharmaceutically acceptable excipients, such as pharmaceutically acceptable carrier and pharmaceutically acceptable excipient known in the art, including a buffer.

In certain embodiments, the pharmaceutical composition comprises an effective amount of the following substances: the antibody or antigen-binding fragment thereof according to the first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, the nucleic acid according to the third aspect, the immunoconjugate according to the fifth aspect, or the population of antibody-drug conjugates according to the ninth aspect.

In certain implementations, the pharmaceutical composition comprises the foregoing antibody-drug conjugate, the stereoisomer thereof, the prodrug thereof, the pharmaceutically acceptable salt thereof, the tautomer thereof, or the pharmaceutically acceptable solvate thereof, and the pharmaceutically acceptable excipients.

In certain implementations, the pharmaceutical composition of the present disclosure comprises the population of antibody-drug conjugates according to the ninth aspect and the pharmaceutically acceptable excipients.

In certain implementations, the pharmaceutical composition of the present disclosure comprises the anti-c-MET antibody or antigen-binding fragment thereof according to the first aspect of the present disclosure and pharmaceutically acceptable excipients.

In certain implementations, the pharmaceutical composition of the present disclosure comprises the host cell of the present disclosure and the pharmaceutically acceptable carrier and/or excipient, wherein the host cell comprises the isolated nucleic acid molecule or the vector as described above.

In certain implementations, the pharmaceutical composition of the present disclosure comprises the multispecific antibody according to the second aspect of the present disclosure and pharmaceutically acceptable excipients.

In some implementations, the drug-to-antibody ratio (average DAR) of the pharmaceutical composition or the population of antibody-drug conjugates is an integer or a decimal number selected from 1-10.

In some implementations, the drug-to-antibody ratio (average DAR) of the pharmaceutical composition or the population of antibody-drug conjugates is selected from 1.5-2.5, 3.5-4.5, 5.5-6.5, and 7.5-8.5;
in some implementations, the DAR of the pharmaceutical composition or the population of antibody-drug conjugates is selected from: 2 ± 0.5, 4 ± 0.5, 5 ± 0.5, 6 ± 0.5, 7 ± 0.5, 8 ± 0.5;
in some implementations, the drug-to-antibody ratio (average DAR) of the pharmaceutical composition or the population of antibody-drug conjugates is selected from about 2.0, 4.0, 6.0, or 8.0; and
in some implementations, the drug-to-antibody ratio (average DAR) of the pharmaceutical composition or the population of antibody-drug conjugates is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, 9, or 9.7.

In some implementations, the pharmaceutical composition comprises the antibody or antigen-binding fragment thereof according to the first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, and the buffer.

In some implementations, the pharmaceutical composition comprises the antibody-drug conjugate, the stereoisomer thereof, the prodrug thereof, the pharmaceutically acceptable salt thereof, the tautomer thereof, or the pharmaceutically acceptable solvate thereof according to the seventh aspect, and the buffer.

In some preferred implementations, the buffer is selected from: a histidine buffer and a phosphate buffer. In some preferred implementations, the buffer is selected from the histidine buffer. In some preferred implementations the buffer is selected from a histidine buffer of 20 mM.

According to an eleventh aspect, provided is use of the antibody or antigen-binding fragment thereof of the present disclosure in the preparation of a kit for detecting the presence or level of c-MET in a sample. According to another aspect, the present disclosure provides a diagnostic or therapeutic kit, comprising one or more of the following substances: the antibody or antigen-binding fragment thereof, the nucleic acid, the vector, the host cell, the multispecific antibody, the antibody-drug conjugate, the population of antibody-drug conjugates, or the pharmaceutical composition described in the present disclosure. Optionally, the diagnostic or therapeutic kit further comprises instructions for use. In some implementations, the kit is applicable to the diagnostic or detection method described in the present disclosure. In some implementations, the kit is applicable to the treatment method described in the present disclosure.

According to a twelfth aspect, the present disclosure provides use of the substances (comprising the foregoing antibody-drug conjugate composition or the foregoing pharmaceutical composition) of the present disclosure in the preparation of a medicine for treatment and/or prophylaxis of diseases (for example, cancer diseases) related to abnormal cell activities. In some implementations, the antibody-drug conjugate composition or the foregoing pharmaceutical composition is of an effective amount, such as a therapeutically effective amount.

In some implementations, provided is use of the anti-c-MET antibody or antigen-binding fragment thereof, the nucleic acid, the vector, the host cell, or the multispecific antibody of the present disclosure in the preparation of a medicine for adjusting (inhibiting or blocking) the activity of c-MET.

In some implementations, provided is use of the anti-c-MET antibody or antigen-binding fragment thereof, the nucleic acid, the vector, the host cell, the antibody-drug conjugate, or the multispecific antibody of the present disclosure in the preparation of a medicine for treatment or prophylaxis of diseases related to the activity of c-MET or diseases related to the target of c-MET.

In some implementations, provided is use of the anti-c-MET antibody or antigen-binding fragment thereof, the nucleic acid, the vector, the host cell, the antibody-drug conjugate, or the multispecific antibody of the present disclosure in the preparation of a medicine for treatment or prophylaxis of tumors related to the activity of c-MET.

In some implementations, provided is use of the substances of the present disclosure in the preparation of a medicine for treatment or prophylaxis of diseases related to the activity of c-MET or diseases related to the target of c-MET, and the substances of the present disclosure are selected from the foregoing antibody-drug conjugate, the stereoisomer thereof, the prodrug thereof, the pharmaceutically acceptable salt thereof, the tautomer thereof, or the pharmaceutically acceptable solvate thereof of the present disclosure, the anti-c-MET antibody or antigen-binding fragment thereof according to the first aspect, the multispecific antibody according to the second aspect, the nucleic acid according to the third aspect, the vector according to the fourth aspect, the vector according to the sixth aspect, the immunoconjugate according to the seventh aspect, the population of antibody-drug conjugates according to the ninth aspect, or the pharmaceutical composition according to the tenth aspect.

In some implementations, provided is use of the antibody or antigen-binding fragment thereof according to the foregoing first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, the population of antibody-drug conjugates according to the ninth aspect, or the pharmaceutical composition according to the tenth aspect of the present disclosure in the preparation of a medicine for treatment or prophylaxis of diseases related to the activity of c-MET or diseases related to the target of c-MET.

According to a thirteenth aspect, the present disclosure provides a method of using the antibody or antigen-binding fragment thereof according to the foregoing first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, the nucleic acid according to the third aspect, the vector according to the fourth aspect, the immunoconjugate according to the fifth aspect or the population of antibody-drug conjugates according to the ninth aspect, or the pharmaceutical composition according to the tenth aspect, in treatment and/or prophylaxis of diseases (for example, tumors) related to abnormal cell activities.

In the foregoing twelfth and thirteenth aspects, the diseases related to the activity of c-MET or the diseases related to the target of c-MET or the diseases related to abnormal cell activities comprise tumors, such as cancers. The cancer may be early, intermediate, or advanced, or be a metastatic cancer. In some implementations, the cancer may be a solid tumor or hematologic tumor.

In an implementation, the tumor means that in tumor tissue or tumor cells of an individual, for example, compared to the adjacent normal tissue or normal cells (for example, normal cells in tissue) of the individual or the same tissue or cells therein in a healthy individual, the protein level (for example, expression) of c-MET is increased, or the nucleic acid level of c-MET is increased.

The tumor is selected from, but not limited to: lung cancer (for example, non-small cell lung cancer, small cell lung cancer, or lung adenocarcinoma), colon cancer (for example, human colon adenocarcinoma), rectal cancer, gastric cancer, colorectal cancer (for example, colorectal adenocarcinoma).

In the foregoing twelfth and thirteenth aspects, the antibody or antigen-binding fragment thereof according to the foregoing first aspect, the multispecific antibody according to the second aspect, the antibody-drug conjugate, the stereoisomer thereof, the prodrug thereof, the pharmaceutically acceptable salt thereof, the tautomer thereof, or the pharmaceutically acceptable solvate thereof according to the seventh aspect, the nucleic acid according to the third aspect, the vector according to the fourth aspect, the immunoconjugate according to the fifth aspect or the population of antibody-drug conjugates according to the ninth aspect, or the pharmaceutical composition according to the tenth aspect, can be further combined with other treatment modalities or therapeutic agents to treat related diseases or for related use.

In some implementations, the treatment modality is surgical treatment or radiation therapy.

According to a fourteenth aspect, the present disclosure further provides a pharmaceutical combination or pharmaceutical combination product, comprising the anti-c-MET antibody or fragment thereof (preferably antigen-binding fragment) of the present invention, or antibody-drug conjugate thereof, and one or more additional therapeutic agents.

Another objective of the present invention is to provide a kit-of-parts, comprising the pharmaceutical combination of the present invention, and preferably the kit is in the form of pharmaceutical dosage units. Therefore, dosage units can be provided based on dosing regimens or dosing intervals.

In an implementation, the kit-of-parts of the present invention comprises, in the same package:
- a first container comprising a pharmaceutical composition containing an anti-c-MET antibody or fragment thereof; and
- a second container comprising a pharmaceutical composition containing additional therapeutic agents.

According to a fifteenth aspect, the present disclosure further provides use of the antibody or antigen-binding fragment thereof according to the foregoing first aspect and the multispecific antibody according to the second aspect in the preparation of an antibody-drug conjugate.

In some implementations, the antibody-drug conjugate is selected from the antibody-drug conjugate, the stereoisomer thereof, the prodrug thereof, the pharmaceutically acceptable salt thereof, the tautomer thereof, or the pharmaceutically acceptable solvate thereof according to the seventh aspect.

The immunoglobulin molecule of the present disclosure can be of any type (such as IgG, IgE, IgM, IgD, IgA, and IgY), class (such as IgG1, IgG2, IgG3, IgG4, IgA1, and IgA2), or subclass of immunoglobulin. Preferably, the antibody of the present disclosure comprises or consists of a VH domain, a VH CDR (mostly represented by HCDR herein), a VL domain, or a VL CDR (mostly represented by LCDR herein) having a sequence and any amino acid sequence described in a specific information table thereof or a fragment or variant thereof.

Preferably, the antibody of the present publication comprises or consists of a VH domain, a VH CDR (mostly represented by HCDR herein), a VL domain, or a VL CDR (mostly represented by LCDR herein) having a sequence and any amino acid sequence described in a specific information table thereof or a fragment or variant thereof.

In the present disclosure, the term "monoclonal antibody" refers to an antibody from a group of antibodies that is substantially homogeneous, that is, the antibodies composing the group are completely identical except for the possible presence of a small amount of natural mutations. The modifier "monoclonal" here indicates that the antibody is characterized by being from a substantially homogeneous group of antibodies and should not be construed as requiring preparation by a specific method.

In some implementations of the present disclosure, the monoclonal antibody further specifically comprises a chimeric antibody, that is, a portion of the heavy chain and/or light chain is identical or homologous to a certain type, class, or subclass of antibody, and the rest is identical or homologous to another type, class, or subclass of antibody, as long as they have the desired biological activity. Chimeric antibodies applicable to the present disclosure comprise primatized antibodies comprising variable region antigen-binding sequences derived from a non-human primate (such as an ancient monkey and orangutan) and human constant region sequences.

The term "antigen-binding fragment" refers to a portion of an antibody, preferably is an antigen-binding region or variable region. Examples of antibody fragments comprise Fab, Fab', F(ab')₂, Fd, Fv, dAb, and complementarity determining region fragment, a diabody, a linear antibody, and a single-chain antibody molecule. The term "antigen-binding fragment" used herein refers to a partial fragment of an antibody with the antigen-binding activity, wherein the fragment has all or a part of the functions of the antibody, comprising but not limited to single-chain Fv (scFv), Fab, Fab', F(ab')₂, disulfide bond-attached Fv (sdFv), Fv, di-scFv, and the like. The term also comprises Fab', which is a monovalent fragment of the variable region of an antibody obtained by treatment on F(ab')2 under reducing conditions. However, the term is not limited to these molecules as long as the fragment has a binding affinity to an antigen. Furthermore, these functional fragments comprise not only fragments obtained by treatment on the full-length molecule of an antibody protein with an appropriate enzyme, but also proteins produced in an appropriate host cell using genetically modified antibody genes.

The term "Fab'" used herein refers to the foregoing monovalent fragment of the variable region of an antibody obtained by treatment on F(ab')₂ under reducing conditions. However, Fab' of the present disclosure also comprises Fab' produced using genetically modified antibody genes.

As used herein, the term "scFv" refers to a single polypeptide chain comprising VL and VH domains, wherein the VL and VH domains are attached to each other via a linker or directly (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Volume 113, Roseburg and Moore, Eds., Springer-Verlag, New York, pp. 269-315 (1994)). Such scFv molecules may have a general structure: NH2-VL-linker-VH-COOH or NH2-VH-linker-VL-COOH. Suitable linkers in the prior art consist of repeated GGGGS amino acid sequences or variants thereof. For example, a linker with the amino acid sequence (GGGGS)4 can be used, but variants thereof can also be used (Holliger et al. (1993), Proc. Natl. Acad. Sci. USA 90: 6444-6448). Other linkers applicable to the present disclosure are described by Alfthan et al. (1995), Protein Eng. 8:725-731; Choi et al. (2001), Eur. J. Immunol. 31: 94-106; Hu et al. (1996), Cancer Res. 56:3055-3061; Kipriyanov et al. (1999), J. Mol. Biol. 293: 41-56; and Roovers et al. (2001), Cancer Immunol. In some cases, a disulfide bond may also be present between VH and VL of scFv. As used herein, the term "di-scFv" refers to an antibody fragment formed by two attached scFvs.

The term "variable region", "variable domain", or "variable domain(s)" refers to the domain(s) involving in binding of an antibody to an antigen in the antibody heavy and/or light chain. Natural IgG antibodies VH and VL each comprise four conserved framework regions (FRs) and three complementarity determining regions (CDRs). The term "complementarity determining region" or "CDR" refers to the region within the variable domain that mainly contributes to binding to an antigen; and "framework" or "FR" refers to the variable domain residue in the variable domain except for CDR residues. The VH comprises 3 CDRs: the HCDR1, the HCDR2, and the HCDR3; and the VL comprises 3 CDRs: the LCDR1, the LCDR2, and the LCDR3. Each VH and VL is composed of three CDRs and four FRs arranged in the following sequence from the amino terminus to the carboxyl terminus: FR1, CDR1, FR2, CDR2, FR3, CDR3, and FR4. A single VH or VL may be sufficient to confer antigen-binding specificity.

The boundaries of the amino acid sequences of CDRs can be determined through various well-known definition schemes, such as: the definition scheme rules of "Kabat" (see Kabat et al. (1991), "Sequences of Proteins of Immunological Interest", 5th Edition, Public Health Service, National Institutes of Health, Bethesda, MD), the definition scheme of "Chothia", the definition scheme of "ABM", the definition scheme of "contact" (see Martin, ACR. Protein Sequence and Structure Analysis of Antibody Variable Domains[J].2001), and the definition scheme of ImMunoGenTics (IMGT) (Lefranc, M.P. et al., Dev. Comp. Immunol., 27, 55-77 (2003); Front Immunol. 2018 Oct 16; 9:2278), etc.; and the correspondences among the various definition schemes are well known to those skilled in the art, and an example is as shown below.

### Relationships among the CDR definition schemes

| Loop | Kabat | AbM | Chothia | Contact | IMGT |
|---|---|---|---|---|---|
| LCDR1 | L24--L34 | L24--L34 | L24--L34 | L30--L36 | L27--L32 |
| LCDR2 | L50--L56 | L50--L56 | L50--L56 | L46--L55 | L50--L51 |
| LCDR3 | L89--L97 | L89--L97 | L89--L97 | L89--L96 | L89--L97 |
| HCDR1 | H31--H35B (Kabat Numbering) | H26-H35B | H26-H32..34 | H30-H35B | H26-H35B |
| HCDR1 | H31--H35 (Chothia Numbering) | H26--H35 | H26--H32 | H30--H35 | H26--H33 |
| HCDR2 | H50--H65 | H50--H58 | H52--H56 | H47--H58 | H51--H56 |
| HCDR3 | H95--H102 | H95--H102 | H95--H102 | H93--H101 | H93--H102 |

As used herein, the antibody or antigen-binding fragment thereof comprising variants, amino acid substitutions, deletions, or additions still have the activity to bind the antigen.

The term "binding molecule" refers to any molecule capable of specifically binding to a target, such as an antibody or antigen-binding fragment thereof or fusion protein. The term "multispecific binding molecule" refers to a multispecific binding molecule that is at least bispecific, for example, a bispecific binding molecule, that is, the molecule comprises at least a first target binding region and a second target binding region, wherein the first target binding region binds to one target or antigen and the second target binding region binds to another antigen or target. The multispecific binding molecules according to the present invention also encompass multispecific molecules comprising a plurality of target binding regions, such as trispecific binding molecules. In some implementations, the multispecific binding molecule is a multispecific antibody. In some implementations, the bispecific binding molecule is a bispecific antibody.

The term "bispecific antibody", also referred to as "bifunctional antibody conjugate", refers to a conjugate formed by a first antibody (fragment) and a second antibody (fragment) via a conjugation arm, and the conjugate maintains the activities of respective antibodies, thus to be bifunctional and bispecific. In an implementation, provided herein is a bispecific antibody having the binding specificity to C-Met and the binding specificity to the second antigen.

The term "multispecific antibodies" comprise, for example, bispecific antibodies, trispecific antibodies which are antibodies having three different antigen-binding specificities, and tetraspecific antibodies which are antibodies having four different antigen-binding specificities. In some implementations, the multispecific antibody has the binding specificity to c-Met and one or more binding specificities to other antigens.

The term "intact antibody" or "full-length antibody" refers to an antibody comprising an antigen-binding variable region as well as a light chain constant region (CL) and heavy chain constant regions, CH1, CH2, and CH3. The constant regions may be native sequences (for example, human native constant region sequences) or amino acid sequence variants thereof. Preferably, the intact antibody is an intact antibody having one or more effector functions. In the present disclosure, a "humanized" form of a non-human (for example, murine) antibody refers to a chimeric antibody that comprises a minimal amount of non-human immunoglobulin sequence. Most humanized antibodies are recipient human immunoglobulins in which residues of the hypervariable region are substituted with residues of a non-human (such as mouse, rat, rabbit, or non-human primate) hypervariable region (donor antibody) having the desired specificity, affinity, and function. In some implementations, residues of the framework region (FR) of the human immunoglobulin are substituted with non-human residues. In addition, the humanized antibodies can further comprise residues that are not present in the recipient antibody or in the donor antibody. These modifications are made to further optimize antibody performance. The humanized antibody generally comprises at least one, and usually two, variable region(s), in which all or substantially all hypervanable loops correspond to those of a non-human immunoglobulin, while the FRs are completely or substantially completely sequences of a human immunoglobulin. The humanized antibody may further comprise at least a portion of an immunoglobulin constant region (Fc, usually a human immunoglobulin Fc).

The intact antibodies can be divided into different "classes" depending on the amino acid sequence of the heavy chain constant region. The main five classes are IgA, IgD, IgE, IgG, and IgM, wherein several classes can be further divided into different "subclasses" (isotypes), such as IgG1, IgG2, IgG3, IgG4, IgA 1, and IgA2. The heavy chain constant regions of different classes of antibodies are referred to as α, β, ε, γ, and µ, respectively. The subunit structures and three-dimensional configurations of different classes of immunoglobulins are well known in the art.

As used herein, the CDRs of the antibody or antigen-binding fragment thereof of the present disclosure can be determined according to various definition schemes known in the art. In certain implementations, the CDRs comprised in the antibody or antigen-binding fragment thereof of the present disclosure are preferably determined according to the definition scheme of Kabat, Chothia, AbM, or IMGT.

As used herein, the term "framework residue region" or "FR residue" refers to those amino acid residues in the antibody variable regions except for the CDR residues as defined above.

The twenty conventional amino acids involved herein are written following conventional usage. See, for example, Immunology-A Synthesis (2nd Edition, E. S. Golub and D. R. Gren, Eds., Sinauer Associates, Sunderland, Mass. (1991)), which is incorporated herein by reference. Herein, the terms "polypeptide" and "protein" have the same meaning and can be used interchangeably. In addition, in the present disclosure, amino acids are generally represented by one-letter and three-letter abbreviations well known in the art. For example, alanine can be represented by A or Ala; arginine can be represented by R or Arg; glycine can be represented by G or Gly; and glutamine can be represented by Q or Gln.

As used herein, the term "immunoconjugate" herein means that an effector molecule is attached to an antibody or antigen-binding fragment thereof via a linker, such that the antibody or antigen-binding fragment thereof can serve as a vector for targeted transport of the effector molecule to a target site. The term "effector molecule" refers to the active moiety that conjugates the antibody or antibody fragment of the present invention, and may comprise any moiety used to attach to the antibody or antibody fragment. In some implementations, the effector molecule may be a drug such as a small molecular drug, DNA, RNA, an enzyme, or a polypeptide. In some implementations, the immunoconjugates encompass the antibody-drug conjugates (ADCs). The effector molecule or the active moiety suitable for attachment to an antibody comprises an anti-tumor agent, an immunomodulatory agent, a biological response modifier, a lectin, a cytotoxic drug, a chromophore, a fluorophore, a chemiluminescent compound, an enzyme, a metal ion, and any combination thereof. In some implementations, the immunoconjugate of the present invention is an antibody-drug conjugate, that is, ADC.

The term "immunomodulatory agent" used herein refers to a natural or synthetic active agent or drug that inhibits or modulates (for example, activates) an immune response. The immune response may be a humoral response or a cellular response. The immunomodulatory agents comprise immunosuppressive agents. In some implementations, the immunomodulatory agents of the present invention comprise immune checkpoint inhibitors or immune checkpoint agonists.

As used herein, the term "prophylaxis" refers to a method performed to prohibit or delay the occurrence of diseases, conditions, or symptoms (such as tumors and infectious diseases) in a subject. As used herein, the term "treatment" refers to a method performed to obtain beneficial or desired clinical results. For the objectives of the present disclosure, the beneficial or desired clinical results comprise, but are not limited to, alleviation of symptoms, reduction in the extent of the diseases, stabilization (that is, no worsening) of the diseases, delaying or slowing the progression of the diseases, ameliorating or alleviating the status of the diseases, and relief of (whether partial or all) symptoms, whether detectable or undetectable. In addition, the "treatment" may further refer to prolonging overall survival compared to expected overall survival (if no treatment is accepted).

As used herein, the term "subject" refers to a mammal, such as a primate mammal, such as a non-human primate mammal or a human. In certain implementations, the subject (for example, human) has a tumor and an infectious disease, or is at the risk of suffering from the foregoing diseases.

As used herein, the term "effective amount" refers to an amount sufficient to obtain, at least in part, the desired effect. For example, an effective amount for disease prophylaxis (such as tumors and infectious diseases) refers to an amount sufficient to prevent, prohibit, or delay the occurrence of diseases (such as tumors and infectious diseases); and an effective amount for disease treatment refers to an amount sufficient to cure or at least partially prohibit diseases and complications thereof in patients who already have the diseases. Assays of such effective amounts are well within the capabilities of those skilled in the art. For example, the amount effective for therapeutic purposes will depend on the severity of the disease to be treated, the overall status of the patient's own immune system, general conditions of the patient such as age, weight, and gender, the administration manner of drugs, other treatments administered concurrently, and the like.

The term "pharmaceutically acceptable excipients" refers to a diluent, an adjuvant (such as Freund's adjuvant (complete and incomplete)), an excipient, a carrier, a stabilizer, or the like, which are administered with an active substance.

The term "pharmaceutical composition" refers to such a composition which is present in such form as to permit the biological activity of an active ingredient comprised therein to be effective, and which comprises no additional components that are unacceptably toxic to a subject to which the pharmaceutical composition is administered.

The term "pharmaceutical combination or combination product" refers to a non-fixed combination product or a fixed combination product, comprising but not limited to the pharmaceutical kit/kit and the pharmaceutical composition. The term "non-fixed combination" means that the active ingredients (for example, (i) the antibodies of the present invention and (ii) additional therapeutic agents) are administered to a patient in separate entities simultaneously, without specific time limitations, or at the same or different time intervals and sequentially, wherein such administration provides two or more active agents of prophylactically or therapeutically effective levels in the patient. The term "fixed combination" means that two or more active agents are administered simultaneously to a patient in the form of a single entity. The dosages and/or time intervals of the two or more active agents are preferably selected, such that the combined use of the components can produce an effect in treating a disease or condition that is greater than that achieved by using any component alone. Each component may be in a separate formulation form, and the formulation forms may be the same or different.

The term "combination therapy" refers to the administration of two or more therapeutic agents or treatment modalities to treat the disease described herein. Such administration comprises co-administration of the therapeutic agents in a substantially simultaneous manner, for example, in a single capsule having a fixed ratio of the active ingredients. Alternatively, such administration comprises co-administration of the individual active ingredients in a plurality of or separate containers (such as tablets, capsules, powders, and liquids). The powders and/or liquids can be reconstituted or diluted to a desired dosage prior to administration. In addition, such administration further comprises using each type of therapeutic agent at approximately the same time or at different times in a sequential manner. In any case, the treatment regimen will provide beneficial effects of the drug combination in treating the combinations or disorders described herein.

The term "label" used herein refers to a compound or composition that is conjugated or fused directly or indirectly to a reagent (such as a polynucleotide probe or an antibody) and facilitates detection of the reagent to which it is conjugated or fused. The label may itself be detectable (such as radioisotope labels or fluorescent labels) or, in the case of an enzymatic label, may catalyze chemical alteration of a substrate compound or composition which is detectable. The term is intended to encompass direct labeling of a probe or antibody by conjugating (that is, physically linking) a detectable substance to the probe or antibody, as well as indirect labeling of the probe or antibody by reacting with another reagent that is directly labeled.

An "isolated" antibody or molecule is such an antibody or a molecule that has been separated from a component of its natural environment. In some implementations, an antibody or a molecule is purified to greater than 95% or 99% purity as determined by, for example, electrophoresis (such as SDS-PAGE, isoelectric focusing (IEF), capillary electrophoresis) or chromatography (such as ion exchange or reverse phase HPLC).

"Identity percentage (%)" of an amino acid sequence refers to a percentage of amino acid residues in a candidate sequence that are identical to the amino acid residues of the specific amino acid sequence described in this specification when the candidate sequence is aligned with the specific amino acid sequence described in this specification and a blank position has been introduced, if necessary, to achieve the maximum sequence identity percentage, without considering any conservative substitutions as a portion of the sequence identity. In some implementations, a variant of the antibody molecule of the present invention is considered in the present invention, the variant has a comparable degree of identity relative to the antibody molecule and sequence thereof specifically disclosed herein, for example, at least 80%, 85%, 90%, 95%, 97%, 98%, 99% or higher identity. The variant may comprise conservative changes.

The term "about" when used in combination with a numerical value is meant to encompass a numerical value within a range from a lower limit that is 5% less than a specified numerical value to an upper limit that is 5% greater than the specified numerical value.

As used herein, the term "and/or" means any one of the options or two or more of the options.

As used herein, the term "comprise" or "include" means the inclusion of the described elements, integers or steps, but not the exclusion of any other elements, integers or steps. When the term "comprise" or "include" is used herein, combinations of the described elements, integers, or steps are also encompassed unless otherwise indicated. For example, when reference is made to an antibody variable region that "comprises" a specific sequence, it is also intended to encompass an antibody variable region consisting of the specific sequences.

The term "pharmaceutically acceptable" means that molecular entities, molecular fragments, or compositions do not produce an adverse, allergic, or other untoward reactions when properly administered to animals or humans. Specific examples of some substances that can serve as pharmaceutically acceptable carriers or components thereof comprise sugars (such as lactose), starch, cellulose and derivatives thereof, vegetable oils, gelatin, polyols (such as propylene glycol), alginic acid, and the like.

The term "drug-to-antibody ratio" or "DAR" refers to the quantitative ratio of the drug moiety (D) conjugated to the antibody moiety (Ab) described herein to the antibody moiety. The DAR of an ADC can range from 1 to 20, but depending on the number of attachment sites on the antibody, and higher loadings are also possible. The term DAR can be used when referring to the quantity of drugs loaded onto a single antibody, or alternatively, when referring to the average or mean DAR of a group of ADCs. The DAR can also be calculated as the average DAR of a molecular population in the product, that is, an overall ratio (molar ratio) of the drug moiety (D) conjugated to the antibody moiety (Ab) described herein to the antibody moiety in the product, which is detected by a detection method (for example, by a conventional method such as mass spectrometry, an ELISA assay, electrophoresis, and/or HPLC), and this DAR is referred to as the average DAR herein. In some implementations, the antibody-drug conjugate of the present disclosure have an average DAR value of 1.0-20.0, such as 1.0-18.0, 1.0-16.0, 2.0-14.0, 3.0-12.0, 4.0-10.0, 5.0-9.0, 6.0-8.0, 1.0-8.0, and 2.0-6.0, such as 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 12.0, and 16.0, with two of these values used as the endpoints of the range.

In the assay process of the DAR value by mass spectrometry, the antibody has been reduced to isolated heavy and light chains, and DAR1 represents a conjugate comprising the light chain or heavy chain conjugated to 1 toxin molecule; DAR2 represents a conjugate comprising the light chain or heavy chain conjugated to 2 toxin molecules; and DAR3 represents a conjugate comprising the light chain or heavy chain conjugated to 3 toxin molecules.

The term "drugs" refer to chemical substances that can change or identify the physiological functions and pathological conditions of an organism, and can be used to prevent, diagnose, and treat diseases; and especially, comprise substances that inhibit or prevent cell functions and/or cause cell death or destruction. The drugs comprise cytotoxic agents, and especially small molecular cytotoxic agents. There is no strict boundary between the drugs and poisons, wherein poisons refer to chemical substances that are toxic to an organism and harm human health in small dosages, and an excessive dosage of any drug can produce toxic reactions.

The cytotoxic agents refer to substances that inhibit or prevent cell functions and/or cause cell death or destruction. In principle, cytotoxic drugs can kill tumor cells at any high enough concentration. However, due to the lack of specificity, while killing tumor cells, they will also cause apoptosis of normal cells, resulting in serious side effects. The cytotoxic agents comprise toxins, such as small molecular toxins or enzymatically active toxins derived from bacteria, fungi, plants, or animals, radioactive isotopes (such as At²¹¹, I¹³1, I¹²⁵ , Y⁹⁰, Re¹⁸⁶, Re¹⁸⁸ , Sm¹⁵3, Bi²¹², P³², and radioactive isotopes of Lu), chemotherapeutic drugs, antibiotics, and nucleolytic enzymes. In addition, the cytotoxic agents comprise, but not limited to, a DNA topoisomerase inhibitor (for example, a camptothecin-based biologically active molecule, such as camptothecin, DXD, camptothecin with a substitutent modified or DXD with a substitutent base modified, hydroxycamptothecin, 9-aminocamptothecin, SN-38, irinotecan, topotecan, belotecan, and rubitecan) or a tubulin inhibitor (for example, an MMAF-like tubulin inhibitor, and an MMAE-like tubulin inhibitor).

To avoid ambiguity, the "drug" that can be a component of an ADC does not only refer to a "pharmaceutical" that has been approved by medical regulatory authorities, but also comprises any compound with a potential therapeutic biological activity in clinical practice, or in R&D and academic research. In addition, it should be understood that the "drug" has a different meaning from the medicine in "use in the preparation of a medicine". It should be understood that, to be attached to a linking body, a drug molecule may need to be functionalized or derivatized, and the compounds thus obtained are also comprised in the category of the drug of the present disclosure.

In the present disclosure, the "active drug unit" is a portion of the antibody-drug conjugate (also referred to as ADC) except for the antibody and the linking group of the present disclosure, which is from the drug defined as above. For convenience, the "active drug unit" in the ADC of the present disclosure may be directly called by the name of the foregoing drug, which is understood by those skilled in the art.

As used in the present disclosure, the term "pharmaceutically acceptable salt" refers to a salt that retains the biological effects and properties of the antibody-drug conjugate or drug-linking body conjugate of the present disclosure, and the salt is not undesirable biologically or in other aspects. The conjugates of the present disclosure (comprising the antibody-drug conjugates and the drug-linking body conjugates) may be present in the form of their pharmaceutically acceptable salts, comprising acid addition salts and base addition salts. In the present disclosure, the pharmaceutically acceptable acid addition salts refer to salts formed by the conjugates in the present disclosure and organic or inorganic acids comprising, but not limited to, hydrochloric acids, sulfuric acids, hydrobromic acids, hydroiodic acids, phosphoric acids, nitric acids, perchloric acids, acetic acids, oxalic acids, maleic acids, fumaric acids, tartaric acids, benzenesulfonic acids, methanesulfonic acids, salicylic acids, succinic acids, citric acids, lactic acids, propionic acids, benzoic acids, p-Toluenesulfonic acids, malic acids, and the like. The pharmaceutically acceptable base addition salts refer to salts formed by the conjugates in the present disclosure and organic or inorganic bases comprising, but not limited to, alkali metal salts, such as lithium, sodium, or potassium salts; alkalineearth metal salts, such as calcium or magnesium salts; and organic base salts, such as ammonium salts formed by organic bases containing N groups.

The pharmaceutically acceptable salts can be obtained using standard procedures well known in the art, for example, by reacting a sufficient amount of a basic compound with a suitable acid that provides a pharmaceutically acceptable anion.

As used in the present disclosure, the term "stereoisomer" refers to an isomer formed due to at least one asymmetric center. A compound with one or more (such as one, two, three, or four) asymmetric centers can produce a racemic mixture, a single enantiomer, a diastereomer mixture, and an individual diastereomer. A specific individual molecule may also be present as a geometric isomer (cis/trans). Similarly, the compounds of the present disclosure may be present as mixtures of two or more structurally different forms (commonly referred to as tautomers) in rapid equilibrium. Representative examples of the tautomers comprise keto-enol tautomers, phenol-keto tautomers, nitroso-oxime tautomers, imine-enamine tautomers, and the like. It should be understood that the scope of the present disclosure encompasses all such isomers or mixtures thereof in any ratio (such as 60%, 65%, 70%, 75%, 80%, 85%, 90%, 95%, 96%, 97%, 98%, 99%).

The carbon-carbon bonds of the compounds of the present disclosure may be depicted using solid lines (-), solid wedges, or dashed wedges in the present disclosure. The use of a solid line to depict a bond attached to an asymmetric carbon atom is intended to indicate that all possible stereoisomers (such as particular enantiomer and racemic mixtures) at that carbon atom are comprised. The use of a solid or dashed wedge to depict a bond attached to an asymmetric carbon atom is intended to indicate that the stereoisomer shown is present. Unless otherwise indicated, the compounds of the present disclosure are intended to be present in the form of stereoisomers (comprising cis- and transisomers, optical isomers (such as R and S enantiomers), diastereomers, geometric isomers, rotational isomer, conformational isomers, atropisomers, and mixtures thereof). The compounds of the present disclosure may exhibit more than one type of isomerization and consist of mixtures thereof (such as racemic mixtures and diastereomeric pairs).

The present disclosure further comprises all pharmaceutically acceptable isotopic compounds, which are identical to the compounds of the present disclosure, except that one or more atoms are substituted with an atom having the same atomic number, but an atomic mass or mass number different from the atomic mass or mass number which predominates in nature. Examples of isotopes suitable for inclusion in the compounds of the present disclosure comprise, but are not limited to, isotopes of hydrogen (such as ²H, ³H); isotopes of carbon (such as ¹¹C, ¹³C, and ¹⁴C); isotopes of chlorine (such as ³⁶Cl); isotopes of fluorine (such as ¹⁸F); isotopes of iodine (such as ¹²³I and ¹²⁵I); isotopes of nitrogen (such as ¹³N and ¹⁵N); isotopes of oxygen (such as ¹⁵O, ¹⁷O, and ¹⁸O); isotopes of phosphorus (such as ³²P); and isotopes of sulphur (such as ³⁵S).

The compounds of the present disclosure may be present in the form of solvates (preferably hydrates), wherein the compounds of the present disclosure comprise polar solvents as structural elements of the crystal lattices of the compounds, such as, especially, water, methanol, or ethanol. The amount of the polar solvent, especially water, may be present in a stoichiometric ratio or a nonstoichiometric ratio.

Also comprised within the scope of the present disclosure are metabolites of the compounds of the present disclosure, that is, substances formed *in-vivo* upon administration of the compounds of the present disclosure. Such products may result, for example, from oxidation, reduction, hydrolysis, amidation, deamidation, esterification, de-esterification, and enzymatic cleavage, of the administered compounds. Therefore, the present invention comprises the metabolites of the compounds of the present disclosure, comprising compounds prepared by a method of contacting the compounds of the present disclosure with a mammal for a time sufficient to produce metabolites thereof.

The present disclosure further comprises within its scope prodrugs of the compounds of the present disclosure. Usually, such prodrugs will be functional group derivatives of the compounds that are readily converted *in-vivo* to the desired therapeutically active compounds. Therefore, in these cases, the term "administration" used in the treatment methods of the present disclosure shall comprise the treatment of various diseases or conditions with prodrug forms of one or more of the claimed compounds, but the prodrug forms are converted *in-vivo* to the foregoing compounds after administration to an individual. For example, a general method for selecting and preparing suitable prodrug derivatives are described in "Design of Prodrug", ed. H. Bundgaard, Elsevier, 1985.

In the present disclosure, pharmaceutically acceptable excipients refer to the excipients and additives used in the production and dispensing of pharmaceuticals, and refer to substances, except for the active ingredients, that have been reasonably evaluated in terms of safety and are comprised in pharmaceutical preparations. In addition to figuration, serving as carriers, and improving stability, the pharmaceutically acceptable excipients or excipients also have important functions such as solubilization, cosolvency, and sustained and controlled release, and are important ingredients that may affect the quality, safety, and effectiveness of pharmaceuticals, which can be divided, based on the origins thereof, into natural products, semi-synthetic products, and full-synthetic products, and can be divided, based on the functions and use thereof, into: solvents, propellants, solubilizers, cosolvents, emulsifiers, colorants, binders, disintegrants, fillers, lubricants, wetting agents, osmotic pressure regulatory agents, stabilizers, glidants, corrigents, preservatives, suspending agents, coating materials, fragrances, anti-adhesive agents, antioxidants, chelants, penetration enhancers, pH regulatory agents, buffers, plasticizers, surface active agents, foaming agents, defoaming agents, thickeners, inclusion agents, humectants, absorbents, diluents, flocculants and deflocculants, filter aids, release retardants, and the like; and can be divided, based on the routes of administration thereof, oral administration, injection, mucosal, transdermal, or topical administration, nasal or oral inhalation administration, ocular administration, and the like. The same pharmaceutically acceptable supplementary material or excipient can be used in pharmaceutical preparations of different routes of administration, and have different effects and use. For using the pharmaceutically acceptable excipients and use thereof, see also "Handbook of Pharmaceutical Excipients", 8th Edition, R.C. Rowe, P.J. Seskey and S.C. Owen, Pharmaceutical Press, London, Chicago.

The pharmaceutical composition can be prepared into various appropriate dosage forms based on the route of administration, such as tablets, capsules, granules, oral solutions, oral suspensions, oral emulsions, powders, tinctures, syrups, injections, suppositories, unguents, creams, pastes, ophthalmic preparations, pills, implants agents, aerosols, powder aerosols, sprays, and the like. The pharmaceutical composition or appropriate dosage form may contain 0.01 mg to 1000 mg of the compound (comprising the conjugate) of the present disclosure or a pharmaceutically acceptable salt thereof, appropriately 0.1 mg to 800 mg, preferably 0.5-500 mg, preferably 0.5 to 350 mg, and particularly preferably 1-250 mg. It should be, and sometimes is, feasible to go beyond the above ranges.

The pharmaceutical composition can be administered in the form of an injection, comprising an injection liquid, sterile powders for injection, and a concentrated solution for injection. The available vehicles and solvents are water, Ringer's solutions, and isotonic sodium chloride solutions. In addition, sterile non-volatile oils may be used as solvents or suspending media, such as monoglycerides or diglycerides. The pharmaceutical composition may be administered in the form of an infusion.

In the present disclosure, for "L is attached to the antibody via a sulphur atom", those skilled in the art can understand that the sulphur atom is from the sulfhydryl group comprised in the antibody itself after a disulfide bond is opened up (for example, the disulfide bond can be opened up by reduction of a reducing agent TCEP, to generate the sulfhydryl group -SH), that is to say, -S- between L and Ab is not an externally attached sulphur atom.

As used herein, the term "comprise" or "include" means the inclusion of the described elements, integers or steps, but not the exclusion of any other elements, integers or steps. When the term "comprise" or "include" is used herein, compositions of the described elements, integers, or steps are also encompassed unless otherwise indicated. For example, when reference is made to an antibody variable region that "comprises" a specific sequence, it is also intended to encompass an antibody variable region consisting of the specific sequences.

In the present disclosure, the term "linking group" or "linking body" refers to the fragment that attach the active drug unit (drug molecule) to the antibody moiety. In this regard, the linking body, prior to being attached to the antibody or antigen-binding fragment thereof (that is, a precursor of the linking body), has a functional group that can form a bond with a functional group of the antibody or antigen-binding fragment thereof.

In the present disclosure, the term "antibody-drug conjugate" or "ADC" refers to a substance obtained by attachment of the active drug unit (drug molecule) to the antibody or antigen-binding fragment thereof. In some implementations of the present disclosure, the active drug unit and a targeting moiety are attached via the linking body. The linking body can be broken in a specific environment (such as an intracellular environment with a low pH value) or under a specific action (such as the action of a lysosomal protease), thereby separating a bioactive compound (such as a c-Myc protein degrader) fragment from the targeting moiety or the antibody or antigen-binding fragment thereof. In some implementations of the present disclosure, the linking body comprise a cleavable or non-cleavable unit, such as a peptide or a disulfide bond. In some implementations of the present disclosure, the active drug unit is directly attached to the targeting moiety or the antibody or antigen-binding fragment thereof via a covalent bond, which can be broken under a specific environment or action, thereby partially separating the active drug unit from of the antibody or antigen-binding fragment thereof.

In the present disclosure, the term "alkyl" refers to an optionally substituted straight-chain or branched-chain fully saturated hydrocarbyl, preferably C₁-C₁₀, and more preferably C₁-C₈, C₁-C₆, or C₁-C₄ alkyl. Examples of the alkyl comprise methyl, ethyl, n-propyl, iso-propyl, n-butyl, sec-butyl, iso-butyl, t-butyl, n-pentyl, n-hexyl, n-heptyl, n-octyl, n-nonyl, n-decyl, and the like.

In the present disclosure, the term "aryl" refers to an optionally substituted C₆-C₁₆ aromatic hydrocarbon group having a single ring (such as phenyl) or a condensed ring (such as naphthyl, anthryl, phenanthryl, and fluorenyl), and preferably a C₆-C₁₀ aromatic hydrocarbon group.

In the present disclosure, the term "heteroaryl" refers to an optionally substituted 5-16-membered aromatic group having one or more (such as 1, 2, 3, or 4) heteroatoms selected from N, O, S, or P, preferably a 5-10-membered aromatic group, and more preferably a 5-6-membered aromatic group. Examples of the heteroaryl comprise imidazolyl, pyrazolyl, triazolyl, tetrazolyl, pyrrolyl, furyl, thienyl, oxazolyl, isoxazolyl, thiazolyl, isothiazolyl, pyridyl, pyrazinyl, pyrimidinyl, pyridazinyl, indolyl, azaindolyl (such as 7-azaindolyl), benzimidazolyl, benzopyrazolyl, benzofuryl, benzothienyl, benzothiazolyl, dibenzofuryl, dibenzothienyl, quinolyl, isoquinolinyl, naphthyridinyl, carbazolyl, azacarbazolyl (such as 1-azacarbazolyl, 2-azacarbazolyl, and 1,8-diazacarbazolyl), indolazinyl, azaindolazinyl, phenoxazinyl, phenothiazinyl, and the like.

As used herein, the term "3-6-membered cycloalkyl" or "C₃₋₆ cycloalkyl" refers to saturated cyclic alkyl comprising 3-6 carbon atoms, comprising cyclic propalkyl (that is, cyclopropyl), cyclic butalkyl (that is, cyclobutyl), cyclic pentalkyl (that is, cyclopentyl), and cyclic hexalkyl (that is, cyclohexyl).

As used herein, the term "5-6-membered heterocycle" refers to a ring comprising 5-6 ring atoms (at least one (such as 1, 2, or 3) of the ring atoms is a heteroatom, such as a nitrogen atom, an oxygen atom, or a sulphur atom), comprising but not limited to pyrrolidine, tetrahydrofuran, piperidine, piperazine, tetrahydropyran, and other rings.

As used herein, the term "5-6-membered heterocyclyl" refers to a cyclic group comprising 5-6 ring atoms (at least one (such as 1, 2, or 3) of the ring atoms is a heteroatom, such as a nitrogen atom, an oxygen atom, or a sulphur atom), comprising but not limited to pyrrolidyl, tetrahydrofuryl, piperidinyl, piperazinyl, tetrahydropyranyl, and the like.

The term "antibody-drug conjugate group" refers to a mixture consisting of a set or a group of antibody-drug conjugates, stereoisomers thereof, prodrugs thereof, pharmaceutically acceptable salts thereof, and tautomers thereof, or pharmaceutically acceptable solvates thereof, wherein q of the antibody-drug conjugates may be the same or different. In addition, the antibody-drug conjugate group may also be referred to as a "population of antibody-drug conjugates" or an "antibody-drug conjugate mixture".

### Beneficial effects of the present invention

In some implementations, the antibody targeting c-MET or antigen-binding fragment thereof developed in the present disclosure binds to c-Met with a high affinity, has a high affinity to human and monkey cMet, and has a good binding specificity for no binding to rat or mouse cMet.

In some implementations, the antibody targeting c-MET or antigen-binding fragment thereof developed in the present disclosure have an antagonistic action, rather than an agonistic action, which is antibody that can antagonize HGF-induced c-Met and protein kinase B (PKB) phosphorylation.

In some implementations, compared to a control antibody, such as ABT700, the antibody targeting c-MET or antigen-binding fragment thereof developed in the present disclosure has the efficient endocytic activity, higher affinity, different binding epitopes, and activity of competitively binding to c-Met with an HGF ligand.

In some implementations, the anti-c-MET antibody provided by the present disclosure has an extremely high degree of humanization and/or thermal stability, thus to be druggable and safe to be administered to human subjects without eliciting an immunogenic response.

Therefore, in some implementations, the antibody targeting c-MET or antigen-binding fragment thereof of the present disclosure exhibits the specificity, reduced toxicity, stability, and enhanced physical and functional properties compared to known therapeutic agents.

At the same time, relative to the development of c-MET signal blocking inhibitors, which will only benefit a portion of the total number of patients with tumors expressing c-MET, the c-MET ADCs of the present disclosure can overcome some limitations of the signal blocking c-MET inhibitors, and benefit more patients with tumors with low expression of c-MET.

In some implementations, the antibody-drug conjugate (ADC) of the present disclosure realizes enrichment of the tumor microenvironment, unique *in-vivo* enzymatic cleavage properties of the linker and conjugation manners with the targeting moiety, and screening and verification in combination with a large amount of *in-vivo* and *in-vitro* drug effects, such that a novel antibody bioactive molecular conjugate is obtained. The conjugates obtained in the foregoing way can be used to realize various surprising technical effects as follows:
In some implementations, the conjugate obtained in the foregoing way has a better solubility and excellent chemical stability, for example, the reversible Michael addition reaction caused by the maleimide attachment manner in traditional ADCs does not occur, so a higher DAR value can be obtained, and in some implementations, the DAR value of the conjugate can reach 6-8, or even higher; and
has extremely high conjugation efficiency, and in some implementations, the coupling efficiency can reach or exceed 90%;
therefore, in an implementation, according to the present disclosure, through extensive research, a type of ADCs that have high plasma stability but at the same time can be lysed in the tumor microenvironment (both within tumor cells and outside tumor cells) are discovered, which can realize the release both within tumor cells and in tumor tissues, maximizing the delivery of ADCs to tumor tissues and tumor cells, thereby also exerting the efficacy in tumor treatment, and thus can produce a good anti-tumor effect in tumors with low antigen expression or no antigen expression; and the ADCs of the present disclosure exhibit obvious tumor inhibitory activity on tumor cells with different c-MET expression levels.

In some implementations, after the ADCs of the present disclosure are incubated at 37°C for 504 h, release percentages of toxins in a PBS solution, cyno plasma, and human plasma are < 0.6%, respectively, indicating excellent plasma stability and circulation stability; the conjugates (ADCs) obtained in the foregoing way improve the exposure of an entire ADC molecule in a relatively acidic tumor environment by adjusting the physical and chemical properties of linkers and the overall ADC molecule, so the ADCs have better tumor tissue targeting ability, that is, the ability to enrich in the tumor microenvironment, increasing the intratumoral and blood concentration ratio of bioactive molecules, and reducing the mechanism-related toxicity of the ADC molecules (toxicity produced after ADCs binding to antigens on cell surfaces in non-tumor tissues and endocytosis, or referred to as "on-target toxicity"), and thus have a higher therapeutic index;
in some implementations, the conjugates obtained in the foregoing way have high stability in *in-vivo* circulation, reducing the shedding of drug molecules in non-target tissues, and reducing the "off-target" toxicity caused by the shedding of toxins in non-target tissues;
in some implementations, the bioactive molecules of the conjugates have a higher anti-tumor cell activity, and thus has an excellent by-stander effect, and the ADCs can more effectively kill tumor cells with high antigen expression and tumor cells with low antigen expression or no antigen expression in tumor tissues;
in some implementations, the antibody-drug conjugate of the present disclosure can form, by utilizing the extracellular cleavage ability of the linker thereof in the tumor microenvironment, an antibody-drug conjugate with an antibody without the cell endocytosis ability, and such an antibody-drug conjugate still has a high anti-tumor activity; and
in some implementations, the antibody-drug conjugate of the present disclosure can form, by utilizing the extracellular cleavage ability of the linker thereof in the tumor microenvironment and the enrichment ability in the tumor microenvironment, an antibody-drug conjugate with an antibody without the cell endocytosis ability and an antibody without the ability to bind an antigen outside the tumor cells, and such an antibody-drug conjugate still has a high anti-tumor activity; and
in conclusion, the ADCs of the present disclosure have a significant clinical value.

### BRIEF DESCRIPTION OF THE DRAWINGS

FIG. 1. Cross-detection of humanized antibodies and rat cMet;
FIG. 2. Cross-detection of humanized antibodies and mouse cMet;
FIG. 3. Detection of epitope competition between humanized antibodies and ABT700;
FIG. 4. Humanized antibodies block HGF-induced ERK phosphorylation;
FIG. 5. Humanized antibodies induce cMet protein degradation;
FIG. 6. *In-vivo* efficacy of anti-human cMet ADCs in NCI-H358 CDX models (Test 1);
FIG. 7. Changes of weights of mice during administration processes of anti-human cMet ADCs in NCI-H358 CDX models (Test 1);
FIG. 8. *In-vivo* efficacy of anti-human cMet ADCs in SW480 CDX models;
FIG. 9. Changes of weights of mice during administration processes of anti-human cMet ADCs in SW480 CDX models;
FIG. 10. *In-vivo* efficacy of anti-human c-Met ADCs in NCI-H716 CDX models;
FIG. 11. Changes of weights of mice during administration processes of anti-human cMet ADCs in NCI-H716 CDX models;
FIG. 12. Verification of c-Met target expression levels in different tumor cells;
FIG. 13. *In-vivo* efficacy of anti-human cMet ADCs in NCI-H358 CDX models (Test 2);
FIG. 14. Changes of weights of mice during administration processes of anti-human cMet ADCs in NCI-H358 CDX models (Test 2);
Table 15. *In-vivo* efficacy of anti-human c-Met ADCs in CR5088 PDX models;
FIG. 16. Changes of weights of mice during administration processes of anti-human cMet ADCs in CR5088 PDX models;
FIG. 17. ADCC activity tests of anti-human cMet antibodies and ADCs on MKN45 cells, the upper panel being a test curve of a positive control, and the lower panel being test curves of 45A5G10-Hz antibodies and ADCs thereof;
FIG. 18. CDC activity tests of anti-human cMet antibodies and ADCs on MKN45 cells, the upper panel being a test curve of a positive control, and the lower panel being test curves of 45A5G10-Hz antibodies and ADCs thereof; and
FIG. 19. *In-vivo* efficacy of anti-human c-Met ADCs in MKN45 gastric cancer models.

### DETAILED DESCRIPTION OF EMBODIMENTS

The present disclosure is further described below through the description of specific implementations, but this is not intended to limit the present disclosure. Those skilled in the art can make various modifications or improvements based on the teachings of the present disclosure without departing from the basic idea and scope of the present disclosure. The reagents or instruments used do not indicate the manufacturer, and are all conventional products that are commercially available.

### Sequences and specific information thereof:

| Name/SED ID No. /Sequence | CDR1 IMGT | CDR2 IMGT | CDR3 IMGT | CDR1 Kabat | CDR2 Kabat | CDR3 Kabat | CDR1 Chothia | CDR2 Chothia | CDR3 Chothia |
|---|---|---|---|---|---|---|---|---|---|
| 45A5G10-VL | 20 ESVDD YGVSF | 21 TAS | 22 QQSK EIPPT | 23 RASESV DDYGV SFVN | 24 TASVQES | 22 QQSK EIPPT | 23 RASESV DDYGV SFVN | 24 TASVQ ES | 22 QQSKEI PPT |
| 45A5G10-VH | 13 GFTFSN YW | 14 IRLKSL NYAT | 15 TEEGF AY | 16 NYWM N | 17 QIRLKSL NYATHYA ESVKG | 19 EGFA Y | 25 GFTFSN Y | 53 RLKSL NYA | 19 EGFAY |
| 55A10G6-VL | 33 ENIYGA | 34 GAT | 35 QNVL SEPLT | 36 GASENI YGALN | 37 GATNLA A | 35 QNVL SEPLT | 36 GASENI YGALN | 37 GATNL AA | 35 QNVLS EPLT |
| 55A10G6-VH | 26 GYTFTD YY | 27 IFPGSG NT | 28 AGGD YGGF TY | 29 DYYLN | 30 WIFPGSG NTKYIEK FKG | 32 GDYG GFTY | 54 GYTFT DY | 55 FPGSG N | 32 GDYGG FTY |
| 44H10E8-VL | 45 QSVLYF SNQKN C | 46 WAS | 47 QQYL SSLT | 48 KSSQSV LYFSNQ KNCLA | 49 WASTRES | 50 QQYL SSLT | 48 KSSQSV LYFSNQ KNCLA | 49 WASTR ES | 50 QQYLS SLT |
| 44H10E8-VH | 39 GYTFIS YT | 40 INPSSG YA | 41 TRPY GDFV DWYF DV | 42 SYTIH | 43 YINPSSG YAKNNP KFQD | 44 PYGD FVDW YFDV | 62 GYTFIS Y | 60 NPSSG Y | 44 PYGDF VDWYF DV |
| 51D5B2-V L | 20 ESVDD YGVSF | 21 TAS | 22 QQSK EIPPT | 23 RASESV DDYGV SFVN | 24 TASVQES | 22 QQSK EIPPT | 23 RASESV DDYGV SFVN | 24 TASVQ ES | 22 QQSKEI PPT |
| 51D5B2-V H | 13 GFTFSN YW | 14 IRLKSL NYAT | 15 TEEGF AY | 16 NYWM N | 17 QIRLKSL NYATHYA ESVKG | 19 EGFA Y | 25 GFTFSN Y | 53 RLKSL NYA | 19 EGFAY |
| 45A5G10-hz VL | 20 ESVDD YGVSF | 21 TAS | 22 QQSK EIPPT | 23 RASESV DDYGV SFVN | 24 TASVQES | 22 QQSK EIPPT | 23 RASESV DDYGV SFVN | 24 TASVQ ES | 22 QQSKEI PPT |
| 45A5G10-hz VH | ESVDD YGVSF 13 GFTFSN YW | 14 IRLKSL NYAT | 15 TEEGF AY | 16 NYWM N | 18 QIRLKSL NYATHYA QSVKG | 19 EGFA Y | 25 GFTFSN Y | 53 RLKSL NYA | 19 EGFAY |
| 55A10G6-hz VL | 33 ENIYGA | 34 GAT | 35 QNVL SEPLT | 36 GASENI YGALN | 37 GATNLA A | 35 QNVL SEPLT | 36 GASENI YGALN | 37 GATNL AA | 35 QNVLS EPLT |
| 55A10G6-hz VH | 26 GYTFTD YY | 27 IFPGSG NT | 28 AGGD YGGF TY | 29 DYYLN | 31 WIFPGSG NTKYSQ KFQG | 32 GDYG GFTY | 54 GYTFT DY | 55 FPGSG N | 32 GDYGG FTY |

| SEQ ID No. | Name | Sequence |
|---|---|---|
| 1 | 45A5G10-VL | IVMTQSPASLPVSLGQRATISCRASESVDDYGVSFVNWFQQKPGQPPKLLIYT ASVQESGVPARFSGSGSGTDFSLNIHPLEEDDTAMYFCQQSKEIPPTFGGGTKL EIK |
| 2 | 45A5G10-VH | EVRLEESGGGFVQPGGSMKISCVASGFTFSNYWMNWVRQSPEKGLEWVAQI RLKSLNYATHYAESVKGRFTISRDDSRNSVYLQMNNLRAEDAGIYYCTEEGF AYWGRGTLVTVSA |
| 3 | 55A10G6-VL | DIQMTQSPPSLSASVGETVTITCGASENIYGALNWYQRKQGKSPQLLIYGATN LAAGMSSRFSGSGSGRQFSLKISSLHPDDVATYYCQNVLSEPLTFGAGTKLEL K |
| 4 | 55A10G6-VH | QIQLQQSGPELVKPGASVKISCKASGYTFTDYYLNWVRQRPGQGLEWIGWIF PGSGNTKYIEKFKGKATLTVDTSSSTAYMLLSSLTSEDSAVYFCAGGDYGGFT YWGQGTLVAVSA |
| 5 | 44H10E8-VL | NIMMTQSPSSLAVSAGEKVTMNCKSSQSVLYFSNQKNCLAWYQQKPGQSPK LLIYWASTRESGVPDRFTGSGSGTDFTLTISSVQADDLAVYYCQQYLSSLTFGA GTKLELK |
| 6 | 44H10E8-VH | QVHLQQSGAELARPGASVKMSCKASGYTFISYTIHWVKQSPGQGLEWIGYIN PSSGYAKNNPKFQDKATLTADTSSTTAYIQLSSLTSEDSAVYYCTRPYGDFVD WYFDVWGTGTTVTVSS |
| 7 | 51D5B2-VL | IVMTQSPASLPVSLGQRATISCRASESVDDYGVSFVNWFQQKPGQPPKLLIYT ASVQESGVPARFSGSGSGTDFRLNIHPLEEDDTAMYFCQQSKEIPPTFGGGTK LEIK |
| 8 | 51D5B2-VH | EVRLEDSGGGFVQPGGSMKISCVASGFTFSNYWMNWVRQSPEKGLEWVAQI RLKSLNYATHYAESVKGRFTISRDDSRNSVYLQMNNLRAEDAGIYYCTEEGF AYWGRGTLVTVSA |
| 9 | 45A5G10-hz VL | DIVMTQSPSSLAVSLGQRATINCRASESVDDYGVSFVNWFQQKPGQPPKLLIY TASVQESGVPDRFSGSGSGTDFTLTISSLQAEDVAVYFCQQSKEIPPTFGQGTK VEIK |
| 10 | 45A5G10-hz VH | EVQLVQSGGGLVQPGGSLRLSCAASGFTFSNYWMNWVRQAPGKGLEWVAQI RLKSLNYATHYAQSVKGRFTISRDDAKNSVYLQMNSLRAEDTAVYYCTEEGF AYWGQGTLVTVSS |
| 11 | 55A10G6-hz VL | DIQMTQSPSSLSASVGDRVTITCGASENIYGALNWYQQKPGKAPKLLIYGATN LAAGVPSRFSGSGSGRDFTLTISSLQPEDFATYYCQNVLSEPLTFGQGTKVEIK |
| 12 | 55A10G6-hz VH | QIQLVQSGAEVKKPGASVKVSCKASGYTFTDYYLNWVRQAPGQGLEWMGW IFPGSGNTKYSQKFQGRVTLTVDTSASTAYMELSSLRSEDTAVYYCAGGDYGG FTYWGQGTLVTVSS |
| 51 | IgG1 constant region | |
| 52 | Kappa constant region | |
| 56 | 45A5G10-hz HC | EVQLVQSGGGLVQPGGSLRLSCAASGFTFSNYWMNWVRQAPGKGLEWVAQI RLKSLNYATHYAQSVKGRFTISRDDAKNSVYLQMNSLRAEDTAVYYCTEEGF AYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVV VDVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQ QGNVFSCSVMHEALHNHYTQKSLSLSPGK |
| 57 | 45A5G10-hz LC | DIVMTQSPSSLAVSLGQRATINCRASESVDDYGVSFVNWFQQKPGQPPKLLIY TASVQESGVPDRFSGSGSGTDFTLTISSLQAEDVAVYFCQQSKEIPPTFGQGTK VEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSF NRGEC |
| 58 | 55A10G6-hz HC | QIQLVQSGAEVKKPGASVKVSCKASGYTFTDYYLNWVRQAPGQGLEWMGW IFPGSGNTKYSQKFQGRVTLTVDTSASTAYMELSSLRSEDTAVYYCAGGDYGG FTYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQ QGNVFSCSVMHEALHNHYTQKSLSLSPGK |
| 59 | 55A10G6-hz LC | DIQMTQSPSSLSASVGDRVTITCGASENIYGALNWYQQKPGKAPKLLIYGATN LAAGVPSRFSGSGSGRDFTLTISSLQPEDFATYYCQNVLSEPLTFGQGTKVEIK RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRG EC |
| 61 | Kabat HCDR2 | WIFPGSGNTKYX1X2KFX3G (WIFPGSGNTKYX1X2KFX3G, where X1, X2, and X3 may be any amino acid, for example, X1 is I or S; X2 is E or Q, and/or X3 is K or Q) |
| 38 | Kabat HCDR2 | QIRLKSLNYATHYAXSVKG (QIRLKSLNYATHYAXSVKG, where X may be any amino acid, such as E or Q) |

The present disclosure is described with reference to the following embodiments that are intended to explain, but not to limit, the present disclosure.

Unless otherwise specified, molecular biology experimental methods and immunoassays used in the present disclosure are basically performed in accordance with methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art appreciate that the embodiments describe the present disclosure by way of examples and are not intended to limit the scope of the present disclosure as claimed.

Abbreviations in the present disclosure have the following meanings, and abbreviations for which no meaning is provided have meanings commonly understood in the art:

| | | | |
|---|---|---|---|
| PB/ PBS | Phosphate-buffered saline | TCEP | Tris(2-carboxyethyl)phosphine |
| FA | Formic acid | ACN | Acetonitrile |
| CCK8 | 2-(2-Methoxy-4-nitrophenyl)-3-(4-nitrophenyl)-5-(2,4-disulfophenyl)-2H-tetrazole monosodium salt | | |

### Embodiment 1. Screening and preparation of anti-c-Met antibodies

### 1.1 Antigen information

1) Plasmids: Human cMet cDNA ORF Clone is purchased from Sino Biological, Inc. (Cat. No. HG10463-CH), and then a nucleotide sequence corresponding to a 25-932 amino acid of a human cMet protein extracellular fragment is cloned into pTT5-mFc and pTT5-hFc vectors (from Sichuan Sibowo Biotechnology Co., Ltd.), finally forming expression plasmids that fuse a human IgG Fc fragment and a mouse IgG Fc fragment, respectively.
2) Antigen expression: HEK293E cells (from Sichuan Sibowo Biotechnology Co., Ltd.) are transiently transfected through PEImax (Polysciences, 24765-1), and after 7 days of expression, hcMet-ECD-mFc and hcMet-ECD-hFc antigen proteins are obtained through purification using a ProA filler (GE, Mabselect XL).
3) Commercial antigen proteins: a human cMet ectodomain recombinant protein (Human HGF R / c-MET Protein, Fc Tag) from Acrobiosystems Co., Ltd. (Cat. No.: MET-H5256), a human cMet ectodomain recombinant protein (Human HGF R / c-MET Protein, His Tag) from Acrobiosystems Co., Ltd. (Cat. No.: MET-H5227), a monkey cMet ectodomain recombinant protein (c-MET Protein, Cynomolgus, Rhesus, Recombinant (His Tag)) from Sino Biological, Inc. (Cat. No.: 90304-C08H), a rat cMet ectodomain recombinant protein (Rat-cMet-hFc, c-MET Protein, Rat, Recombinant (hFc Tag)) from Sino Biological, Inc. (Cat. No.: 80004-R02H), a mouse cMet ectodomain recombinant protein (mouse cMet-ECD-His, c-MET Protein, Mouse, Recombinant (ECD, His Tag)) from Sino Biological, Inc. (Cat. No.: 50622-M08H), and a human HGF recombinant protein (HGF Protein, Human, Recombinant) from Sino Biological, Inc. (Cat. No.: 10463-HNAS).

### 1.2 Immunization and antibody screening

4 mice, all female, are selected from each of strains of CD1 (Vital River), KM (Vital River), and Balb/c (Gempharmatech Co., Ltd.), and are enrolled into a group at about 6 weeks old. Immunization is performed using cMET-His and hcMET-mFc (Sichuan Sibowo Biotechnology Co., Ltd.), and after 2 times of booster immunization, serum titers are detected through ELISA. For a mouse with a higher ELISA protein titer, an affinity titer of tumor cells MKN-45 and a competition FACS titer of MKN45-HGF are detected through FACS. Finally, 1 mouse from each of CD1, KM, and Balb/c is selected, and the spleen and lymph node thereof are used to prepare cell suspensions for hybridoma fusion with SP2/0 mouse myeloma cells. ELISA preliminary screening is performed through human hMET-ECD-hFc antigen proteins, and after the preliminary screening, positive hybridomas are selected to be cloned. Hybridoma cells are cultured in a serum-free medium, and a collected monoclonal supernatant is purified through the ProA filler to obtain murine-derived antibodies. Affinities of the murine-derived antibodies to human MET-ECD-hFc and cyno MET-ECD-hFc proteins are detected through ELISA, and the affinity of the murine-derived antibodies to the MKN45 tumor cells and blocking of MKN45-HGF ligand competition is detected through FACS. After several rounds of screening, a total of 18 positive clones are obtained, 18 murine-derived antibodies are evaluated, and evaluation results of 4 cloned murine-derived antibodies having a higher affinity are shown in Table 1.

**Table 1. Detection of affinities of anti-cMET murine-derived antibodies and HGF competitiveness**

| Name of Murine-derived Antibody | cMet-ECD-hFc (EC50=ng/ml) | Cyno.cMet-ECD-hFc (EC50=ng/ml) | MKN45 (EC50=ng/ml) | MKN45-HGF competition (IC50=ng/ml) |
|---|---|---|---|---|
| 55A10G6 | 7.701 | 14.9 | 63.08 | 31.28 |
| 45A5G10 | 6.387 | 10.34 | 41.25 | 53.28 |
| 44H10E8 | 15.76 | 25.72 | 149.7 | 88.57 |
| 41F5B2 | 4.807 | ND | 348.5 | No activity |

| | | | | |
|---|---|---|---|---|
| ND indicates not detected | | | | |

To tag sequences of the 4 monoclonal clones 55A10G6, 45A5G10, 51D5B2, and 44H10E8 having the higher affinity, a sequence tagging method is as follows: collecting about 1x10⁵ candidate hybridoma cells, extracting RNAs through Trizol, and then performing reverse transcription using a PrimeScript RT reagent kit through PolyA to obtain cDNA; and separately designing upstream primers in the upstream of a heavy chain and a light chain, and designing downstream primers in a heavy chain CH1 region and a light chain CL region. Products are amplified through PCR, fragment recovery is performed through an agarose gel recovery kit; and a sample is sent to Beijing Tsingke Biotechnology Co., Ltd. for sequencing, and specific sequences of a variable region and a CDR of the murine-derived antibody are shown in a sequence information table.

### 1.3 Antibody humanization

Using a CDR grafting method, first, a human germline sequence with the highest homology to an original murine-derived sequence will be found through a conventional BLAST method as a template; the CDR of the murine-derived antibody is grafted onto the human-derived template to construct a chimera; an FR amino acid in the murine-derived antibody that can retain its original conformation is analysed based on a structure, and the corresponding amino acid in the chimera is subjected to back mutation to be a murine-derived amino acid to retain the original affinity; and calculation and immunogenicity analysis is performed on the constructed humanized antibody to find a high-immunogenic fragment for a substitution of a low-immunogenic fragment. For a substitution of a high-immunogenic site in the CDR, for example, in humanization, for a substitution of 45A5G10 Kabat HCDR2, QIRLKSLNYATHYA**E**SVKG (SEQ ID No. 17) is substituted with QIRLKSLNYATHYA**Q**SVKG (SEQ ID No. 18), for a substitution of 55A10G6 Kabat HCDR2, WIFPGSGNTKY**IE**KF**K**G (SEQ ID No. 30) is substituted with WIFPGSGNTKY**SQ**KF**Q**G (SEQ ID No. 31). Murine-derived antibodies 55A10G6 (55A10G6 for short) and 45A5G10 (45A5G10 for short) are humanized to obtain humanized variable regions 55A10G6-HZ VH, 55A10G6-HZ VL, 45A5G10-HZ VH, and 45A5G10-HZ VL. For specific sequences of a variable region and a CDR of each humanized antibody, refer to the sequence information table, where the CDR is provided according to IMGT, chothia, and Kabat definition schemes.

### 1.4 Expression of anti-c-Met humanized antibodies

A heavy chain variable region 55A10G6-hz vh of the humanized antibody is connected to a heavy chain IgG1 constant region (SEQ ID NO: 51), and a light chain variable region 55A10G6-hz vl of the humanized antibody is connected to a Kappa constant region (SEQ ID NO: 52). Sequences obtained by connections are submitted to General Biosystems Co., Ltd. for gene synthesis, and constructed onto PTT5 vectors after codon optimization; and after being synthesized, plasmids are transfected into HEK293E cells (from Sichuan Sibowo Biotechnology Co., Ltd.) through PEImax and express for 7 days, and then centrifugation is performed to collect a supernatant. The supernatant is purified using the ProA filler. All purified antibodies are ultrafiltered into the PBS buffer, subjected to a concentration assay, and stored at -20 degrees. Antibodies 55A10G6-hz are obtained.

A heavy chain variable region 45A5G10-hz vh of the humanized antibody is connected to a heavy chain IgG1 constant region (SEQ ID NO: 51), and a light chain variable region 45A5G10-hz vl of each humanized antibody is connected to a Kappa constant region (SEQ ID NO: 52). Sequences obtained by connections are submitted to General Biosystems Co., Ltd. for gene synthesis, and constructed onto PTT5 vectors after codon optimization. After being synthesized, plasmids are transfected into HEK293E cells through PEImax and express for 7 days, and then centrifugation is performed to collect a supernatant. The supernatant is purified using the ProA filler. All purified antibodies are ultrafiltered into the PBS buffer, subjected to a concentration assay, and stored at -20 degrees. Antibodies 45A5G10-hz are obtained.

Amino acid sequences of heavy and light variable regions of a control antibody (ABT700 with a sequence from KEGG, ID: D11307) are submitted to General Biosystems Co., Ltd. for gene synthesis, and constructed onto PTT5 vectors after codon optimization. After being synthesized, plasmids are transfected into HEK293E cells through PEImax and express for 7 days, and then centrifugation is performed to collect a supernatant. The supernatant is purified using the ProA filler. All purified antibodies are ultrafiltered into the PBS buffer, subjected to a concentration assay, and stored at -20 degrees.

### Embodiment 2. Evaluation of anti-c-Met antibodies

### 2.1 Evaluation of ELISA affinities of anti-c-Met antibody proteins

Antigen proteins cMet-ECD-his are diluted with a carbonate-buffered saline (CBS) to 1 µg/ml, and then the antigens are coated for subsequent blocking with 2% BSA (in the phosphate-buffered saline PBS) at 37°C for 2 hours, humanized antibodies to be tested that are gradiently diluted (starting at 2 ug/mL for 3-fold dilution with 11 concentration points) are added for incubation at 37°C for 2 hours, HRP-labeled anti-human specific secondary antibodies (Jackson, 115-035-164) are added for incubation at 37°C for 1 hour, a TMB substrate is added for color development, and after the color development is stopped using HCl of 2M, final products are added into a machine to read out absorbance values at 450 nM; and antigen proteins anti-His-Rabbit Fc (from Chengdu NB Biolab Co., Ltd.) are diluted with the CBS to 1 ug/ml, and then the antigens are coated for subsequent blocking with 2% BSA (in the PBS) at 37°C for 2 hours, cyno.cMet-ECD-His of 0.5 ug/ml is added for incubation at 37°C for 2 hours, after 2 hours, humanized antibodies to be tested that are gradiently diluted (starting at 2 ug/mL for 3-fold dilution with 11 concentration points) are added for incubation at 37°C for 2 hours, HRP-labeled anti-human specific secondary antibodies (Jackson, 115-035-164) are added for incubation at 37°C for 1 hour, a TMB substrate is added for color development, and after the color development is stopped using HCl of 2M, final products are added into a machine to read out absorbance values at 450 nM.

The experimental results are shown in Table 2. 2 humanized antibodies have a higher affinity to both human and cyno Met proteins, and have a significantly stronger affinity to the cyno Met than that of ABT700.

**Table 2. Evaluation of ELISA affinities of humanized antibodies**

| Name of Antibody | h.cMet EC50 (ng/mL) | cynoMet EC50 (ng/mL) |
|---|---|---|
| 45A5G10-Hz | 4.375 | 3.911 |
| 55A10G6-Hz | 4.782 | 6.401 |
| ABT700 | 4.148 | 143.4 |

### 2.3 Evaluation of flow affinities of anti-c-Met antibodies

MKN45 cells (Nanjing Cobioer Biosciences Co., Ltd., CBP60488) are collected by trypsin digestion and centrifugation, and are washed three times with a pre-cooled PBS, the cells are resuspended with 1% BSA (in the PBS), and are plated into a 96-well conical bottom plate with 2^10⁵ cells (50 ul) per well, anti-cMet humanized antibodies to be tested are gradiently diluted with 1% BSA starting at 20 ug/ml with a 4-fold dilution gradient and 8 concentration points, 50 ul diluted antibodies are mixed well with the cells in the conical bottom plate for incubation at 4°C for 1 hour, after three times of washing with the pre-cooled PBS, 100 ul of 1% BSA (containing 1 uL anti-human APC fluorescent secondary antibodies, BioLegend, Cat. No. 410712) is added to each well for incubation at 4°C for 0.5 hours, and after three times of washing with the pre-cooled PBS, the cells are resuspended for detection by a flow cytometer (Beckman, cytoflex).

The experimental results are shown in Table 3. 2 humanized antibodies both have a higher affinity to MKN45 cells, and the affinities of the two antibodies to MKN45 are both higher than that of a control antibody ABT700.

**Table 3. Evaluation of flow affinities of humanized antibodies**

| Name of Antibody | MKN45 EC50 (ng/mL) | Maximum signal value |
|---|---|---|
| 45A5G10-Hz | 29.47 | 78766 |
| 55A10G6-Hz | 49.55 | 78639 |
| ABT700 | 100.4 | 70415 |

### 2.4 Evaluation of dynamic affinities of anti-c-Met antibodies

Dynamic affinities of the humanized antibodies are assayed through ForteBio. Experimental steps are as follows: 1. sensor preparation: taking out a ProA sensor, and pre-wetting the sensor with a PBST diluent (pH 7.4) for 10 min; 2. sample dilution: separately diluting antibodies that need to be solidified to 5 ug/ml, antigens h.cMet-ECD-His (Acrobiosystems Co., Ltd., Cat. No.: MET-H5227) starting at 500 nM for 2-fold dilution with 5 concentration points and a 0 concentration point set; 3. setting a program, putting a sensor plate and a sample plate away, starting the program, and the sensor being regenerated with a glycine solution of 20 mM (pH 1.7); 4. using Octet analysis software to analyse data. The experimental results are shown in Table 4. Both the antibody 45A5G10-Hz and the antibody 55A10G6-Hz have a higher dynamic affinity to human cMet proteins.

**Table 4. Detection of dynamic affinities of humanized antibodies**

| Name of Antibody | h.cMet-ECD-His | | |
|---|---|---|---|
| | KD (M) | ka (1/Ms) | kdis (1/s) |
| 45A5G10-Hz | 1.30E-12 | 1.77E+05 | 2.30E-07 |
| 55A10G6-Hz | 1.75E-10 | 3.46E+10 | 6.06E-05 |
| ABT700 | 2.32E-12 | 1.69E+05 | 3.91E-07 |

### 2.5 Quality identification of anti-c-Met antibodies

1) Purities of the humanized antibodies are identified through SEC, and a detection method is as follows:
   instrument: Waters Alliance e2695 HPLC;
   chromatographic column: Thermo MabPac SEC-1, 5 um, 7.8*300 mm;
   mobile phase: Na₂HPO₄ of 61 mmol/L, NaH₂PO₄ of 39 mmol/L, NaCl of 200 mmol/L, 5% IPA; and
   instrument parameters: sample chamber temperature: 8 C; column temperature: 30 C; flow rate: 0.5 ml/min; injection volume: 20 ug; detection wavelength: 280 nm; isocratic operation: 30 min.
2) Hydrophilicity and hydrophobicity of the humanized antibodies are detected through HIC, and a detection method is as follows: Tosoh's hydrophobic chromatographic column (TOSOH Tskgel Buty-NPR (2.5), 4.6*100) is used to perform hydrophilicity and hydrophobicity detection on Agilent HPLC equipment, a mobile phase A is (NH₄)₂SO₄ of 1.5 M, and a mobile phase B is Na₂HPO₄ of 25 mM (pH=7.0) + 25% IPA. Instrument parameters are set as follows: sample chamber temperature: 8°C, column temperature: 30°C, flow rate: 0.5 mL/min, detection wavelength: 280 nm. A humanized antibody sample to be tested is diluted with the mobile phase A to a final concentration of 1 mg/mL, and a sample of 20 uL is injected for gradient elution.
3) Tm values of the humanized antibodies are assayed by DSF, to reflect thermal stability of the antibodies. Experimental steps are as follows: diluting the humanized antibody sample to be tested to 1 mg/mL with the PBS; diluting a SYPRO Orange dye (Thermo#56651) with ddH₂O to 40X; reaction system: 12.5 uL of sample + 2.5 uL of dye of 40X + 5 uL of ddH₂O; sealing a membrane, and performing Short Spin; and detecting through Q-PCR, Q-PCR parameter settings: Target (ROX), program (25°C, 3 min; 1% rate, 95°C; 95°C, 2 min).

Detection results of the SEC, HIC, and Tm values are shown in Table 5. The SEC results show that purities of 2 humanized antibodies are both higher, >90%; the HIC results show that 2 humanized antibodies are weak in binding to the hydrophobic chromatography column, and have short retention times and good hydrophilicity; and the Tm values show that two humanized antibodies both have good thermal stability.

**Table 5. Quality identification of humanized antibodies**

| Antibody | SEC | | | HIC (min) | Tm (°C) |
|---|---|---|---|---|---|
| | HMW% | Main peak % | LMW % | | |
| 45A5G10-Hz | 1.6 | 93.42 | 4.9 | 13.9 | 77.72 |
| 55A10G6-Hz | 3 | 92.66 | 4.2 | 15.0 | 79.62 |

### 2.6 Cross-detection of anti-c-Met antibodies and rat and mouse cMet

Antigen proteins Rat-cMet-hFc are diluted with the CBS to 0.5 ug/ml, and then the antigens are coated for subsequent blocking with 2% BSA (in PBS) at 37°C for 2 hours, humanized antibodies to be tested (biotin-labeled) that are gradiently diluted (starting at 2 ug/mL for 3-fold dilution with 11 concentration points) are added for incubation at 37°C for 2 hours, HRP-labeled anti-biotin secondary antibodies (proteintech, sa00001-0) are added for incubation at 37°C for 1 hour, a TMB substrate is added for color development, and after the color development is stopped using HCl of 2M, final products are added into a machine to read out absorbance values at 450 nM;
and proteins anti-His-hFc (from Chengdu NB Biolab Co., Ltd.) are diluted with the CBS to 0.5 ug/ml, and then are coated for subsequent blocking with 2% BSA (in PBS) at 37°C for 2 hours, mouse cMet-ECD-His of 1 ug/ml is added for incubation at 37°C for 2 hours, after 2 hours, humanized antibodies to be tested that are gradiently diluted (starting at 2 ug/mL for 3-fold dilution with 11 concentration points) are added for incubation at 37°C for 2 hours, HRP-labeled anti-human specific secondary antibodies (Jackson, 115-035-164) are added for incubation at 37°C for 1 hour, a TMB substrate is added for color development, and after the color development is stopped using HCl of 2M, final products are added into a machine to read out absorbance values at 450 nM.

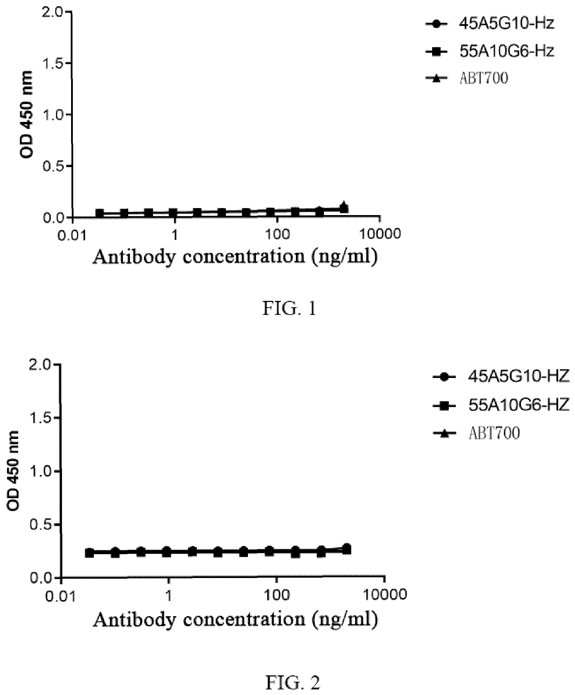

The experimental results are shown in FIG. 1 and FIG. 2. Two humanized antibodies 45A5G10-Hz and 55A10G6-Hz do not cross with the rat or mouse cMet (both do not bind to the rat and mouse cMet).

### 2.7 Evaluation of endocytic activity of anti-c-Met antibodies

MKN45 cells are collected by trypsin digestion and centrifugation, incubated with humanized antibodies to be detected of a concentration of 10 ug/mL at 4°C for 1 hour, and washed 3 times with the PBS, the cells are resuspended with DMEM + 10% FBS that has been pre-warmed at 37°C, and divided into 3 portions for incubation at 37°C for 0, 2, and 4 hours, respectively, after 3 times of washing with a pre-cooled PBS, 1.2 uL of anti-human APC fluorescent secondary antibodies are added for incubation at 4°C for 0.5 hours, and after three times of washing with the PBS, the cells are resuspended and added into a machine. Endocytosis rates are calculated according to the following formula: endocytosis rate (%) = [1 - (an average fluorescence value of a test sample at this time point - an average fluorescence value of a negative control sample at this time point)/(an average fluorescence value of the test sample at 0 hour - an average fluorescence value of the negative control sample at 0 hour)] * 100.

The experimental results are shown in Table 6. Endocytic activity of the antibodies 45A5G10-Hz and 55A10G6-Hz within the same period of time are both higher than that of ABT700, and endocytosis rates can reach about more than 60% in 4 hours.

**Table 6. Detection of endocytic activity of humanized antibodies**

| Endocytosis rate % | 1h | 2h | 4h |
|---|---|---|---|
| 45A5B10-HZ | 11.9 | 28.3 | 64.4 |
| 55A10G6-HZ | 18.4 | 33.0 | 59.1 |
| ABT700 | 7.9 | 19.2 | 44.5 |

### 2.8 Evaluation of epitope competition of anti-c-Met antibodies

Proteins anti-His-Rabbit Fc are coated with the CBS to 0.5 ug/ml, and are blocked with 2% BSA (in PBS) at 37°C for 2 hours, h.cMet-ECD-His of 1 ug/ml is added for incubation at 37°C for 2 hours, after 2 hours, humanized antibodies to be tested that are gradiently diluted (starting at 10 ug/mL for 3-fold dilution with 11 concentration points) are added to a final concentration from 5 ug/ml, for incubation at the room temperature for 0.5 hours, after the incubation is completed, ABT700-Biotin of 200 ng/ml is added to a final concentration of 100 ng/ml, for incubation at the room temperature for 2 hours, after 2 hours, HRP-labeled anti-biotin secondary antibodies (proteintech, sa00001-0) are added for incubation at 37°C for 1 hour, a TMB substrate is added for color development, and after the color development is stopped using HCl of 2M, final products are added into a machine to read out absorbance values at 450 nM.

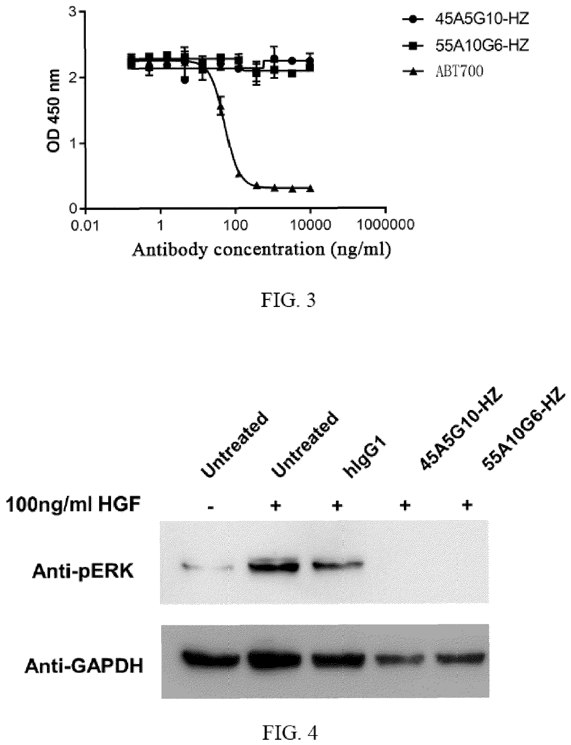

The experimental results are shown in FIG. 3. There is no epitope competition between ABT700 and both humanized antibodies 45A5G10-Hz and 55A10G6-Hz.

### 2.9 Evaluation of competition between anti-c-Met antibodies and HGF ligand

cMET, a tyrosine kinase receptor expressed on a cell membrane, binds to a ligand HGF via a Sema domain, thereby resulting a downstream phosphorylation cascade reaction and ultimately promoting proliferation of cells. Therefore, competitive actions of the humanized antibodies on ligand binding and actions of inhibiting HGF-induced ERK phosphorylation are detected.

MKN45 cells are collected by trypsin digestion and centrifugation, and are washed three times with a pre-cooled PBS, the cells are resuspended with 1% BSA (in PBS), and are plated into a 96-well conical bottom plate with 2^10⁵ cells (50 ul) per well, anti-cMet humanized antibodies are gradiently diluted with 1% BSA, starting at 15 ug/ml to a final concentration from 5 ug/ml with a 3-fold dilution gradient and 8 dilution points, 50 ul diluted antibodies are mixed well with the cells in the conical bottom plate, HGF-His-biotin (from Chengdu NB Biolab Co., Ltd.) is diluted with 1% BSA to 150 ng/ml, and is added into the well plate with 50 ul/well, to make a final concentration of ligands of 50 ng/ml, for incubation at 4°C for 1 hour after fully mixing, after three times of washing with the pre-cooled PBS, 100 ul of 1% BSA (containing 1 uL anti-biotin PE fluorescent secondary antibodies, BioLegend, Cat. No. 405204) is added to each well for incubation at 4°C for 0.5 hours, and after three times of washing with the pre-cooled PBS, the cells are resuspended and detected by a flow cytometer (Beckman, cytoflex).

The experimental results are shown in Table 7. 2 humanized antibodies both have competitive activity with the HGF ligands on the MKN45 cells, and the competitive activity is higher than that of ABT700.

**Table 7. Detection of competition between humanized antibodies and HGF ligand**

| Name of Antibody | MKN45 IC50 (ng/mL) | Maximum inhibition rate % |
|---|---|---|
| 45A5G10-HZ | 35 | 96 |
| 55A10G6-HZ | 35.19 | 97 |
| ABT700 | 167 | 75 |

### 2.10 Anti-c-Met antibodies inhibit HGF-induced ERK phosphorylation

MDA-MB-468 cells (ATCC, Cat. No. HTB-132) are counted by trypsin digestion, the cells are resuspended in an L15 medium to 2*10^5/ml, then the cells are plated into a 24-well plate with 1 ml of cell suspension per well in a total of 4 wells, and then the cells are put back into the 37°C cell culture solution to rest overnight for starvation culture. After 16 hours, cells in 6 wells are grouped and are set as untreated, HGF (Sino Biological, Inc., Cat. No.: 10463-HNAS), HGF + isotype control (hIgG1, prepared by MediLink Therapeutics (Suzhou) Co., Ltd.), HGF + 45A5G10-Hz, and HGF + 55A10G6-Hz, respectively, then the corresponding sterile samples are added to the corresponding cell wells, antibodies to be tested are separately added 15 min in advance (to a final concentration of 15 ug/ml), HGF is added after 15 min to a final concentration of 100 ng/ml, and the cells are put back into the 37°C cell culture solution for 15 min of resting culture. After 15 min, the cells are washed with the PBS for 3 times, and then an SDS lysis buffer is directly added to each well to lyse the cells to obtain proteins. The obtained proteins are subjected to conventional SDS-PAGE and Western blot detection, where detection antibodies for ERK phosphorylation are from CST (Cat. No. #5726S), and detection antibodies of GAPDH are from Sino Biological, Inc. (Cat. No. 100242-MM05), and the dilution ratios of two primary antibodies are both 1:2000, for incubation overnight at 4°C.

The results are shown in FIG. 4. HGF separate treatment can significantly induce ERK phosphorylation of the MDA-MB-468 cells, while adding the antibodies 45A5G10-Hz and 55A10G6-Hz in advance can completely block the HGF-induced ERK phosphorylation.

### 2.11. Humanized antibodies induce cMet protein degradation

MKN-45 cells are counted by trypsin digestion, the cells are resuspended with RPMI 1640 + 10% FBS (fetal bovine serum) + Ps (penicillin-streptomycin) to 2*10^5/ml, then the cells are plated into a 24-well plate with 1 ml of cell suspension per well in a total of 4 wells, and then the cells are put back into the 37°C cell culture solution to rest overnight. After 16 hours, the cells in the 4 wells are grouped and are set as untreated, isotype control (hIgG1), 45A5G10-Hz, and 55A10G6-Hz, respectively, then the corresponding sterile samples are added to the corresponding cell wells, a final concentration of the antibodies is 20 ug/ml, and the cells are put back into the 37°C cell culture solution for 60 hours of resting culture. After 60 hours, the cells are washed with the PBS for 3 times, and then an SDS lysis buffer is directly added to each well to lyse the cells to obtain proteins. The obtained proteins are subjected to conventional SDS-PAGE and Western blot detection, where detection antibodies of cMet are from ProteinTech (Cat. No. 25869-1-AP), and detection antibodies of GAPDH are from Sino Biological, Inc. (Cat. No. 100242-MM05), and the dilution ratios of two primary antibodies are both 1:2000, for incubation overnight at 4°C.

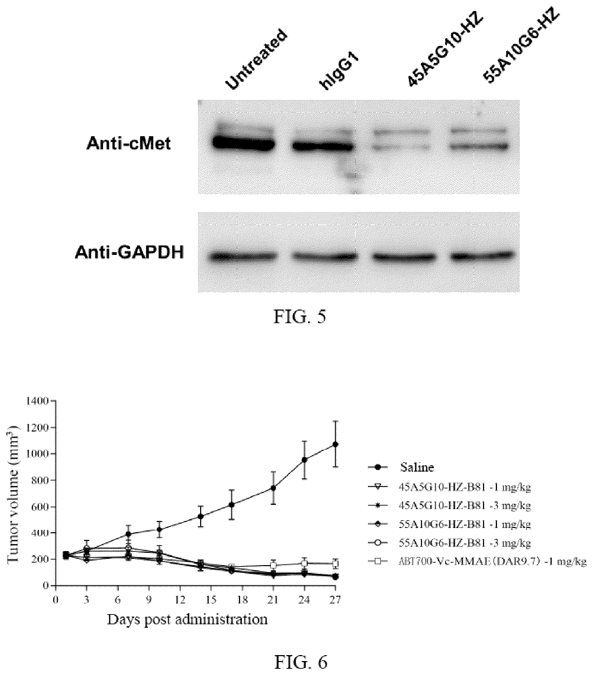

As shown in FIG. 5, the results show that compared to treatment with the isotype control antibody IgG1, the anti-cMet antibodies 45A5G10-Hz and 55A10G6-Hz can significantly induce cMet degradation.

### Embodiment 3. Preparation of anti-c-Met ADCs

### Embodiment 3.1 Preparation of anti-c-MET-B81 ADCs

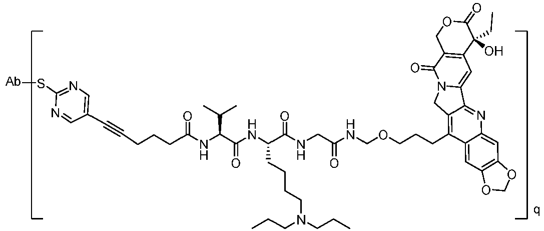

Ab is a c-MET antibody (for example, 45A5G10-Hz and 55A10G6-Hz as described below), and q can be determined based on the data provided below.

### 3.1.1 Preparation of a 45A5G10-HZ-B81 (DAR8) sample

A total of 20 mg of anti-c-Met antibodies 45A5G10-HZ are added to a sodium edetate solution with a final concentration of 1 mM and mixed well, and the pH of the sample is adjusted to 7.5 with a disodium hydrogen phosphate solution of 0.5 M; a TCEP solution of 20 mmol/L that is 6.5 molar equivalents of the antibodies is added, mixed well, and placed at 37°C for 90 minutes; and B81 of 10 mmol/L that is 15 molar equivalents of the antibodies and dissolved in dimethyl sulfoxide (obtained with reference to DL-037 preparation in Embodiment 2.37 of WO2022170971) is added to the foregoing solution system, and mixed well, to rest at 37°C for 3 hours and obtain a conjugated sample, after the reaction is completed, a 30KDa ultrafiltration tube is used to replace the sample into a histidine buffer of 20 mM with a pH of 6.0 and remove low-molecular substances, and finally, the sample is concentrated to obtain a solution containing anti-c-Met antibody ADCs 45A5G10-HZ-B81 (DAR8).

### RP-LC/MS is used to assay the DAR value of the conjugated sample

LC/MS type:

| Name | Manufacturer | Type |
|---|---|---|
| UPLC | AB SCIEX | ExionLC |
| High-resolution mass spectrometer | AB SCIEX | X500B |

Liquid phase parameters

| Chromatographic column | Xbridge Protein BEH SEC (2.5 µm, 4.6*150 mm) | | | | |
|---|---|---|---|---|---|
| Flow rate | 0.3 ml/min | | | | |
| Column Temp | 30°C | | | | |
| Injection volume | 1 µl | | | | |
| Sample chamber temperature | 8°C | | | | |
| Mobile phase | Mobile phase A: 0.1% FA in H₂O | | | | |
| | Mobile phase B: 0.1% FA in ACN | | | | |
| Time/min | 0 | 5 | 7 | 7.1 | 10 |
| A% | 90 | 20 | 20 | 90 | 90 |
| B% | 10 | 80 | 80 | 10 | 10 |

The results show that the light chain of the 45A5G10-HZ-B81 (DAR8) sample is conjugated to 0-1 toxin molecule (ratios of LC and DAR1 are 0% and 100.0%, respectively), and the heavy chain is conjugated to 0-3 toxin molecules (ratios of mAb, DAR1, DAR2, and DAR3 are 0%, 0%, 0%, and 100.0%, respectively), from which the conjugation ratio (DAR value) of the 45A5G10-HZ-B81 (DAR8) sample is calculated to be 8.0. It can be deduced that q is 8.

The foregoing mAb represents an unconjugated monoclonal antibody; LC represents the antibody light chain; HC represents the antibody heavy chain; DAR1 represents a conjugate comprising the light chain or heavy chain conjugated to 1 toxin molecule; DAR2 represents a conjugate comprising the light chain or heavy chain conjugated to 2 toxin molecules; and DAR3 represents a conjugate comprising the light chain or heavy chain conjugated to 3 toxin molecules; wherein the theoretical molecular weight of the monoclonal antibody is calculated based on the G0F glycoform. The same is true for mAb, LC, HC, DAR1, DAR2, and DAR3 below.

### 3.1.2 Preparation of a 45A5G10-HZ-B81 (DAR4) sample

A total of 20 mg of anti-c-Met antibodies 45A5G10-HZ are added to a sodium edetate solution with a final concentration of 1 mM and mixed well, and the pH of the sample is adjusted to 7.5 with a disodium hydrogen phosphate solution of 0.5 M; a TCEP solution of 20 mmol/L that is 2.7 molar equivalents of the antibodies is added, and mixed well, to rest at 5°C for 5 hours; and B81 of 10 mmol/L that is 6.5 molar equivalents of the antibodies and dissolved in dimethyl sulfoxide (obtained with reference to DL-037 preparation in Embodiment 2.37 of WO2022170971) is added to the foregoing solution system, and mixed well, to rest at the room temperature for 1 hour and obtain a conjugated sample, after the reaction is completed, a 30KDa ultrafiltration tube is used to replace the sample into a histidine buffer of 20 mM with a pH of 6.0 and remove low-molecular substances, and finally, the sample is concentrated to obtain a solution containing anti-c-Met antibody ADCs 45A5G10-HZ-B81 (DAR4).

### Native-LC/MS is used to assay the DAR value of the conjugated sample

LC/MS type:

| Name | Manufacturer | Type |
|---|---|---|
| UPLC | AB SCIEX | ExionLC |
| High-resolution mass spectrometer | AB SCIEX | X500B |

Liquid phase parameters

| Chromatographic column | Xbridge Protein BEH SEC (2.5 µm, 4.6*150 mm) |
|---|---|
| Flow rate | 0.2 ml/min |
| Column Temp | 30°C |
| Injection volume | 30ug |
| Sample chamber temperature | 8°C |
| Mobile phase | CH₃COONH₄ of 20 mM |
| Elution mode | isocratic |

The results show that in the 45A5G10-HZ-B81 (DAR4) sample, an entire antibody is conjugated to 0-8 toxin molecules (ratios of mAb, q being 2, q being 4, q being 6, q being 8 are 0%, 22%, 56%, 23%, and 0%, respectively), from which the conjugation ratio (DAR value) of the 45A5G10-HZ-B81 (DAR4) sample is calculated to be 4.0.

### 3.1.3: Preparation of 55A10G6-HZ-B81 (DAR8)

A total of 20 mg of anti-c-Met antibodies 55A10G6-HZ are added to a sodium edetate solution with a final concentration of 1 mM and mixed well, and the pH of the sample is adjusted to 7.5 with a disodium hydrogen phosphate solution of 0.5 M; a TCEP solution of 20 mmol/L that is 6.5 molar equivalents of the antibodies is added, mixed well, and placed at 37°C for 90 minutes; and B81 of 10 mmol/L that is 15 molar equivalents of the antibodies and dissolved in dimethyl sulfoxide is added to the foregoing solution system, and mixed well, to rest at 37°C for 3 hours and obtain a conjugated sample, after the reaction is completed, a 30KDa ultrafiltration tube is used to replace the sample into a histidine buffer of 20 mM with a pH of 6.0 and remove low-molecular substances, and finally, the sample is concentrated to obtain a solution containing anti-c-Met antibody ADCs **55A10G6-HZ-**B81 (DAR8).

The DAR value of the conjugated sample is assayed referring to the foregoing LC/MS in 3.1.1, the light chain of the 55A10G6-HZ-B81 sample is conjugated to 0-1 toxin molecule (ratios of LC and DAR1 are 0% and 100.0%, respectively), and the heavy chain is conjugated to 0-3 toxin molecules (ratios of mAb, DAR1, DAR2, and DAR3 are 0%, 0%, 0%, and 100.0%, respectively), from which the conjugation ratio (DAR value) of the 55A10G6-HZ-B81 sample is calculated to be 8.0. It can be deduced that q is 8.

### Embodiment 3.2 Preparation of anti-c-MET-vc-MMAE

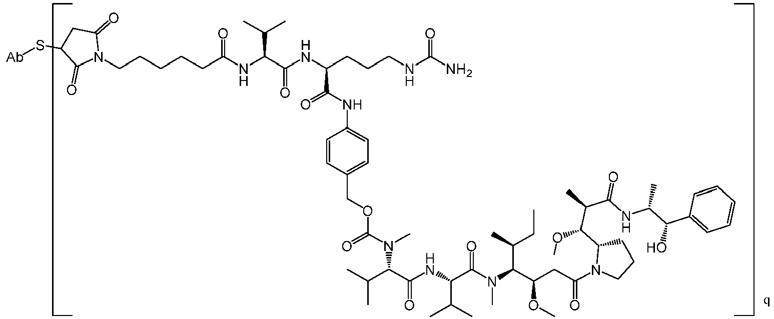

Ab is a c-MET antibody (for example, ABT700 as described below), and q can be determined based on the data provided below.

### 3.2.1 Preparation of an ABT700-vc-MMAE (DAR9.7) sample

A total of 20 mg of anti-c-Met antibodies ABT700 are added to a sodium edetate solution with a final concentration of 1 mM and mixed well, and the pH of the sample is adjusted to 7.5 with a disodium hydrogen phosphate solution of 0.5 M; a TCEP solution of 20 mmol/L that is 6.5 molar equivalents of the antibodies is added, mixed well, and placed at 37°C for 90 minutes; and vc-MMAE (CAS No.: 646502-53-6, purchased from MedChemExpress) of 10 mmol/L that is 15 molar equivalents of the antibodies and dissolved in dimethyl sulfoxide is added to the foregoing solution system, and mixed well, to rest at 37°C for 3 hours and obtain a conjugated sample, after the reaction is completed, a 30KDa ultrafiltration tube is used to replace the sample into a histidine buffer of 20 mM with a pH of 6.0 and remove low-molecular substances, and finally, the sample is concentrated to obtain a solution containing anti-c-Met antibody ADCs ABT700-vc-MMAE (DAR9.7).

The DAR value of the conjugated sample is assayed referring to the foregoing LC/MS in 3.1.1, the light chain of the ABT700-Vc-MMAE (DAR9.7) sample is conjugated to 1-3 toxin molecules (ratios of DAR1, DAR2, and DAR3 are 64.3%, 29.8%, and 5.9%, respectively), and the heavy chain is conjugated to 3-4 toxin molecules (ratios of DAR3 and DAR4 are 56.4% and 43.6%, respectively), from which the conjugation ratio (DAR value) of the ABT700-Vc-MMAE (DAR9.7) sample is calculated to be 9.7.

### 3.2.2 Preparation of an ABT700-vc-MMAE (DAR4) sample

A total of 20 mg of anti-c-Met antibodies ABT700 are added to a sodium edetate solution with a final concentration of 1 mM and mixed well, and the pH of the sample is adjusted to 7.5 with a disodium hydrogen phosphate solution of 0.5 M; a TCEP solution of 20 mmol/L that is 2.8 molar equivalents of the antibodies is added, mixed well, and placed at the room temperature for 90 minutes; and vc-MMAE (CAS No.: 646502-53-6, purchased from MedChemExpress) of 10 mmol/L that is 4.2 molar equivalents of the antibodies and dissolved in dimethyl sulfoxide is added to the foregoing solution system, and mixed well, to rest at the room temperature for 3 hours and obtain a conjugated sample, after the reaction is completed, a 30KDa ultrafiltration tube is used to replace the sample into a histidine buffer of 20 mM with a pH of 6.0 and remove low-molecular substances, and finally, the sample is concentrated to obtain a solution containing anti-c-Met antibody ADCs ABT700-Vc-MMAE (DAR4).

The DAR value of the conjugated sample is assayed referring to the foregoing LC/MS in 3.1.1, the light chain of the ABT700-VC-MMAE (DAR4) sample is conjugated to 0-1 toxin molecule (ratios of LC and DAR1 are 52.2% and 47.8%, respectively), and the heavy chain is conjugated to 0-3 toxin molecules (ratios of mAb, DAR1, DAR2, and DAR3 are 17.5%, 47.2%, 22.9%, and 12.5%, respectively), from which the conjugation ratio (DAR value) of the ABT700-VC-MMAE (DAR4) sample is calculated to be 4.0.

### Embodiment 4. In-vitro evaluation of anti-human c-Met antibodies and ADCs thereof

### 4.1 Detection of protein affinities of anti-human cMet ADCs

Proteins anti-His-Rabbit Fc are coated with the CBS to 0.5 µg/ml, and are blocked with 2% BSA (in PBS) at 37°C for 2 hours, h.cMet-ECD-His of 1 µg/ml is added for incubation at 37°C for 2 hours, after 2 hours, antibodies to be tested or ADCs that are gradiently diluted (starting at 2 µg/mL for 3-fold dilution with 11 concentration points) are added for incubation at 37°C for 2 hours, after 2 hours, HRP-labeled anti-human specific secondary antibodies (Jackson, 115-035-164) are added for incubation at 37°C for 1 hour, a TMB substrate is added for color development, and after the color development is stopped using HCl of 2M, final products are added into a machine to read out absorbance values at 450 nM.

The experimental results are shown in Table 8. The antigen-binding affinities of 45A5G10-HZ, 45A5G10-HZ-B81 (DAR8), 55A10G6-Hz, and 55A10G6-HZ-B81 (DAR8) do not change significantly, while the antigen binding affinity of ABT700 after conjugating into ADCs significantly decreases.

**Table 8. Detection of protein affinities of cMET antibodies before and after conjugating into ADCs**

| Sample Name | EC50 (ng/mL) |
|---|---|
| 45A5G10-HZ | 4.372 |
| 45A5G10-HZ-B81 (DAR8) | 3.106 |
| 55A10G6-HZ | 6.142 |
| 55A10G6-HZ-B81 (DAR8) | 3.923 |
| ABT700 | 5.257 |
| ABT700-vc-MMAE (DAR9.7) | 2432 |

### 4.2 Detection of cell affinities of anti-human cMet ADCs

### Test 1

NCI-H358 (human non-small cell lung cancer cells, purchased from ATCC, Cat. No. CRL-5807), LS1034 cells (human colon adenocarcinoma cells, Nanjing Cobioer Biosciences Co., Ltd., Cat. No. CBP60013), NCI-H69 (human small cell lung cancer cells, Wuhan Pricella Biotechnology Co., Ltd., Cat. No. CL-0677), and NCI-H716 (human colorectal adenocarcinoma cells, purchased from ATCC, Cat. No. CCL-251) cells are collected by trypsin digestion and obtained directly by centrifugation, and are washed three times with a pre-cooled PBS, the cells are resuspended with 1% BSA (in PBS), and are plated into a 96-well conical bottom plate with 2*10⁵ cells (50 ul) per well, ADCs of anti-cMet humanized antibodies are gradiently diluted with 1% BSA starting at 15 µg/ml with a 4-fold dilution gradient and 8 concentration points, 50 ul of diluted ADCs are mixed well with the cells in the conical bottom plate for incubation at 4°C for 1 hour, after three times of washing with the pre-cooled PBS, 100 ul of 1% BSA (containing 1 uL of anti-human APC fluorescent secondary antibodies, BioLegend, Cat. No. 410712) is added to each well for incubation at 4°C for 0.5 hours, and after three times of washing with the pre-cooled PBS, the cells are resuspended for detection by a flow cytometer (Beckman, cytoflex).

The experimental results are shown in Table 9. The ADCs of 2 humanized antibodies after conjugating to B81 both have a higher affinity to the NCI-H358, LS1034, NCI-H69, and NCI-H716 cells, and the affinities of the ADCs of the two antibodies to three types of tumor cells are all higher than that of ABT700-VcMMAE (DAR9.7). The affinities of the ADCs of the two antibodies to the NCI-H716 cells are higher than that of ABT700-VcMMAE (DAR4).

**Table 9. Detection of cell affinities of anti-human cMet ADCs**

| Sample Name | NCI-H358 | | NCI-H69 | | LS1034 | | NCI-H716 | |
|---|---|---|---|---|---|---|---|---|
| Detection result | EC50 (nM) | Maximum signal value | EC50 (nM) | Maximum signal value | EC50 (nM) | Maximum signal value | EC50 (nM) | Maximum signal value |
| 45A5G10-HZ-B81 (DAR8) | 0.2392 | 45966 | 0.09617 | 10181 | 0.1171 | 9173 | 0.4033 | 2988 |
| 55A10G6-HZ-B81 (DAR8) | 0.1921 | 46313 | 0.07617 | 10484 | 0.0988 | 9601 | 0.5425 | 2941 |
| ABT700-VcMMAE (DAR4) | Not detected | | | | | | 11.56 | 3737 |
| ABT700-VcMMAE (DAR9.7) | 0.9169 | 32386 | 0.2116 | 8214 | 0.8524 | 8960 | Not detected | Not detected |

### Test 2

MKN45 (from JCBR JCRB0254) cells are collected by trypsin digestion and centrifugation, and are washed three times with a pre-cooled PBS, the cells are diluted to 1x10⁶ cells/mL, and are plated into a 96-well conical bottom plate with 1x10⁵ cells (100 µL) per well, 45A5G10-HZ-B81 (DAR8) and 45A5G10-HZ are gradiently diluted with an FACS buffer starting at 25 nM with a 4-fold dilution gradient and 8 concentration points, and are mixed well with the cells in the conical bottom plate to 100 µL/well for incubation at 4°C for 0.5 hours, after three times of washing with the pre-cooled PBS, 100 µL of fluorescent secondary antibodies PE anti-human IgG diluted with a cold FACS buffer are added to each well for incubation at 4°C for 0.5 hours, and after three times of washing with the pre-cooled PBS, the cells are resuspended for detection by a flow cytometer.

The results show that 45A5G10-HZ-B81 (DAR8) and 45A5G10-HZ have higher binding activity to MKN45, 45A5G10-HZ and 45A5G10-HZ-B81 (DAR8) have a comparable affinity to MKN45, and EC₅₀ are sequentially 0.215 nM and 0.189 nM, respectively.

### 4.3 Detection of in-vitro killing activities of ADCs

### Test 1

Tumor cells in a good growth status are collected by digestion and centrifugation, SW480 (purchased from ATCC, Cat. No. CCL-228), NCI-H358 (purchased from ATCC, Cat. No. CRL-5807), LS1034 cells (Nanjing Cobioer Biosciences Co., Ltd., Cat. No. CBP60013), NCI-H716 (purchased from ATCC, Cat. No. CCL-251) cells are directly collected by centrifugation, the foregoing cells are all resuspended with RPMI 1640 + 5% FBS + Ps, and are counted, and 5000 (LS1034 and NCI-H716) or 3000 (SW480 and NCI-H358) cells are plated in each well in a volume of 100 uL; ADCs to be tested are diluted separately with resuspension media corresponding to the foregoing 3 types of cells, starting at 300 µg/mL (2 µM) for 3-fold dilution with 11 concentration points, and added into a plate with 100 µL/well, to make the ADCs have a final concentration of 150 µg/mL, for incubation at 37°C for 5 days (LS1034 and NCI-H358) or 7 days (NCI-H716 and SW480); after the incubation is completed, CCK8 is added with 20 µL/well, for a reaction for 1-3 hours; and values are read out at 450 nm by a microplate reader and imported into Graphpad Prism for curve-fitting.

The experimental results are shown in Table 10. Both 45A5G10-Hz-B81 (DAR8) and 55A10G6-Hz-B81 (DAR8) can effectively kill four types of tumor cells of LS1034, NCI-H358, NCI-H716, and SW480. On the LS1034 cells, the cell killing activity of 45A5G10-Hz-B81 (DAR8) is comparable to ABT700-VcMMAE (DAR9.7), while 55A10G6-Hz-B81 (DAR8) is slightly weaker than ABT700-VcMMAE (DAR9.7); on NCI-H358 cells, the killing activity of 45A5G10-Hz-B81 (DAR8) is stronger than that of 55A10G6-Hz-B81 (DAR8); on the NCI-H716 cells, the killing activities of 45A5G10-Hz-B81 (DAR8) and 55A10G6-Hz-B81 (DAR8) are both stronger than that of ABT700-VcMMAE (DAR4); and on the SW480 cells, the cell killing activities of 45A5G10-Hz-B81 (DAR8) and ABT700-VcMMAE (DAR4) are stronger, while 55A10G6-Hz-B81 (DAR8) is comparable to ABT700-VcMMAE (DAR4).

**Table 10. Detection of in-vitro cell killing activities of anti-human cMet ADCs**

| Name | EC50 of ADC killing (nM) | | | |
|---|---|---|---|---|
| | LS1034 | NCI-H358 | NCI-H716 | SW480 |
| 45A5G10-HZ-B81 (DAR8) | 64.91 | 81.64 | 6.961 | 55.16 |
| 55A10G6-HZ-B81 (DAR8) | 100.8 | 145.1 | 4.661 | 87.51 |
| ABT700-Vc-MMAE (DAR4) | Not detected | Not detected | 287.5 | 71.64 |
| ABT700-Vc-MMAE (DAR9.7) | 53.67 | >10000 | Not detected | Not detected |

### Test 2

NCI-H358 cells (from ECACC-95111733), NCI-H441 cells (from ATCC-HTB-174), and MKN45 cells (from JCBR JCRB0254) are collected by trypsin digestion and centrifugation, and RPMI 1640 media containing 10% FBS are used to resuspend the cells, the cells are counted, and 1500 (NCI-H441/NCI-H358/MKN45) cells are plated in each well in a volume of 135 µL; 45A5G10-HZ-B81 (DAR8) is separately diluted with the resuspension media corresponding the foregoing 3 types of cells, starting at 20000 nM for 4-fold dilution with 9 concentration points, further diluted with the media to a start of 500 nM, and added into a plate with 15 µL/well, to make the ADCs have a final concentration of 50 nM, for incubation at 37°C for 6 days; after the incubation is completed, a CellTiter-Glo working solution is added with 75 µL/well, the plate is shook on an orbital shaker for 2 minutes to induce cell lysis, and placed at the room temperature for 10 minutes to stabilize luminescence signals; and a microplate reader is used to detect the luminescence signals.

The experimental results are shown in Table 11. The results show that 45A5G10-HZ-B81 (DAR8) has a stronger anti-proliferative effect on the NCI-H441 cells, and its corresponding IC50 is 0.031 nM. 45A5G10-HZ-B81 (DAR8) has an anti-proliferative effect on the NCI-H358 cells, and its corresponding IC50 is 0.161 nM. 45A5G10-HZ-B81 (DAR8) has a stronger anti-proliferative effect on the MKN45 cells, and its corresponding IC50 is 0.057 nM.

**Table 11. Anti-proliferative effects of anti-human cMet ADCs**

| Cell line | Compound to be tested | IC50 (nM) | Bottom (%) | Top (%) |
|---|---|---|---|---|
| NCI-H358 | 45A5G10-HZ-B81 (DAR8) | 0.161 | 5.76 | 43.07 |
| NCI-H441 | 45A5G10-HZ-B81 (DAR8) | 0.031 | 2.94 | 76.44 |
| MKN45 | 45A5G10-HZ-B81 (DAR8) | 0.057 | 3.14 | 88.29 |

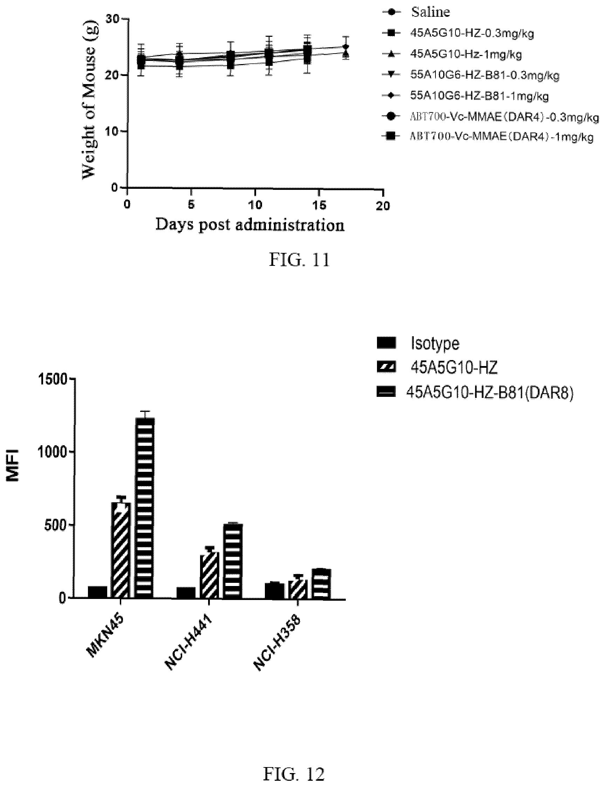

The results show that 45A5G10-HZ-B81 (DAR8) can significantly inhibit the proliferation of NCI-H441, NCI-H358, and MKN45 cell lines, and the maximum inhibition rates are 76.44%, 43.07%, and 88.29%, respectively. The target expression level of the NCI-H358 cells is significantly lower than that of the NCI-H441 cells (the results of FACS verification of c-Met target expression in cells are shown in FIG. 12), but still has a maximum inhibition rate of >40%.

### 4.4 Forte bio detection of affinities of anti-c-Met antibodies and ADCs thereof to a human Fc receptor and a complement C1q

### • 4.4.1: Detection of affinities of FcγRI to 45A5G10-HZ-B81 (DAR8) and 45A5G10-HZ

The Fc receptor FcγRI (also known as CD64), can bind to the Fc end of an IgG antibody, and participate in antibody-dependent cell-mediated cytotoxicity (ADCC). The ability of a therapeutic monoclonal antibody to bind to the Fc receptor affects the safety and efficacy of the antibody.

In this experiment, a Fortebio Octet molecular interaction instrument is used to detect the affinity constants of 45A5G10-HZ-B81 (DAR8) and 45A5G10-HZ to FcγRI, to evaluate potential ADCC and ADCP activities thereof.

The experimental method for detecting the affinity constants of the corresponding antibodies to FcγRI using the Fortebio Octet molecular interaction instrument is briefly described as follows: A sample dilution buffer is a PBS solution containing 0.02% Tween-20 and 0.1% BSA (pH 7.4). An FcγRI (purchased from ACRO Biosystems, Cat. No. FCA- H52H1) solution with a concentration of 5 µg/mL is added to an HIS 1K sensor, to immobilize FcγRI on the surface of the sensor. The binding and dissociation parameters of the antibodies to/from FcγRI are all assayed in the buffer, and the concentrations of the antibodies are 200, 100, 50, 25, 12.5, and 6.25 nM. After the sensor with the antigens immobilized is equilibrated in the buffer for 60s, binding of FcγRI immobilized on the sensor to the antibodies is assayed for a period of time of 60s; and the assay time of dissociation of FcγRI from the antibodies is 120s. The data is analysed by DataAnalysis11, to obtain the affinity constants of the antibodies to FcγRI.

The assay results of the affinity constants of FcγRI to 45A5G10-HZ-B81 (DAR8) and 45A5G10-HZ are shown in the following Table 12.

**Table 12. Kinetic parameters of binding of 45A5G10-HZ-B81 (DAR8) and 45A5G10-HZ to FcγRI**

| **Sample** | **Sample concentration** | **KD (M)** | **Kon (1/Ms)** | **Kdis (1/s)** | **RMax** | **R^2** |
|---|---|---|---|---|---|---|
| 45A5G10-HZ-B81 (DAR8) | 200-6.25 nM | 1.07E-08 | 4.17E+05 | 4.45E-03 | 0.7543 | 0.9979 |
| 45A5G10-HZ | | 1.43E-08 | 3.52E+05 | 5.05E-03 | 0.6191 | 0.9983 |

The results show that the binding activity of 45A5G10-HZ-B81 (DAR8) to FcγRI is similar to that of 45A5G10-HZ.

### • 4.4.2: Detection of affinities of FcγRIIIa_V176 to 45A5G10-HZ-B81 (DAR8) and 45A5G10-HZ

The Fc receptor FcγRIIIa_V176 (also known as CD16a_V176), can bind to the Fc end of an IgG antibody, and participate in antibody-dependent cell-mediated cytotoxicity (ADCC).

In this experiment, a Fortebio Octet molecular interaction instrument is used to detect the affinity constants of 45A5G10-HZ-B81 (DAR8) and 45A5G10-HZ to FcγRIIIa_V176, to evaluate potential ADCC activities thereof.

The experimental method for detecting the affinity constants of the corresponding antibodies to FcγRIIIa_V176 using the Fortebio Octet molecular interaction instrument is briefly described as follows: A sample dilution buffer is a PBS solution containing 0.02% Tween-20 and 0.1% BSA (pH 7.4). An FcγRIIIa_V176 (purchased from ACRO Biosystems, Cat. No. CD8- H52H4) solution with a concentration of 10 µg/mL is added to an HIS1K sensor, to immobilize FcγRIIIa_V176 on the surface of the sensor. The binding and dissociation parameters of the antibodies to/from FcγRIIIa_V176 are all assayed in the buffer, and the concentrations of the antibodies are 2500, 1250, 625, 312.5, 156.25, and 78.125 nM. After the sensor with the antigens immobilized is equilibrated in the buffer for 60s, binding of FcγRIIIa_V176 immobilized on the sensor to the antibodies is assayed for a period of time of 60s; and the assay time of dissociation of FcγRIIIa_V176 from the antibodies is 60s. The data is analysed by DataAnalysis11, to obtain the affinity constants of the antibodies to FcγRIIIa_V176.

The assay results of the affinity constants of FcγRIIIa_V176 to 45A5G10-HZ-B81 (DAR8) and 45A5G10-HZ are shown in the following Table 13.

**Table 13. Kinetic parameters of binding of 45A5G10-HZ-B81 (DAR8) and 45A5G10-HZ to FcγRIIIa _V176**

| **Sample** | **Sample concentration** | **KD (M)** | **RMax** | **R^2** |
|---|---|---|---|---|
| 45A5G10-HZ-B81 (DAR8) | 2500-78.125 nM | 1.70E-06 | 0.8795 | 0.9989 |
| 45A5G10-HZ | | 5.30E-07 | 0.9802 | 0.9965 |

The results show that the binding activity of 45A5G10-HZ-B81 (DAR8) to FcγRIIIa_V176 is similar to that of 45A5G10-HZ.

### • 4.4.3: Detection of affinities of FcγRIIIa_F176 to 45A5G10-HZ-B81 (DAR8) and 45A5G10-HZ

The Fc receptor FcγRIIIa_F176 (also known as CD16a_F176), can bind to the Fc end of an IgG antibody, and participate in antibody-dependent cell-mediated cytotoxicity (ADCC).

In the binding experiments between the two samples and FcγRIIIa_F176 (purchased from ACRO Biosystems, Cat. No. CDA -H 522 0), except that the interval of analyte concentration in the binding step is optimized to (5000, 2500, 1250, 625, 3 12.5, 156.25 nM), the other steps are consistent with the steps of the binding experiment of the CD16a (V 176).

The assay results of the affinity constants of FcγRIIIa_F176 to 45A5G10-HZ-B81 (DAR8) and 45A5G10-HZ are shown in the following Table 14.

**Table 14: Kinetic parameters of binding of 45A5G10-HZ-B81 (DAR8) and 45A5G10-HZ to FcγRIIIa_F176**

| **Sample** | **Sample concentration** | **KD (M)** | **RMax** | **R^2** |
|---|---|---|---|---|
| 45A5G10-HZ-B81 (DAR8) | 5000-156.25 nM | 5.00E-06 | 0.7037 | 0.9983 |
| 45A5G10-HZ | | 1.40E-06 | 0.8088 | 0.9932 |

The results show that the binding activity of 45A5G10-HZ-B81 (DAR8) to FcγRIIIa_F176 is similar to that of 45A5G10-HZ.

### • 4.4.4: Detection of affinities of FcγRIIa_H167 to 45A5G10-HZ-B81 (DAR8) and 45A5G10-HZ

The Fc receptor FcγRIIa_H167 (also known as CD32a_H167), can bind to the Fc end of an IgG antibody, and participate in antibody-dependent cell-mediated cytotoxicity (ADCC).

In this experiment, a Fortebio Octet molecular interaction instrument is used to detect the affinity constants of 45A5G10-HZ-B81 (DAR8) and 45A5G10-HZ to FcγRIIa_H167, to evaluate potential ADCC activities thereof.

The experimental method for detecting the affinity constants of the corresponding antibodies to FcγRIIa_H167 using the Fortebio Octet molecular interaction instrument is briefly described as follows: A sample dilution buffer is a PBS solution containing 0.02% Tween-20 and 0.1% BSA (pH 7.4). An FcγRIIa_H167 (purchased from ACRO Biosystems, Cat. No. CD1 H5223) solution with a concentration of 10 µg/mL is added to an HIS1K sensor, to immobilize FcγRIIa_H167 on the surface of the sensor. The binding and dissociation parameters of the antibodies to/from FcγRIIa_H167 are all assayed in the buffer, and the concentrations of the antibodies are 5000, 2500, 1250, 625, 312.5, and 156.25 nM. After the sensor with the antigens immobilized is equilibrated in the buffer for 60s, binding of FcγRIIa_H167 immobilized on the sensor to the antibodies is assayed for a period of time of 60s; and the assay time of dissociation of FcγRIIa_H167 from the antibodies is 60s. The data is analysed by DataAnalysis11, to obtain the affinity constants of the antibodies to FcγRIIa_H167.

The assay results of the affinity constants of FcγRIIa_H167 to 45A5G10-HZ-B81 (DAR8) and 45A5G10-HZ are shown in the following Table 15.

**Table 15. Kinetic parameters of binding of 45A5G10-HZ-B81 (DAR8) and 45A5G10-HZ to FcγRIIa_H167**

| **Sample** | **Sample concentration** | **KD (M)** | **RMax** | **R^2** |
|---|---|---|---|---|
| 45A5G 1 0-HZ-B81 (DAR8) | 5000-156.25 nM | 5.20E-06 | 0.9232 | 0.9988 |
| 45A5G10-HZ | | 2.00E-06 | 0.8687 | 0.9974 |

The results show that the binding activity of 45A5G10-HZ-B81 (DAR8) to FcγRIIa_H167 is similar to that of 45A5G10-HZ.

### • 4.4.5: Detection of affinities of FcγRIIa_R167 to 45A5G10-HZ-B81 (DAR8) and 45A5G10-HZ

The Fc receptor FcγRIIa_R167 (also known as CD32a_R167), can bind to the Fc end of an IgG antibody, and participate in antibody-dependent cell-mediated cytotoxicity (ADCC).

In this experiment, a Fortebio Octet molecular interaction instrument is used to detect the affinity constants of 45A5G10-HZ-B81 (DAR8) and 45A5G10-HZ to FcγRIIa_R167, to evaluate potential ADCC activities thereof.

The binding experiments between the two samples and FcγRIIa_R167 (purchased from ACRO Biosystems, Cat. No. CDA -H 522 1) are the same as the steps of the binding experiments of FcγRIIa_H167 except for the binding step.

The assay results of the affinity constants of FcγRIIa_R167 to 45A5G10-HZ-B81 (DAR8) and 45A5G10-HZ are shown in the following Table 16.

**Table 16. Kinetic parameters of binding of 45A5G10-HZ-B81 (DAR8) and 45A5G10-HZ to FcγRIIa_R167**

| **Sample** | **Sample concentration** | **KD (M)** | **RMax** | **R^2** |
|---|---|---|---|---|
| 45A5G10-HZ-B81 (DAR8) | 5000-156.25 nM | 3.70E-06 | 0.8074 | 0.9985 |
| 45A5G10-HZ | | 2.30E-06 | 0.7391 | 0.9976 |

The results show that the binding activity of 45A5G10-HZ-B81 (DAR8) to FcγRIIa_R167 is similar to that of 45A5G10-HZ.

### • 4.4.6: Detection of affinities of FcγRIIb/c to 45A5G10-HZ-B81 (DAR8) and 45A5G10-HZ

The Fc receptor FcγRIIb/c (also known as CD32b/c) can bind to the Fc end of an IgG antibody, negatively regulate the functions of immune cells, inhibit the activation and proliferation of the immune cells, and inhibit the secretion of cytokines.

In this experiment, a Fortebio Octet molecular interaction instrument is used to detect the affinity constants of 45A5G10-HZ-B81 (DAR8) and 45A5G10-HZ to FcγRIIb/c, to evaluate the binding abilities of 45A5G10-HZ-B81 (DAR8) and 45A5G10-HZ to FcγRIIb/c.

The experimental method for detecting the affinity constants of the corresponding antibodies to FcγRIIb/c using the Fortebio Octet molecular interaction instrument is briefly described as follows: A sample dilution buffer is a PBS solution containing 0.02% Tween-20 and 0.1% BSA (pH 7.4). An FcγRIIb/c (purchased from ACRO Biosystems, Cat. No. CDB-H5228) solution with a concentration of 10 µg/mL is added to an HIS1K sensor, to immobilize FcγRIIb/c on the surface of the sensor. The binding and dissociation parameters of the antibodies to/from FcγRIIb/c are all assayed in the buffer, and the concentrations of the antibodies are 10000, 5000, 2500, 1250, 625, and 312.5 nM. After the sensor with the antigens immobilized is equilibrated in the buffer for 60s, binding of FcγRIIb/c immobilized on the sensor to the antibodies is assayed for a period of time of 60s; and the assay time of dissociation of FcγRIIb/c from the antibodies is 60s. The data is analysed by DataAnalysis11, to obtain the affinity constants of the antibodies to FcγRIIb/c.

The assay results of the affinity constants of FcγRIIb/c to 45A5G10-HZ-B81 (DAR8) and 45A5G10-HZ are shown in the following Table 17.

**Table 17. Kinetic parameters of binding of 45A5G10-HZ-B81 (DAR8) and 45A5G10-HZ to FcγRIIb/c**

| **Sample** | **Sample concentration** | **KD (M)** | **RMax** | **R^2** |
|---|---|---|---|---|
| 45A5G10-HZ-B81 (DAR8) | 10000-312.5 nM | 8.80E-06 | 0.8126 | 0.9993 |
| 45A5G10-HZ | | 4.20E-06 | 0.6437 | 0.9991 |

The results show that the binding activity of 45A5G10-HZ-B81 (DAR8) to FcγRIIb/c is similar to that of 45A5G10-HZ.

### • 4.4.7: Detection of affinities of FcRn to 45A5G10-HZ-B81 (DAR8) and 45A5G10-HZ

The Fc receptor FcRn can bind to the Fc end of an IgG antibody, to protect antibody macromolecules from destruction, and then release the antibody macromolecules in a blood environment with a pH of 7.4.

In this experiment, a Fortebio Octet molecular interaction instrument is used to detect the affinity constants of 45A5G10-HZ-B81 (DAR8) and 45A5G10-HZ to FcRn, to evaluate the binding abilities of 45A5G10-HZ-B81 (DAR8) and 45A5G10-HZ to FcRn.

The experimental method for detecting the affinity constants of the corresponding antibodies to FcRn using the Fortebio Octet molecular interaction instrument is briefly described as follows: A sample dilution buffer is a PBS solution containing 0.02% Tween-20 and 0.1% BSA (pH 7.4). An FcRn (purchased from ACRO) solution with a concentration of 2 µg/mL is added to an HIS 1K sensor, to immobilize FcRn on the surface of the sensor. The binding and dissociation parameters of the antibodies to/from FcRn are all assayed in the buffer, and the concentrations of the antibodies are 1000, 500, 250, 125, 62.5, and 31.25 nM. After the sensor with the antigens immobilized is equilibrated in the buffer for 60s, binding of FcRn immobilized on the sensor to the antibodies is assayed for a period of time of 60s; and the assay time of dissociation of FcRn from the antibodies is 60s. The data is analysed by DataAnalysis11, to obtain the affinity constants of the antibodies to FcRn.

The assay results of the affinity constants of FcRn to 45A5G10-HZ-B81 (DAR8) and 45A5G10-HZ are shown in the following Table 18.

**Table 18 Kinetic parameters of binding of 45A5G10-HZ-B81 (DAR8) and 45A5G10-HZ to FcRn**

| **Sample** | **Sample concentration** | **KD (M)** | **RMax** | **R^2** |
|---|---|---|---|---|
| 45A5G10-HZ-B81 (DAR8) | 1000-31.25 nM | 3.40E-07 | 1.057 | 0.9757 |
| 45A5G10-HZ | | 3.70E-07 | 0.7423 | 0.9913 |

The results show that the binding activity of 45A5G10-HZ-B81 (DAR8) to FcRn is similar to that of 45A5G10-HZ.

### • 4.4.8: Assay of affinities of Clq to 45A5G10-HZ-B81 (DAR8) and 45A5G10-HZ

The serum complement C1q can bind to the Fc end of an IgG antibody and mediate the CDC effect. The ability of a therapeutic monoclonal antibody to bind to C1q affects the safety and efficacy of the antibody.

In this experiment, a Fortebio Octet molecular interaction instrument is used to detect the affinity constants of 45A5G10-HZ-B81 (DAR8) and 45A5G10-HZ to C1q, to evaluate potential CDC activities of the antibodies.

The experimental method for detecting the affinity constants of the corresponding antibodies to C1q using the Fortebio Octet molecular interaction instrument is briefly described as follows: A sample dilution buffer is a PBS solution containing 0.02% Tween-20 and 0.1% BSA (pH 7.4). Antibodies of 50 µg/mL are immobilized on an FAB2G sensor with an immobilization height of about 3.0 nm, the sensor is equilibrated in the buffer for 60s, the antibodies immobilized on the sensor bind to the antigens C1q (purchased from Sigma, Cat. No. C1740-1MG), the antigen concentrations of 45A5G10-HZ are 20, 10, 5, 2.5, 1.25, 0.625 nM, the antigen concentrations of 45A5G10-HZ-B81 (DAR8) of are 500, 250, 125, 62.5, 31.25, 15.625 nM, for a period of time of 60s, and the antigens and antibodies are dissociated in the buffer, for a period of time of 60s. The sensor is regenerated using glycine of 10 mM with a pH of 1.7, for a period of time of 5s, which is repeated 3 times. The data is analysed by DataAnalysis11, to obtain the affinity constants.

The assay results of the affinity constants of C1q to 45A5G10-HZ-B81 (DAR8) and 45A5G10-HZ are shown in the following Table 19.

**Table 19. Kinetic parameters of binding of 45A5G10-HZ-B81 (DAR8) and 45A5G10-HZ to C1q**

| **Sample** | **Sample concentration** | **KD (M)** | **Kon (1/Ms)** | **Kdis (1/s)** | **RMax** | **R^2** |
|---|---|---|---|---|---|---|
| 45A5G10-HZ-B81 (DAR8) | 500-15.625 nM | 2.00E-07 | NA | NA | 0.2772 | 0.9969 |
| 45A5G10-HZ | 20-0.625 nM | 3.99E-09 | 5.13E+06 | 2.05E-02 | 0.4326 | 0.9878 |

The results show that 45A5G10-HZ and 45A5G10-HZ-B81 (DAR8) can bind to C1q, and affinity constants are 3.99E-09M and 2.00E-07M, respectively; and the results show that the binding activity of 45A5G10-HZ to C1q is effectively eliminated after conjugation to toxins.

### 4.5 Forte bio detection of ADCC activities of anti-c-Met antibodies and ADCs thereof

The ADCC effect means that an effector immune cell with a killing activity recognize the Fc fragment of the antibody binding to the antigen of a target cell via the Fc receptor (FcR) expressed on the surface of the effector immune cell, and directly kill the target cell.

For detection of the ADCC activities of 45A5G10-HZ-B81 (DAR8) and 45A5G10-HZ on MKN45 cells (from Sichuan Sibowo Biotechnology Co., Ltd.) expressing c-MET antigens, the method is as follows:
0.5 g of BSA is weighed, added to 50 mL of RPMI1640 basic medium for full dissolution, and then filtered through a 0.22 µM micropore filter, to obtain a sample dilution (RPMI1640 + 1% BSA), which is ready for use. The day before the experiment, target cells (MKN45) in the exponential growth phase are digested with 0.25% trypsin in a biological safety cabinet to prepare a single cell suspension, and then counted using a cell counter, the cell density is adjusted using a RPMI1640 + 10% FBS medium to 2x10⁵ cells/mL, the cells are added to a cell culture plate with 100 µL/well, and after incubation overnight in a 37°C carbon dioxide cell incubator, the supernatant is discarded, and the rest is for standby application. Effector cells (Jurkat-NFAT-CD16a, from Wuhan Taituozhong Biotechnology Co., Ltd.) in the exponential growth phase are collected and counted using the cell counter, the cell density is adjusted using an RPMI1640 basic medium to 4x10⁶ cells/mL, and the cells are added to experimental wells containing the MKN45 cells with 50 µL/well, that is, a sample group (ADCs and antibodies) and a negative control group (hIgG1). Daudi cells (CD20 target cells, from ATCC, Cat. No. CCL213) and effector cells (Jurkat-NFAT-CD16a) in the exponential growth phase are separately collected and counted using the cell counter, then an appropriate amount of the Daudi cell and Jurkat-NFAT-CD16 cell suspension are separately measured and mixed well, the cell densities are adjusted using the RPMI1640 basic medium to make the Daudi cells have a final density of 4x10⁵ cells/mL and the Jurkat-NFAT-CD16a cells have a final density of 4x10⁶ cells/mL, and the cells are added to experimental wells of the positive control group of the experimental plate with 50 µL/well. The tested substances are diluted with sample diluents to a 2x concentration (that is, 40000 ng/mL), and then further diluted in a 5-fold serial gradient with a total of 8 concentrations. Rituximab is diluted in a 7-fold serial gradient with a total of 8 concentrations. Based the layout of the experimental plate, 50 µL of 2x tested substances subjected to dilution in each gradient are added to each well. The cell culture plate is placed on a microplate shaker at 500 rpm/min for well mixing for 5 minutes, and the 96-well plate is placed in a 37°C carbon dioxide constant-temperature incubator for incubation for about 6 hours. After the incubation is completed, Bio-Glory one-step (from Adamas life, Cat. No. RA-GL04) reagents that are pre-melted and equilibrated to the room temperature are added to the experimental wells of the cell culture plate with 50 µL/well, the cell culture plate is shook at the room temperature for 10 minutes for well mixing, and a microplate reader is used to assay bioluminescence signal values.

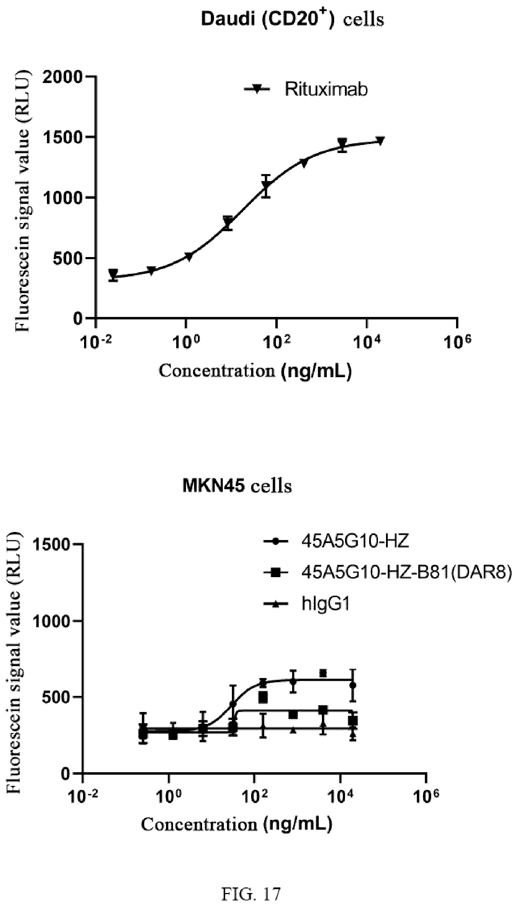

The detection results of the ADCC activities of 45A5G10-HZ-B81 (DAR8) and 45A5G10-HZ on the MKN45 cells expressing the c-MET antigens are shown in FIG. 17.

The results show that in the mixed culture system of MKN45 and JurkatNFAT-CD16a, at the same dose level, the ADCC activity induced by 45A5G10-HZ-B81 (DAR8) is significantly lower than that of 45A5G10-HZ.

The results show that 45A5G10-HZ has a weak ADCC effect, while 45A5G10-HZ-B81 (DAR8) has no obvious ADCC effect.

### 4.6 Forte bio detection of CDC activities of anti-c-Met antibodies and ADCs thereof

The CDC effect is achieved through an antibody binding to the corresponding antigen on the cell membrane surface, and at the same time binding to a complement C1q, activating the classic pathway of the complement-dependent cytotoxic effect, and then forming a membrane attack complex, thereby exerting a lytic effect on the target cell.

For detection of the CDC activities of 45A5G10-HZ-B81 (DAR8) and 45A5G10-HZ on MKN45 cells expressing c-MET antigens, the method is as follows:
0.5 g of BSA is weighed, added to 50 mL of RPMI1640 basic medium for full dissolution, and then filtered through a 0.22 µM micropore filter, to obtain a sample dilution (RPMI1640 + 1% BSA), which is ready for use. Target cells (MKN45, Daudi) in the exponential growth phase are taken to prepare a single cell suspension, and then counted using a cell counter, the MKN45 cells are adjusted using an RPMI1640 basic medium to 2x10⁵ cells/mL, the density of the Daudi cells is adjusted using the RPMI1640 basic medium to 6x10⁵ cells/mL, and after those cells are mixed well by pipetting, 40 µL of cell suspension is added to each well of a cell culture plate based on the layout of the experimental plate. 1 bottle of freeze-dried guinea pig serum complements (from BERSEE, Cat. No. BM361Y) is added to 1 mL of DMEM basic medium for redissolution, and a concentration of the complements at this time is 100%. 1000 µL of 100% complements are added to 1000 µL of DMEM basic medium and mixed well, a concentration of the complements at this time is 50%, 20 µL of 50% serum complements are added to each experimental well of the cell culture plate, to make the complements in the well have a final concentration of 10%. The samples to be tested are diluted with sample diluents to a 2.5x concentration (that is, 250000 ng/mL), and then further separately diluted in a 3-fold serial gradient with a total of 12 concentrations, and 40 µL of each tested substance diluent are added to each well. The cell culture plate is placed on a microplate shaker at 500 rpm/min for well mixing for 5 minutes, and then placed in a 37°C carbon dioxide incubator for incubation for about 6 hours. After the incubation is completed, Cell Titer Turbo2.0 reagents that are pre-melted and equilibrated to the room temperature are added to the experimental wells of the cell culture plate with 50 µL/well, the cell culture plate is shook at the room temperature for 10 minutes for well mixing, and a microplate reader is used to assay bioluminescence signal values.

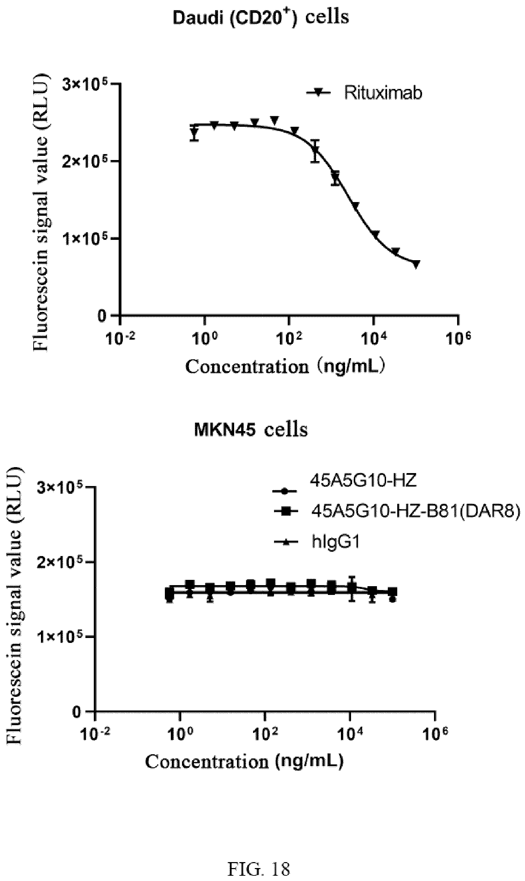

The detection results of the CDC activities of 45A5G10-HZ-B81 (DAR8) and 45A5G10-HZ on the MKN45 cells expressing the c-MET antigens are shown in FIG. 18. The results show that when the complements are present, both 45A5G10-HZ-B81 (DAR8) and 45A5G10-HZ do not mediate the CDC effect at all the dose levels.

### • 4.7: Cross-detection of anti-c-MET antibodies and human, monkey, rat, and mouse cMet

Antigen proteins human c-MET (purchased from ACRO Biosystems, Cat. No. MET-H5227), cynomolgus c-MET (purchased from ACRO Biosystems, Cat. No. MET-C52H9), rat c-MET (purchased from Sino biological, Cat. No. 80004-R08H), mouse c-MET (purchased from Sino biological, Cat. No. 50622-M08H) are diluted with a CBS to 1 ug/mL, then the antigens are coated, and blocked with 2% BSA(in PBS) at 37°C for 1.5 hours, 45A5G10-HZ-B81 (DAR8) and 45A5G10-HZ that are gradiently diluted (starting at 1 ug/mL for 4-fold dilution with 8 concentration points) are added for incubation at 37°C for 1 hour, HRP-labeled secondary antibodies (Goat Anti-Human IgG, Monkey ads-HRP, purchased from SouthernBiotech, Cat. No. F4522-V172E) are added for incubation at 37°C for 1 hour, a TMB substrate is added for color development, and after the color development is stopped using a stop solution, final products are added into a machine to read out an OD value of each well at 450 nm (reference wavelength 630 nm);

The experimental results are as shown in Table 20.

**Table 20. Species cross-reactions of 45A5G10-HZ or 45A5G10-HZ-B81 (DAR8)**

| **Protein** | **EC₅₀ (ng/mL)** | | **EC₅₀ (nM)** | | **Maximum OD (450-630 nm)** | |
|---|---|---|---|---|---|---|
| | **45A5G10-HZ** | **45A5G10-HZ-B81 (DAR8)** | **45A5G10-HZ** | **45A5G10-HZ-B81 (DAR8)** | **45A5G10-HZ** | **45A5G10-HZ-B81 (DAR8)** |
| Human c-MET | 25.43 | 23.63 | 0.17 | 0.15 | 2.2920 | 2.1736 |
| Monkey c-MET | 29.64 | 32.42 | 0.20 | 0.21 | 2.2768 | 2.2009 |
| Rat c-MET | NA | NA | NA | NA | NA | NA |
| Mouse c-MET | NA | NA | NA | NA | NA | NA |

45A5G10-HZ or 45A5G10-HZ-B81 (DAR8) only specifically binds to the human c-MET and the monkey c-MET. The EC50 values of species protein binding of 45A5G10-HZ to the human c-MET and the monkey c-MET are 25.43 ng/mL and 29.64 ng/mL, respectively. The EC50 values of species protein binding of 45A5G10-HZ-B81 (DAR8) to the human c-MET and the monkey c-MET are 23.63 ng/mL and 32.42 ng/mL, respectively.

The results show that the binding ability of 45A5G10-HZ-B81 (DAR8) to c-MET antigens of different species is similar to that of 45A5G10-HZ.

### Embodiment 5. In-vivo evaluation of anti-human cMet-ADCs

### 5.1 Test of efficacy of anti-human cMet-ADCs on non-small cell lung cancer transplanted tumors

### Test 1

To verify the *in-vivo* efficacy of the anti-human cMet ADC drugs and evaluate the anti-tumor effects of the tested drugs in female Balb/c Nude mouse models subj ected to subcutaneous xenografts, 5-6 week old female Balb/c Nude mice (Vital River) are purchased, human non-small cell lung cancer NCI-H358 cells that have grown to the logarithmic growth phase are digested with EDTA and then resuspended with PBS, each mouse is subcutaneously inoculated with 5*10⁶ cells, and when the tumor grows to a size of 100-200 m³, intravenous administration is performed once a week with 1 mg/kg or 3 mg/kg each time.

The main observation indicators of this experiment are: 1) TGI (%), which is calculated according to the formula: TGI (%) = (1 - T/C) x 100% (T and C are relative tumor volumes of the treatment group and the control group at a specific time point, respectively); 2) photos of tumor volumes and tumor weights at the end of the experiment.

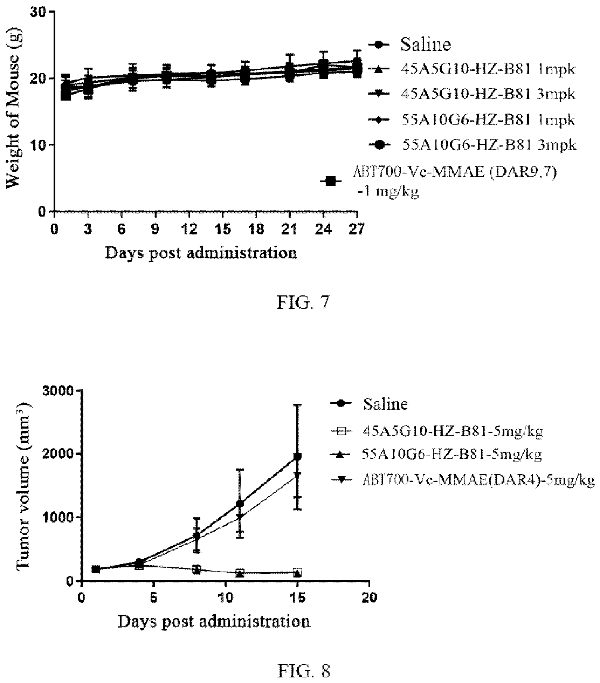

The experimental results are shown in Table 21 and FIG. 6. 45A5G10-Hz-B81 (DAR8) and 55A10G6-Hz-B81 (DAR8) both have good tumor inhibitory effects in the mice, and have better tumor inhibition effects than that of ABT700-VcMMAE at the dosage of 1 mg/kg. The changes of weights of the mice are shown in FIG. 7, indicating that the ADCs have no effect on the weights of the mice during the entire administration process.

**Table 21. In-vivo efficacy of anti-human cMet ADCs in NCI-H358 CDX models**

| Grouping | Amount | Sample Name | Dosage (mg/kg) | Administration method | Administration frequency | TGI (%) on Day 14 | P value |
|---|---|---|---|---|---|---|---|
| 1 | 5 | Saline | / | i.v. | QW*3 | / | / |
| 2 | 5 | 45A5G10-HZ-B81 (DAR8) | 1 | i.v. | QW*3 | 93.9 | <0.001 |
| 3 | 5 | 45A5G10-Hz-B81 (DAR8) | 3 | i.v. | QW*3 | 93.1 | <0.001 |
| 4 | 5 | 55A10G6-HZ-B81 (DAR8) | 1 | i.v. | QW*3 | 93.8 | <0.001 |
| 5 | 5 | 55A10G6-HZ-B81 (DAR8) | 3 | i.v. | QW*3 | 93.9 | <0.001 |
| 6 | 5 | ABT700-Vc-MMAE (DAR9.7) | 1 | i.v. | QW*3 | 84.6 | <0.001 |

### Test 2

The objective of this experiment is to evaluate the anti-tumor efficacy of the tested product 45A5G10-HZ-B81 (DAR8) in the transplanted tumor models of the human non-small cell lung cancer cells NCI-H358.

Female BALB/c Nu nude mice are subcutaneously inoculated with the NCI-H358 cells, when the tumors grow to a size of about 180 mm³ (no more than 200 mm³), 56 animals are screened and divided into 7 groups, including a Vehicle group (saline), a dosage group of toxins (toxin small molecules released by 45A5G10-HZ-B81 (DAR8), which can be prepared with reference to Embodiment A1.9 of WO2022170971) of 0.07 mg/kg, a dosage group of IgG1-B81 (isotype control antibody ADCs, obtained with reference to IgG1-ADC-07 preparation in Embodiment 4.6.1 of WO2022170971) of 3 mg/kg, and dosage groups of 45A5G10-HZ-B81 (DAR8) of 0.3, 1, and 3 mg/kg, and there are 8 animals in each group. Animals in each group are administered via tail vein injection once a week (QW) for 3 consecutive weeks. The day of grouping is set as the 1st day (Day 1) of the experiment, the administration begins on the 1st day, after the last tumor test (the 22nd day after the first administration, Day 22) ends, the animals are sacrificed to isolate the tumors for weighing.

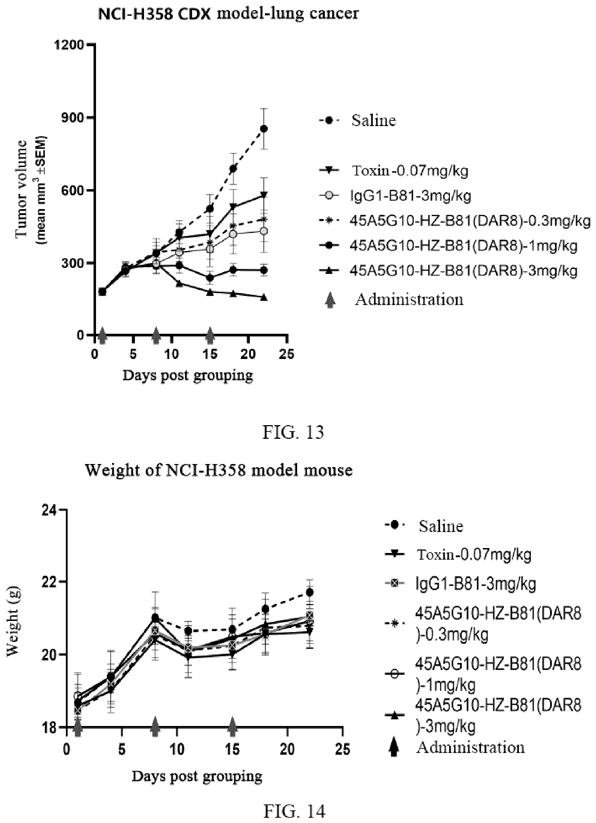

The results are shown in Table 22 and FIG. 13. The tumor volumes of animals in the dosage groups of 45A5G10-HZ-B81 (DAR8) of 0.3, 1, and 3 mg/kg are significantly lower than those in the Vehicle group (P < 0.01 or P < 0.001), and the tumor growth inhibition (TGI) rates are 43.4%, 68.0%, and 81.1%, respectively, which are positively correlated with the dosages. The tumor volumes of the animals in the group of IgG1-B81 of 3 mg/kg is significantly lower than those in the Vehicle group (P < 0.01), and the tumor growth inhibition (TGI) rate is 49.3%, indicating that the anti-c-Met antibody-drug conjugates and drug linkers (toxin-linkers) thereof provided by the present disclosure can realize anti-tumor effects but not require the endocytosis of antibodies or tumor cells to show positive expression of antigens.

The measurement results of animal weights are shown in FIG. 14. When the experiment ends (Day 22), the average weight and the change rate of the weights of the animals in the dosage groups of 45A5G10-HZ-B81 (DAR8) continue to increase during the administration process, which have no significant difference compared to the Vehicle group (P > 0.05).

FIG. 22. *In-vivo* efficacy of anti-human cMet ADCs in NCI-H358 transplanted tumor models

| Grouping | Dosage (mg/kg) | Average tumor volume (mm³, Mean ± SD) Day22 | T/C (%) Day22 | Tumor growth inhibition TGI (%) Day22 | P value |
|---|---|---|---|---|---|
| Vehicle | / | 853.93±237.3 | - | - | - |
| Toxin | 0.07 | 578.44±208.02 | 67.9 | 32.1 | 0.149 |
| IgG1-B81 | 3 | 432.05 ± 246.59*** | 50.7 | 49.3 | 0.001 |
| 45A5G10-HZ-B81 (DAR8) | 0.3 | 480.85 ± 256.49** | 56.6 | 43.4 | 0.009 |
| 45A5G10-HZ-B81 (DAR8) | 1 | 271.85 ± 69.29*** | 32.0 | 68.0 | < 0.001 |
| 45A5G10-HZ-B81 (DAR8) | 3 | 160.18 ± 29.36*** | 18.9 | 81.1 | < 0.001 |

### 5.2 Test of efficacy of anti-human cMet-ADCs on human colon cancer CDX models

A similar method is used to evaluate the anti-tumor effects of anti-cMet ADC test drugs in female BALB/c nude mouse animal models subjected to subcutaneous xenografts of human colon cancer SW480 cell lines.

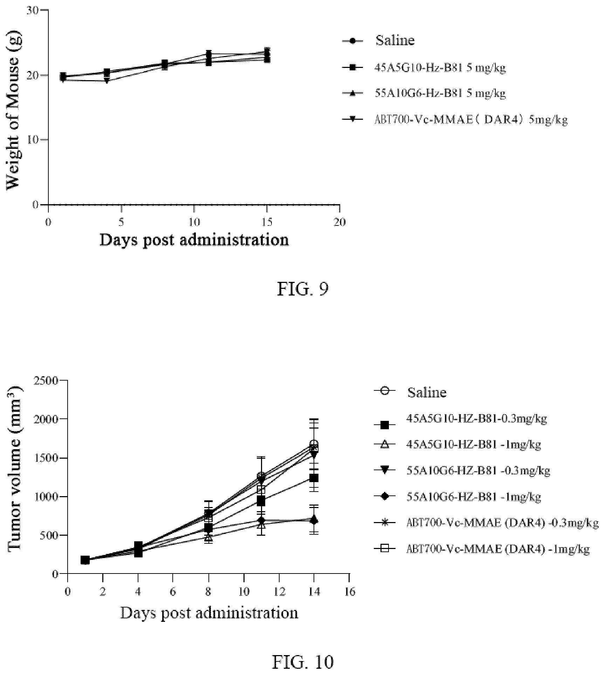

The experimental results are shown in Table 23 and FIG. 8. 45A5G10-Hz-B81 (DAR8) and 55A10G6-Hz-B81 (DAR8) both have good tumor inhibitory effects on tumor cells with low expression of human cMet in the mice, and the TGI at the dosage of 5 mg/kg is significantly higher than that of ABT700-VcMMAE (DAR4). The changes of weights of the mice are shown in FIG. 9, indicating that the ADCs have no effect on the weights of the mice during the entire administration process.

FIG. 23. *In-vivo* efficacy of anti-human cMet ADCs in SW480 CDX models

| Grouping | Number of Mice | Sample | Dosage (mg/kg) | Administration method | Administration frequency | TGI (%) on Day 15 | P value |
|---|---|---|---|---|---|---|---|
| 1 | 5 | Saline | / | i.v. | QW*2 | / | / |
| 2 | 5 | 45A5G10-HZ-B81 (DAR8) | 5 | i.v. | QW*2 | 92.92 | <0.01 |
| 3 | 5 | 55A10G6-HZ-B81 (DAR8) | 5 | i.v. | QW*2 | 93.7 | <0.01 |
| 4 | 5 | ABT700-Vc-MMAE (DAR4) | 5 | i.v. | QW*2 | 15.06 | <0.1 |

### 5.3 Test of efficacy of anti-human cMet-ADCs on human colorectal adenocarcinoma CDX models

A similar method is used to evaluate the anti-tumor effects of cMet ADC test drugs in female BALB/c nude mouse animal models subjected to subcutaneous xenografts of human colorectal adenocarcinoma NCI-H716 cell lines.

The experimental results are shown in Table 24 and FIG. 10. 45A5G10-Hz-B81 (DAR8) and 55A10G6-Hz-B81 (DAR8) both have good tumor inhibitory effects in the mice, and the TGI at the dosage of 1 mg/kg is significantly higher than that of ABT700-VcMMAE (DAR4). The changes of weights of the mice are shown in FIG. 11, indicating that the ADCs have no effect on the weights of the mice during the entire administration process.

**Table 24. In-vivo efficacy of anti-human cMet ADCs in NCI-H716 CDX models**

| Grouping | Number of Mice | Sample | Dosage (mg/kg) | Administration method | Administration frequency | TGI (%) on Day 14 |
|---|---|---|---|---|---|---|
| 1 | 5 | Saline | / | i.v. | QW*3 | / |
| 2 | 5 | 45A5G10-HZ-B81 (DAR8) | 0.3 | i.v. | QW*3 | 24.7 |
| 3 | 5 | 45A5G10-HZ-B81 (DAR8) | 1 | i.v. | QW*3 | 56.7 |
| 4 | 5 | 55A10G6-HZ-B81 (DAR8) | 0.3 | i.v. | QW*3 | 7.6 |
| 5 | 5 | 55A10G6-HZ-B81 (DAR8) | 1 | i.v. | QW*3 | 58.6 |
| 6 | 5 | ABT700-Vc-MMAE (DAR4) | 0.3 | i.v. | QW*3 | 1.7 |
| 7 | 5 | ABT700-Vc-MMAE (DAR4) | 1 | i.v. | QW*3 | 3.5 |

### 5.4 Test of efficacy of anti-human cMet-ADCs on human colorectal adenocarcinoma PDX models

The objective of this experiment is to evaluate the anti-tumor effects of the tested product 45A5G10-HZ-B81 (DAR8) in human colorectal cancer CR5088 PDX models (from Crown Bioscience Co., Ltd.).

NOD/SCID mice are subcutaneously inoculated with CR5088 tumor blocks (from Crown Bioscience Co., Ltd.), to establish subcutaneous xenograft models of human colorectal cancer. The experiment is divided into 3 dosage groups of the tested drug 45A5G10-HZ-B81 (DAR8) (1 mg/kg, 3 mg/kg, and 10 mg/kg) and a vehicle control group, which are a total of 4 groups with 8 animals in each group. Administration is performed once a week for a total of 3 weeks. The tumor growth inhibition (TGI) rates are calculated based on the tumor volumes for efficacy evaluation, and the safety is evaluated based on the changes of weights and death of the animals.

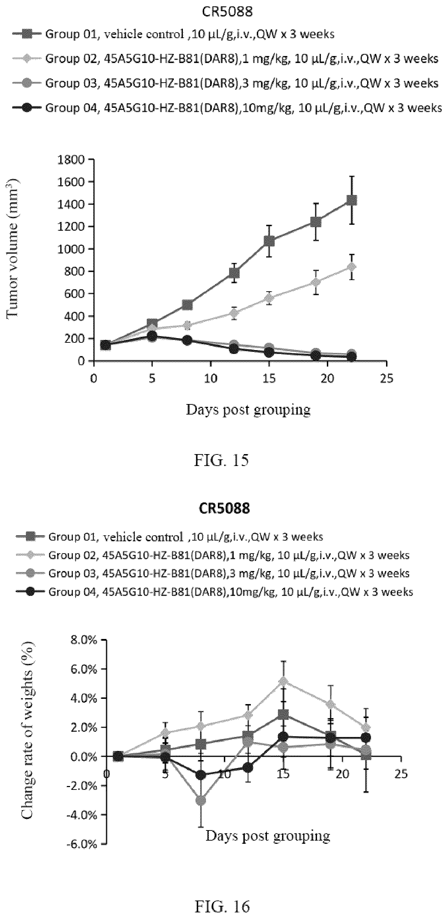

The results of the tumor volumes are shown in Table 25 and FIG. 15. 45A5G10-HZ-B81 (DAR8) shows a tendency to inhibit tumor growth at the dosages of 1 mg/kg, 3 mg/kg, and 10 mg/kg, and the tumor growth inhibition rates are 41.54%, 95.89%, and 97.56%, respectively, wherein the two dosage groups of 3 mg/kg and 10 mg/kg both have statistically significant differences compared to the blank control group, with P values less than 0.001.

The results of changes of the animal weights are shown in FIG. 16. No mice in the groups experience serious weight loss (BWL < 15%), there are no unexpected deaths of the mice during the experiment, and the mice are well tolerated.

**Table 25. Efficacy of anti-human cMet-ADCs in human colorectal adenocarcinoma PDX models**

| Group | Administration treatment (N = 8 mice/group) | Day 22 | | |
|---|---|---|---|---|
| | | Tumor volume (mm3) | TGI | P value |
| 1 | Blank control group | 1435.33 +/- 214.85 | / | / |
| 2 | 45A5G10-HZ-B81 (DAR8), 1 mg/kg | 839.07 +/- 113.87 | 41.54% | <0.1 |
| 3 | 45A5G10-HZ-B81 (DAR8), 3 mg/kg | 59.06 +/- 10.14 | 95.89% | <0.001 |
| 4 | 45A5G10-HZ-B81 (DAR8), 10mg/kg | 35.03 +/- 3.95 | 97.56% | <0.001 |

### 5.5 Test of efficacy of anti-human cMet-ADCs on gastric cancer transplanted tumors

The objective of this experiment is to evaluate the anti-tumor efficacy of the tested product 45A5G10-HZ-B81 (DAR8) in the transplanted tumor models of human gastric cancer MKN45.

Female NCG mice (InnoModels Biotechnology (Beijing) Co., Ltd.) are subcutaneously inoculated with MKN45 cells (from JCBR JCRB0254), to establish human gastric cancer models. The experiment is divided into a saline control group, a toxin (toxins released by 45A5G10-HZ-B81 (DAR8), 0.23 mg/kg) treatment group, 45A5G10-HZ (10 mg/kg), an IgG1-B81 (10 mg/kg) treatment group, a 45A5G10-HZ-B81 (DAR8) (1 mg/kg) treatment group, a 45A5G10-HZ-B81 (DAR8) (3 mg/kg) treatment group, and a 45A5G10-HZ-B81 (DAR8) (10 mg/kg) treatment group, and there are 8 mice in each group. The efficacy is evaluated based on the tumor growth inhibition (TGI) rates, and the safety is evaluated based on the changes of weights and death of the animals.

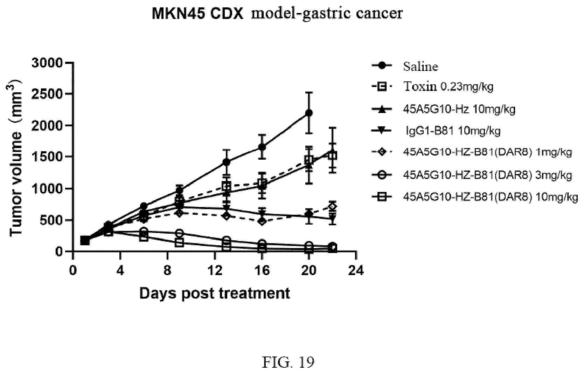

The results of tumor volumes are shown in Table 26 and FIG. 19. 45A5G10-HZ has the effect of inhibiting the growth of the subcutaneous transplanted tumors of human-derived gastric cancer MKN45. 45A5G10-HZ-B81 (DAR8) is injected via the tail vein at dosages of 1 mg/kg, 3 mg/kg, and 10 mg/kg, once a week for 3 consecutive times, and the growth of the subcutaneous transplanted tumors of human gastric cancer MKN45 is significantly inhibited, and the relative tumor growth inhibition TGI rates are 72%, 96%, and 98%, respectively, and all have statistically significant differences compared to the vehicle control group. The tumor volumes of the animals in the group of IgG1-B81 of 10 mg/kg is significantly lower than those in the physiological group (P < 0.01), and the tumor growth inhibition (TGI) rate is 75%, indicating that the anti-c-Met antibody-drug conjugates and drug linkers (toxin-linkers) thereof provided by the present disclosure can realize anti-tumor effects but not require the endocytosis of antibodies or tumor cells to show positive expression of antigens.

**Table 26: Test data of efficacy of transplanted tumor models of NCI-H358 cells**

| **Set No.** | **Administration** | **Tumor volume on Day 22 (mm³) (*x̅* ± S)** | **Relative tumor volume on Day22 ( *x̅* ± S)** | **TGI (%)** | **T/C (%)** |
|---|---|---|---|---|---|
| 1 | **Saline** | 2200.07±324.17 | 12.64±1.92 | -- | -- |
| 2 | Toxin 0.23 mg/kg | 1448.68±178.45 | 8.52±1.26 | 33 | 67 |
| 3 | 45A5G10-HZ 10 mg/kg | 1370.06±297.24 | 8.02±1.92 | 37 | 63 |
| 4 | IgG1-B81 10 mg/kg | 558.23±117.79 | 3.14±0.61 | 75 | 25 |
| 5 | 45A5G10-HZ-B81 (DAR8) 1 mg/kg | 598.96±46.95 | 3.54±0.41 | 72 | 28 |
| 6 | 45A5G10-HZ-B81 (DAR8) 3 mg/kg | 95.1±13.55 | 0.5±0.08 | 96 | 4 |
| 7 | 45A5G10-HZ-B81 (DAR8) 10 mg/kg | 36.09±4.24 | 0.2±0.02 | 98 | 2 |

| | | | | | |
|---|---|---|---|---|---|
| Note: 1. The data is expressed in "Mean ± Standard error". | | | | | |

2. T/C % = TRTV/CRTV x 100%; TGI % = (1 - T/C) x 100% (TRTV: average RTV of the treatment group; CRTV: average RTV of the vehicle control group; and RTV = Vt/V0, wherein V0 is the tumor volume of the animal at the time of grouping, and Vt is the tumor volume of the animal after treatment).

### Embodiment 6. Stability test of plasma of c-MET antibody-drug conjugates

The stability of 45A5G10-HZ-B81 (DAR8) in plasma is evaluated by assays of the release of bioactive molecular toxins in human plasma incubated with 45A5G10-HZ-B81 (DAR8).

### Experimental system information

| | |
|---|---|
| Tested drug | 45A5G10-HZ-B81 (DAR8) |
| Test concentration | 100 µg/mL |
| Test condition | 37°C, a number of duplicate samples is 3 |
| Time point | 0 h ± 10 s, 24 h ± 10 min, 48 h ± 10 min, 72 h ± 15 min, 168 h ± 15 min, 240 h ± 15 min, 336 h ± 15 min, 504 h ± 15 min |
| test system | PBS of 0.1 M (control group) cyno plasma and human plasma (each experimental group) |
| Analytical method | LC-MS/MS |

### 1. Experimental steps

### Plasma preparation:

Frozen plasma is quickly thawed at 37°C, and the plasma needs to be placed on ice before use.

The thawed plasma is filtered with a 0.22 µm filter membrane and is used immediately after filtration.

Dilution process of tested drug:
Step 1 Prepare a drug diluent of 1 mg/mL: Take a certain volume of 45A5G10-HZ-B81 (DAR8) with a concentration of 26.6 mg/mL, and add the corresponding volume of PBS of 0.1 M to prepare a drug diluent of 1 mg/mL, for use after filtration with a 0.22 µm filter membrane.
Step 2 Prepare stability samples: Take a certain volume of plasma or PBS of 0.1 M and add to the drug diluent to prepare a system with a concentration of 100 µg/mL, mix well and gently, and dispense 200 µL into an EP tube (centrifuge tube) (at the all time points, this operation is performed on ice), and seal well.

0 min sample: Immediately add 5-fold volume of methanol after the corresponding sample is dispensed in step 2, mix well in vortex for 2 min, centrifuge (4°C, 17000 xg) for 10 min, then take the supernatant, and froze the supernatant at -60°C or below for being tested.

The stability samples prepared above are incubated at 37°C, and are precipitated with 5-fold volume of methanol at 24 h ± 10 min, 48 h ± 10 min, 72 h ± 15 min, 168 h ± 15 min, 240 h ± 15 min, 336 h ± 15 min, and 504 h ± 15 min, and then the supernatant is frozen at -60°C or below for being tested. Free small molecules are detected using an LC-MS/MS method.

### 2. Sample detection

The concentrations of small molecular drugs in all samples of each group are detected using the LC-MS/MS method.

### 3. Data processing

Calculation of the theoretical total concentration of free small molecules: Ctotal (toxin) = (C administration concentration of (45A5G10-HZ-B81 (DAR8))/molecular weight of 45A5G10-HZ-B81 (DAR8)) x antibody conjugate ratio (DAR) x molecular weight of toxin Release percentage of free small molecular drugs (%) = C at each time point (toxin)/Ctotal (toxin) × 100%

The concentration data at each time point is rounded to 3 significant figures, and the mean, standard deviation, and release percentage are rounded to 2 decimal places.

### 4. Results

The concentrations and release percentages of toxins in plasma of different species at each time point are summarized in the following Table 27,

| **Substrate** | **Time (h)** | **Toxin release percentage (%)** |
|---|---|---|
| PBS | 0 | 0.23 |
| | 24 | 0.21 |
| | 48 | 0.20 |
| | 72 | 0.21 |
| | 168 | 0.23 |
| | 240 | 0.30 |
| | 336 | 0.43 |
| | 504 | 0.58 |
| Cyno Plasma | 0 | 0.07 |
| | 24 | 0.09 |
| | 48 | 0.11 |
| | 72 | 0.13 |
| | 168 | 0.19 |
| | 240 | 0.24 |
| | 336 | 0.31 |
| | 504 | 0.44 |
| Human Plasma | 0 | 0.06 |
| | 24 | 0.09 |
| | 48 | 0.11 |
| | 72 | 0.13 |
| | 168 | 0.19 |
| | 240 | 0.24 |
| | 336 | 0.27 |
| | 504 | 0.38 |

The results show that after 45A5G10-HZ-B81 (DAR8) is incubated at 37°C for 504 h, the release percentages of toxins in the PBS solution, cyno plasma, and human plasma are 0.58%, 0.44%, and 0.38%, respectively. As the incubation time increases, the release of toxins of all substrates assayed have no significant increase (< 0.6%), confirming that the ADCs have good plasma stability and circulation stability. ADCs of other embodiments of the present application also show good plasma stability and circulation stability in the same test experiment, such as having a release percentage of toxins of less than 1%.

Although the specific embodiments of the present disclosure have been described in detail, those skilled in the art will understand that various modifications and changes can be made to the details based on all teachings that have been published, and these changes fall within the scope of the present disclosure. The scope of the present disclosure is provided by the appended claims and any equivalents thereof.

## Claims

1. An anti-c-MET antibody or antigen-binding fragment thereof, comprising a heavy chain variable region and a light chain variable region, wherein the CDRs of the heavy chain variable region and/or the CDRs of the light chain variable region have sequences identical to the sequences of the CDRs of a sequence-defined antibody or have 1, 2, or 3 amino acid substitutions compared to the CDRs of the sequence-defined antibody, wherein the sequence-defined antibody comprises:
(1) the amino acid sequence of a heavy chain variable region set forth in SEQ ID NO: 2, 4, 6, 8, 10, or 12; and/or
(2) the amino acid sequence of a light chain variable region set forth in SEQ ID NO: 1, 3, 5, 7, 9, or 11.

2. The anti-c-MET antibody or antigen-binding fragment thereof according to claim 1,
comprising: the HCDR1, the HCDR2, and the HCDR3 comprised in the heavy chain variable region set forth in SEQ ID NO: 10; and the LCDR1, the LCDR2, and the LCDR3 comprised in the light chain variable region set forth in SEQ ID NO: 9;
the HCDR1, the HCDR2, and the HCDR3 comprised in the heavy chain variable region set forth in SEQ ID NO: 12; and the LCDR1, the LCDR2, and the LCDR3 comprised in the light chain variable region set forth in SEQ ID NO: 11;
the HCDR1, the HCDR2, and the HCDR3 comprised in the heavy chain variable region set forth in SEQ ID NO: 2; and the LCDR1, the LCDR2, and the LCDR3 comprised in the light chain variable region set forth in SEQ ID NO: 1;
the HCDR1, the HCDR2, and the HCDR3 comprised in the heavy chain variable region set forth in SEQ ID NO: 4; and the LCDR1, the LCDR2, and the LCDR3 comprised in the light chain variable region set forth in SEQ ID NO: 3;
the HCDR1, the HCDR2, and the HCDR3 comprised in the heavy chain variable region set forth in SEQ ID NO: 6; and the LCDR1, the LCDR2, and the LCDR3 comprised in the light chain variable region set forth in SEQ ID NO: 5; or
the HCDR1, the HCDR2, and the HCDR3 comprised in the heavy chain variable region set forth in SEQ ID NO: 8; and the LCDR1, the LCDR2, and the LCDR3 comprised in the light chain variable region set forth in SEQ ID NO: 7; wherein
preferably, the HCDRs 1-3 and the LCDRs 1-3 are determined according to the definition scheme of IMGT, Kabat, or Chothia.

3. An anti-c-MET antibody or antigen-binding fragment thereof, comprising a heavy chain variable region and a light chain variable region, wherein
a. the heavy chain variable region comprises the HCDR1, the HCDR2, and the HCDR3, which comprise the amino acid sequences set forth in SEQ ID NO: 13, SEQ ID NO: 14, and SEQ ID NO: 15, respectively; and the light chain variable region comprises the LCDR1, the LCDR2, and the LCDR3, which comprise the amino acid sequences set forth in SEQ ID NO: 20, SEQ ID NO: 21, and SEQ ID NO: 22, respectively;
b. the heavy chain variable region comprises the HCDR1, the HCDR2, and the HCDR3, which comprise the amino acid sequences set forth in SEQ ID NO: 16, SEQ ID NO: 17, and SEQ ID NO: 19, respectively; and the light chain variable region comprises the LCDR1, the LCDR2, and the LCDR3, which comprise the amino acid sequences set forth in SEQ ID NO: 23, SEQ ID NO: 24, and SEQ ID NO: 22, respectively;
c. the heavy chain variable region comprises the HCDR1, the HCDR2, and the HCDR3, which comprise the amino acid sequences set forth in SEQ ID NO: 16, SEQ ID NO: 18, and SEQ ID NO: 19, respectively; and the light chain variable region comprises the LCDR1, the LCDR2, and the LCDR3, which comprise the amino acid sequences set forth in SEQ ID NO: 23, SEQ ID NO: 24, and SEQ ID NO: 22, respectively;
d. the heavy chain variable region comprises the HCDR1, the HCDR2, and the HCDR3, which comprise the amino acid sequences set forth in SEQ ID NO: 25, SEQ ID NO: 53, and SEQ ID NO: 19, respectively; and the light chain variable region comprises the amino acid sequences set forth in SEQ ID NO: 23, SEQ ID NO: 24, and SEQ ID NO: 22, respectively;
e. the heavy chain variable region comprises the HCDR1, the HCDR2, and the HCDR3, which comprise the amino acid sequences set forth in SEQ ID NO: 26, SEQ ID NO: 27, and SEQ ID NO: 28, respectively; and the light chain variable region comprises the LCDR1, the LCDR2, and the LCDR3, which comprise the amino acid sequences set forth in SEQ ID NO: 33, SEQ ID NO: 34, and SEQ ID NO: 35, respectively;
f. the heavy chain variable region comprises the HCDR1, the HCDR2, and the HCDR3, which comprise the amino acid sequences set forth in SEQ ID NO: 29, SEQ ID NO: 30, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively;
g. the heavy chain variable region comprises the HCDR1, the HCDR2, and the HCDR3, which comprise the amino acid sequences set forth in SEQ ID NO: 29, SEQ ID NO: 31, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively;
h. the heavy chain variable region comprises the HCDR1, the HCDR2, and the HCDR3, which comprise the amino acid sequences set forth in SEQ ID NO: 54, SEQ ID NO: 55, and SEQ ID NO: 32, respectively; and the light chain variable region comprises the LCDR1, the LCDR2, and the LCDR3, which comprise the amino acid sequences set forth in SEQ ID NO: 36, SEQ ID NO: 37, and SEQ ID NO: 35, respectively;
i. the heavy chain variable region comprises the HCDR1, the HCDR2, and the HCDR3, which comprise the amino acid sequences set forth in SEQ ID NO: 39, SEQ ID NO: 40, and SEQ ID NO: 41, respectively; and the light chain variable region comprises the LCDR1, the LCDR2, and the LCDR3, which comprise the amino acid sequences set forth in SEQ ID NO: 45, SEQ ID NO: 46, and SEQ ID NO: 47, respectively;
j. the heavy chain variable region comprises the HCDR1, the HCDR2, and the HCDR3, which comprise the amino acid sequences set forth in SEQ ID NO: 42, SEQ ID NO: 43, and SEQ ID NO: 44, respectively; and the light chain variable region comprises the LCDR1, the LCDR2, and the LCDR3, which comprise the amino acid sequences set forth in SEQ ID NO: 48, SEQ ID NO: 49, and SEQ ID NO: 50, respectively; or
k. the heavy chain variable region comprises the HCDR1, the HCDR2, and the HCDR3, which comprise the amino acid sequences set forth in SEQ ID NO: 62, SEQ ID NO: 60, and SEQ ID NO: 44, respectively; and the light chain variable region comprises the LCDR1, the LCDR2, and the LCDR3, which comprise the amino acid sequences set forth in SEQ ID NO: 48, SEQ ID NO: 49, and SEQ ID NO: 50, respectively; wherein
preferably,
a. the heavy chain variable region comprises the HCDR1, the HCDR2, and the HCDR3 set forth in SEQ ID NO: 13, SEQ ID NO: 14, and SEQ ID NO: 15, respectively; and the light chain variable region comprises the LCDR1, the LCDR2, and the LCDR3 set forth in SEQ ID NO: 20, SEQ ID NO: 21, and SEQ ID NO: 22, respectively; wherein
the foregoing HCDRs 1-3 and LCDRs 1-3 are determined according to the definition scheme of IMGT;
b. the heavy chain variable region comprises the HCDR1, the HCDR2, and the HCDR3 set forth in SEQ ID NO: 16, SEQ ID NO: 17, and SEQ ID NO: 19, respectively; and the light chain variable region comprises the LCDR1, the LCDR2, and the LCDR3 set forth in SEQ ID NO: 23, SEQ ID NO: 24, and SEQ ID NO: 22, respectively; wherein
the foregoing HCDRs 1-3 and LCDRs 1-3 are determined according to the definition scheme of Kabat;
c. the heavy chain variable region comprises the HCDR1, the HCDR2, and the HCDR3 set forth in SEQ ID NO: 16, SEQ ID NO: 18, and SEQ ID NO: 19, respectively; and the light chain variable region comprises the LCDR1, the LCDR2, and the LCDR3 set forth in SEQ ID NO: 23, SEQ ID NO: 24, and SEQ ID NO: 22, respectively; wherein
the foregoing HCDRs 1-3 and LCDRs 1-3 are determined according to the definition scheme of Kabat;
d. the heavy chain variable region comprises the HCDR1, the HCDR2, and the HCDR3 set forth in SEQ ID NO: 25, SEQ ID NO: 53, and SEQ ID NO: 19, respectively; and the light chain variable region comprises the LCDR1, the LCDR2, and the LCDR3 set forth in SEQ ID NO: 23, SEQ ID NO: 24, and SEQ ID NO: 22, respectively; wherein
the foregoing HCDRs 1-3 and LCDRs 1-3 are determined according to the definition scheme of Chothia;
e. the heavy chain variable region comprises the HCDR1, the HCDR2, and the HCDR3 set forth in SEQ ID NO: 26, SEQ ID NO: 27, and SEQ ID NO: 28, respectively; and the light chain variable region comprises the LCDR1, the LCDR2, and the LCDR3 set forth in SEQ ID NO: 33, SEQ ID NO: 34, and SEQ ID NO: 35, respectively; wherein
the foregoing HCDRs 1-3 and LCDRs 1-3 are determined according to the definition scheme of IMGT;
f. the heavy chain variable region comprises the HCDR1, the HCDR2, and the HCDR3 set forth in SEQ ID NO: 29, SEQ ID NO: 30, and SEQ ID NO: 32, respectively; and the light chain variable region comprises the LCDR1, the LCDR2, and the LCDR3 set forth in SEQ ID NO: 36, SEQ ID NO: 37, and SEQ ID NO: 35, respectively; wherein
the foregoing HCDRs 1-3 and LCDRs 1-3 are determined according to the definition scheme of Kabat;
g. the heavy chain variable region comprises the HCDR1, the HCDR2, and the HCDR3 set forth in SEQ ID NO: 29, SEQ ID NO: 31, and SEQ ID NO: 32, respectively; and the light chain variable region comprises the LCDR1, the LCDR2, and the LCDR3 set forth in SEQ ID NO: 36, SEQ ID NO: 37, and SEQ ID NO: 35, respectively; wherein
the foregoing HCDRs 1-3 and LCDRs 1-3 are determined according to the definition scheme of Kabat;
h. the heavy chain variable region comprises the HCDR1, the HCDR2, and the HCDR3 set forth in SEQ ID NO: 54, SEQ ID NO: 55, and SEQ ID NO: 32, respectively; and the light chain variable region comprises the LCDR1, the LCDR2, and the LCDR3 set forth in SEQ ID NO: 36, SEQ ID NO: 37, and SEQ ID NO: 35, respectively; wherein
the foregoing HCDRs 1-3 and LCDRs 1-3 are determined according to the definition scheme of Chothia;
i. the heavy chain variable region comprises the HCDR1, the HCDR2, and the HCDR3 set forth in SEQ ID NO: 39, SEQ ID NO: 40, and SEQ ID NO: 41, respectively; and the light chain variable region comprises the LCDR1, the LCDR2, and the LCDR3 set forth in SEQ ID NO: 45, SEQ ID NO: 46, and SEQ ID NO: 47, respectively; wherein
the foregoing HCDRs 1-3 and LCDRs 1-3 are determined according to the definition scheme of IMGT;
j. the heavy chain variable region comprises the HCDR1, the HCDR2, and the HCDR3 set forth in SEQ ID NO: 42, SEQ ID NO: 43, and SEQ ID NO: 44, respectively; and the light chain variable region comprises the LCDR1, the LCDR2, and the LCDR3 set forth in SEQ ID NO: 48, SEQ ID NO: 49, and SEQ ID NO: 50, respectively; wherein
the foregoing HCDRs 1-3 and LCDRs 1-3 are determined according to the definition scheme of Kabat; or
k. the heavy chain variable region comprises the HCDR1, the HCDR2, and the HCDR3 set forth in SEQ ID NO: 62, SEQ ID NO: 60, and SEQ ID NO: 44, respectively; and the light chain variable region comprises the LCDR1, the LCDR2, and the LCDR3 set forth in SEQ ID NO: 48, SEQ ID NO: 49, and SEQ ID NO: 50, respectively; wherein
the foregoing HCDRs 1-3 and LCDRs 1-3 are determined according to the definition scheme of Chothia.

4. The anti-c-MET antibody or antigen-binding fragment thereof according to any one of claims 1-3, comprising the heavy chain variable region and the light chain variable region, wherein 4, 3, 2, or 1 CDR(s) of 6 CDRs of the HCDRs 1-3 and the LCDRs 1-3 comprised in the heavy chain variable region and the light chain variable region comprise to 1, 2, or 3 amino acid substitutions; wherein
preferably, the substitutions are conservative substitutions.

5. The anti-c-MET antibody or antigen-binding fragment thereof according to any one of claims 1-4, comprising the heavy chain variable region and the light chain variable region, wherein
(1) the heavy chain variable region has at least 90%, 91%, 92%, 93%, 94%, 95%, 96%, 97%, 98%, 99%, or 100% sequence identity to SEQ ID NO: 10, and/or the light chain variable region has at least 90%, 91%, 92%, 93%, 94%, 95%, 96%, 97%, 98%, 99%, or 100% sequence identity to SEQ ID NO: 9;
(2) the heavy chain variable region has at least 90%, 91%, 92%, 93%, 94%, 95%, 96%, 97%, 98%, 99%, or 100% sequence identity to SEQ ID NO: 12, and/or the light chain variable region has at least 90%, 91%, 92%, 93%, 94%, 95%, 96%, 97%, 98%, 99%, or 100% sequence identity to SEQ ID NO: 11;
(3) the heavy chain variable region has at least 90%, 91%, 92%, 93%, 94%, 95%, 96%, 97%, 98%, 99%, or 100% sequence identity to SEQ ID NO: 2, and/or the light chain variable region has at least 90%, 91%, 92%, 93%, 94%, 95%, 96%, 97%, 98%, 99%, or 100% sequence identity to SEQ ID NO: 1;
(4) the heavy chain variable region has at least 90%, 91%, 92%, 93%, 94%, 95%, 96%, 97%, 98%, 99%, or 100% sequence identity to SEQ ID NO: 4, and/or the light chain variable region has at least 90%, 91%, 92%, 93%, 94%, 95%, 96%, 97%, 98%, 99%, or 100% sequence identity to SEQ ID NO: 3;
(5) the heavy chain variable region has at least 90%, 91%, 92%, 93%, 94%, 95%, 96%, 97%, 98%, 99%, or 100% sequence identity to SEQ ID NO: 6, and/or the light chain variable region has at least 90%, 91%, 92%, 93%, 94%, 95%, 96%, 97%, 98%, 99%, or 100% sequence identity to SEQ ID NO: 5; or
(6) the heavy chain variable region has at least 90%, 91%, 92%, 93%, 94%, 95%, 96%, 97%, 98%, 99%, or 100% sequence identity to SEQ ID NO: 8, and/or the light chain variable region has at least 90%, 91%, 92%, 93%, 94%, 95%, 96%, 97%, 98%, 99%, or 100% sequence identity to SEQ ID NO: 7.

6. The anti-c-MET antibody or antigen-binding fragment thereof according to any one of claims 1-5, comprising the heavy chain variable region and the light chain variable region, wherein:
(1) the heavy chain variable region comprises or consists of the amino acid sequence set forth in SEQ ID NO: 10; and the light chain variable region comprises or consists of the amino acid sequence set forth in SEQ ID NO: 9;
(2) the heavy chain variable region comprises or consists of the amino acid sequence set forth in SEQ ID NO: 12; and the light chain variable region comprises or consists of the amino acid sequence set forth in SEQ ID NO: 11;
(3) the heavy chain variable region comprises or consists of the amino acid sequence set forth in SEQ ID NO: 2; and the light chain variable region comprises or consists of the amino acid sequence set forth in SEQ ID NO: 1;
(4) the heavy chain variable region comprises or consists of the amino acid sequence set forth in SEQ ID NO: 4; and the light chain variable region comprises or consists of the amino acid sequence set forth in SEQ ID NO: 3;
(5) the heavy chain variable region comprises or consists of the amino acid sequence set forth in SEQ ID NO: 6; and the light chain variable region comprises or consists of the amino acid sequence set forth in SEQ ID NO: 5; or
(6) the heavy chain variable region comprises or consists of the amino acid sequence set forth in SEQ ID NO: 8; and the light chain variable region comprises or consists of the amino acid sequence set forth in SEQ ID NO: 7.

7. The anti-c-MET antibody or antigen-binding fragment thereof according to any one of claims 1-6, further comprising a heavy chain constant region and a light chain constant region of the antibody; wherein
preferably, the heavy chain constant region is selected from a human IgG1, IgG2, IgG3, or IgG4 constant region, and the light chain constant region is selected from a human antibody κ or λ chain constant region; and
more preferably, the antibody comprises a heavy chain constant region set forth in SEQ ID NO: 51 and a light chain constant region set forth in SEQ ID NO: 52.

8. The anti-c-MET antibody or antigen-binding fragment thereof according to any one of claims 1-7, comprising:
a. a heavy chain having at least 85%, 90%, 91%, 92%, 93%, 94%, 95%, 96%, 97%, 98%, 99%, or 100% sequence identity to SEQ ID NO: 56, and/or a light chain having at least 85%, 90%, 91%, 92%, 93%, 94%, 95%, 96%, 97%, 98%, 99%, or 100% sequence identity to SEQ ID NO: 57; wherein
preferably, the anti-c-MET antibody comprises: a heavy chain comprising or consisting of the amino acid sequence set forth in SEQ ID NO: 56 and a light chain comprising or consisting of the amino acid sequence set forth in SEQ ID NO: 57; or
b. a heavy chain having at least 85%, 90%, 91%, 92%, 93%, 94%, 95%, 96%, 97%, 98%, 99%, or 100% sequence identity to SEQ ID NO: 58, and/or a light chain having at least 85%, 90%, 91%, 92%, 93%, 94%, 95%, 96%, 97%, 98%, 99%, or 100% sequence identity to SEQ ID NO: 59; wherein
preferably, the anti-c-MET antibody comprises: a heavy chain comprising or consisting of the amino acid sequence set forth in SEQ ID NO: 58 and a light chain comprising or consisting of the amino acid sequence set forth in SEQ ID NO: 59.

9. An anti-c-MET antibody or antigen-binding fragment thereof, wherein the antibody competes for binding to a human c-MET with the anti-c-MET antibody or antigen-binding fragment thereof according to any one of claims 1-8.

10. A multispecific antibody, comprising the anti-c-MET antibody or antigen-binding fragment thereof according to any one of claims 1-9, wherein
preferably, the multispecific antibody is a bispecific antibody, or a trispecific antibody, or a tetraspecific antibody.

11. A nucleic acid molecule, encoding the anti-c-MET antibody or antigen-binding fragment thereof according to any one of claims 1-10, or the multispecific antibody according to claim 10.

12. A host cell, comprising the nucleic acid molecule according to claim 11.

13. An immunoconjugate, comprising: the anti-c-MET antibody or antigen-binding fragment thereof according to any one of claims 1-9 and an effector molecule, wherein the effector molecule is conjugated to the anti-c-MET antibody; and
preferably, the effector molecule is selected from an anti-tumor agent, an immunomodulatory agent, a biological response modifier, a lectin, a cytotoxic drug, a chromophore, a fluorophore, a chemiluminescent compound, an enzyme, a metal ion, and any combination thereof.

14. A method for *in-vivo* and/or *in-vitro* immune detection or assay of c-MET, comprising a step of contacting the anti-c-MET antibody according to any one of claims 1-10 with a subject or a sample from the subject.

15. An antibody-drug conjugate, a stereoisomer thereof, a prodrug thereof, a pharmaceutically acceptable salt thereof, a tautomer thereof, or a pharmaceutically acceptable solvate thereof, comprising the antibody or antigen-binding fragment thereof according to any one of claims 1-9, or the multispecific antibody according to claim 10.

16. The antibody-drug conjugate, the stereoisomer thereof, the prodrug thereof, the pharmaceutically acceptable salt thereof, the tautomer thereof, or the pharmaceutically acceptable solvate thereof according to claim 15, wherein the antibody-drug conjugate has a structure as represented in Formula (I): 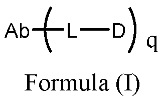 wherein:
Ab is the antibody or antigen-binding fragment thereof according to any one of claims 1-9, or the multispecific antibody according to claim 10;
D is an active drug unit;
q is an integer selected from 1-20, for example, selected from 1, 2, 3, 4, 5, 6, 7, 8, 9, 10, 11, 12, 13, or 14;
L is a linking group covalently attached to the antibody or antigen-binding fragment thereof Ab and the active drug unit D, respectively;
the L is covalently attached to an amino residue or a sulfhydryl residue on the antibody Ab;
preferably, the L is covalently attached to the sulfhydryl residue on the antibody Ab; and more preferably, the L is covalently attached to a sulfhydryl residue formed after an interchain disulfide bond on the antibody Ab being opened up; and
preferably, the active drug unit is selected from a cytotoxic agent; and more preferably, the active drug unit is selected from a DNA topoisomerase inhibitor (for example, a camptothecinbased biologically active molecule, such as camptothecin, DXD, camptothecin with a substitutent modified or DXD with a substitutent modified, hydroxycamptothecin, 9-aminocamptothecin, SN-38, irinotecan, topotecan, belotecan, and rubitecan) or a tubulin inhibitor (for example, an MMAF-like tubulin inhibitor, and an MMAE-like tubulin inhibitor).

17. The antibody-drug conjugate, the stereoisomer thereof, the prodrug thereof, the pharmaceutically acceptable salt thereof, the tautomer thereof, or the pharmaceutically acceptable solvate thereof according to claim 16, wherein the L is a cleavable linker or a non-cleavable linker,
preferably, the cleavable linker comprises a peptide unit comprising 2-10 amino acid residues selected from natural amino acid residues, non-natural amino acid residues, or amino acid residues set forth in AA₁ or stereoisomers thereof;
more preferably, the peptide unit is a dipeptide, tripeptide, tetrapeptide, pentapeptide, hexapeptide, heptapeptide, octapeptide, nonapeptide, or decapeptide comprising at least 1 (for example, 1, 2, or 3) amino acid residues set forth in AA₁ or stereoisomers thereof;
The amino acid residue set forth in AA¹ has a structure as follows, 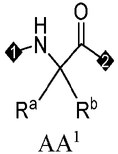
wherein:
either of R^{a} and R^{b} is H, and the other is 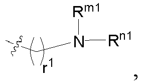 and r¹ is 4;
or R^{a} and R^{b}, taken together with the carbon atom to which they are attached, form a 5-6-membered heterocycle substituted with R⁰;
R^{m1} and Rⁿ¹ are each independently selected from hydrogen, C₁₋₆ alkyl, and C₃₋₆ cycloalkyl;
R⁰ is selected from C₁₋₆ alkyl, C₃₋₆ cycloalkyl, -NR^{m2}Rⁿ², and a 5-6-membered heterocyclyl optionally substituted with C₁₋₆ alkyl; and
R^{m2} and Rⁿ² are each independently selected from hydrogen and C₁₋₆ alkyl;
preferably, the amino acid residue is selected from -Val-, -Ala-, -Gly-, -Cit-, -AA¹-, -Arg-, - Phe-, -Lys-, and -Asn-; and
preferably, the peptide unit is selected from -valine-citrulline- (-Val-Cit-), -valine-alanine- (-Val-Ala-), -valine-lysine- (-Val-Lys-), -valine-arginine- (-Val-Arg-), -phenylalanine-citrulline- (-Phe-Cit-), -phenylalanine-lysine- (-Phe-Lys-), -phenylalanine-arginine- (-Phe-Arg-)-, -alanine-alanine-alanine- (-Ala-Ala-Ala-), -alanine-alanine-asparagine- (-Ala-Ala-Asn-), -valine-AA¹-glycine- (-Val-AA¹-Gly-), -valine-AA¹-alanine- (-Val-AA¹-Ala-), -glycine-glycine-phenylalanine-glycine- (-Gly-Gly-Phe-Gly-), and -glycine-glycine-valine-alanine- (-Gly-Gly-Val-Ala-).

18. The antibody-drug conjugate, the stereoisomer thereof, the prodrug thereof, the pharmaceutically acceptable salt thereof, the tautomer thereof, or the pharmaceutically acceptable solvate thereof according to claim 16 or 17, wherein the L is 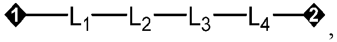 wherein,
L₁ is selected from: 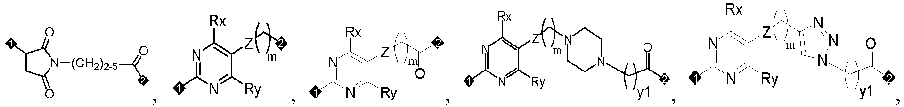 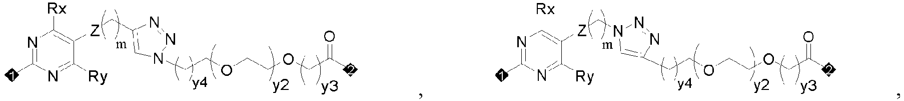 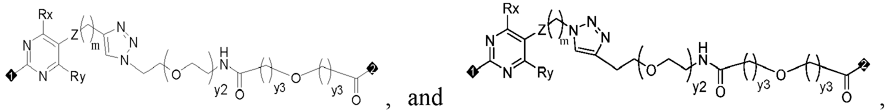 each Z is independently selected from a direct bond, a carbon-carbon triple bond, a carbon-carbon double bond, C₆₋₁₀ aryl, a 5-10-membered heteroaryl, and acylamino (preferably selected from a direct bond, a carbon-carbon triple bond, and a carbon-carbon double bond); Rx and Ry are independently selected from H and C₁₋₄ alkyl; each m is independently selected from 0, 1, 2, 3, 4, 5, and 6; y1 is selected from any integer from 1 to 6 (such as 4, 5, or 6); each y2 is independently selected from any integer from 0 to 15 (such as from 6 to 15); each y3 is independently selected from 1, 2, and 3; each y4 is independently selected from 0 and 1; the position 1 is attached to the antibody or antigen-binding fragment thereof via an S atom, and the position 2 is attached to L₂ or L₃;
L₂ is absent or present, when L₂ is present, L₂ is selected from 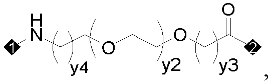 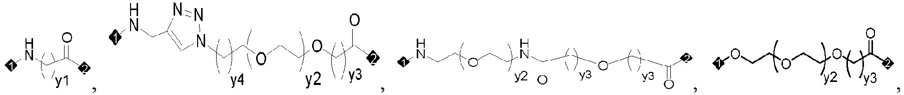 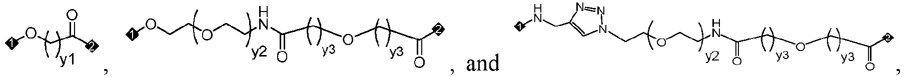 each y1 is selected from any integer from 1 to 6 (such as 4, 5, 6), each y2 is independently selected from any integer from 0 to 10 (such as from 6 to 10), each y3 is independently selected from 1 or 2, each y4 is independently selected from 0 or 1, the position 1 is attached to L₁, and the position 2 is attached to L₃;
L₃ is selected from 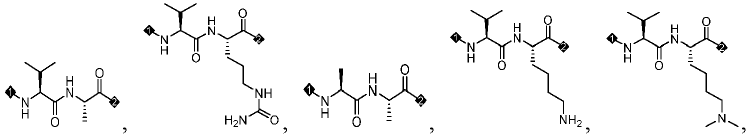 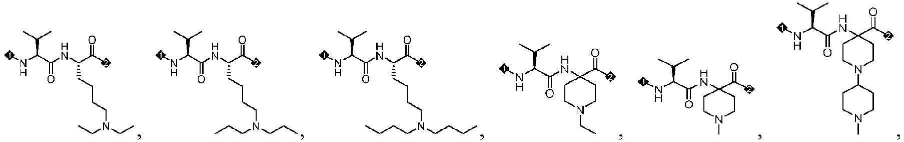 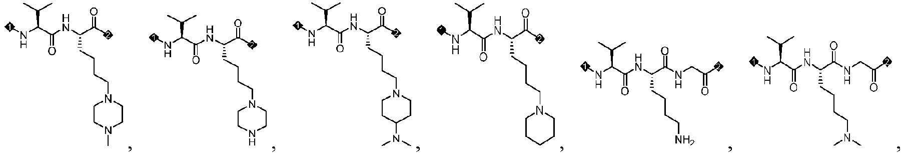 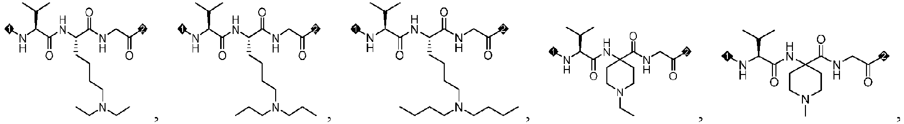 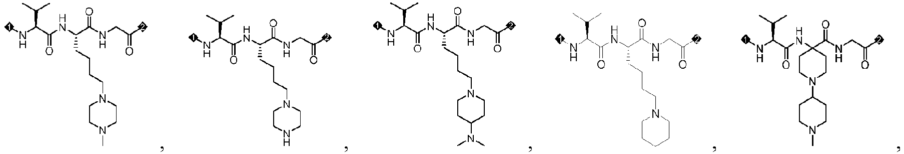 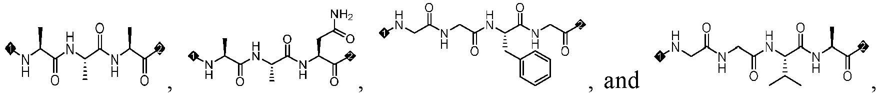 the position 1 is attached to L₁ or L₂, and the position 2 is attached to L₄ or D; and
L₄ is absent or present, when L₄ is present, L₄ is selected from 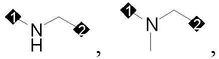 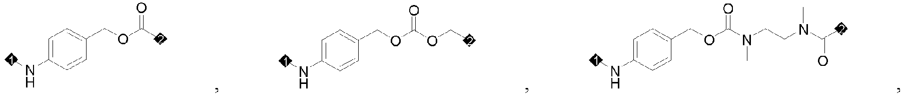 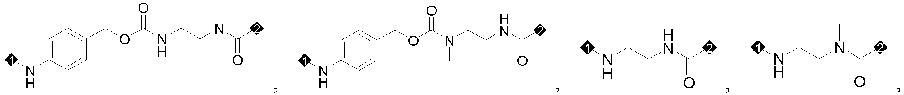 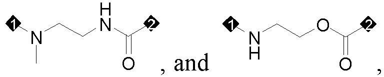 the position 1 is attached to L₃, and the position 2 is attached to D.

19. The antibody-drug conjugate, the stereoisomer thereof, the prodrug thereof, the pharmaceutically acceptable salt thereof, the tautomer thereof, or the pharmaceutically acceptable solvate thereof according to claim 18, wherein,
L₁ is selected from 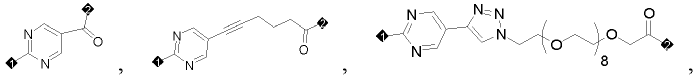 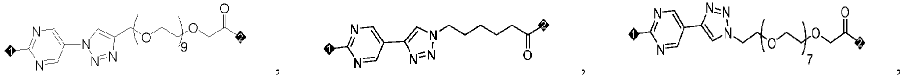 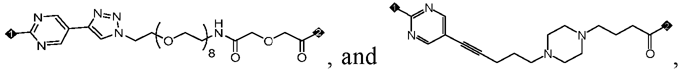 the position 1 is attached to Tb via an S atom, and the position 2 is attached to L₂ or L₃;
preferably, L₁ is selected from 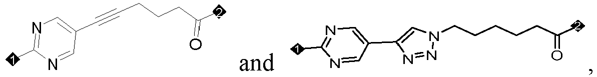 the position 1 is attached to Tb via an S atom, and the position 2 is attached to L₂ or L₃; and
L₂ is absent or present, when L₂ is present, L₂ is selected from 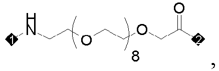 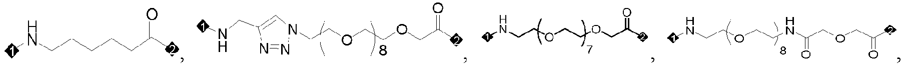 and 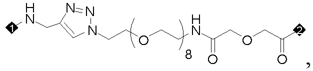 the position 1 is attached to L₁, and the position 2 is attached to L₃; and
preferably, L₂ is absent.

20. The antibody-drug conjugate, the stereoisomer thereof, the prodrug thereof, the pharmaceutically acceptable salt thereof, the tautomer thereof, or the pharmaceutically acceptable solvate thereof according to claim 19, wherein the 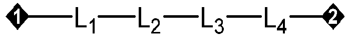 has a structure as follows: 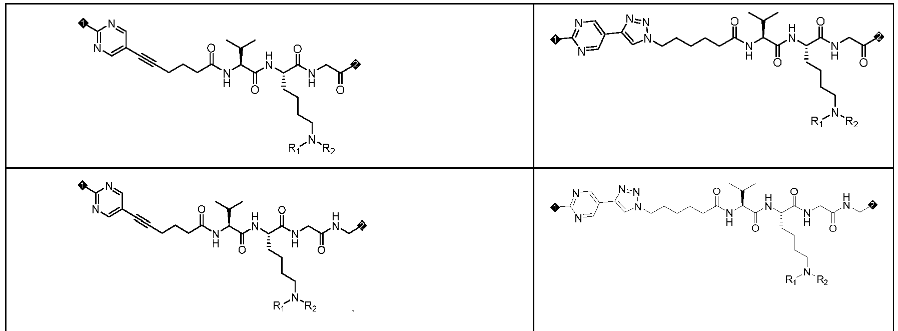 wherein R₁ and R₂ are independently selected from C₁₋₆ alkyl and H; and is preferably C₁₋₄ alkyl; and the position 1 is attached to the antibody or antigen-binding fragment thereof via an S atom, and the position 2 is attached to D.

21. The antibody-drug conjugate, the stereoisomer thereof, the prodrug thereof, the pharmaceutically acceptable salt thereof, the tautomer thereof, or the pharmaceutically acceptable solvate thereof according to any one of claims 17-20, wherein the antibody-drug conjugate is a compound of Formula (IIA-1), Formula (IIA-2), Formula (IIB-1), or Formula (IIB-2): 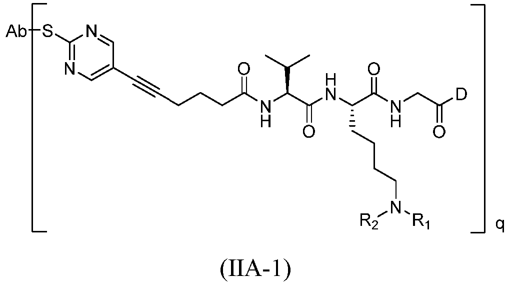 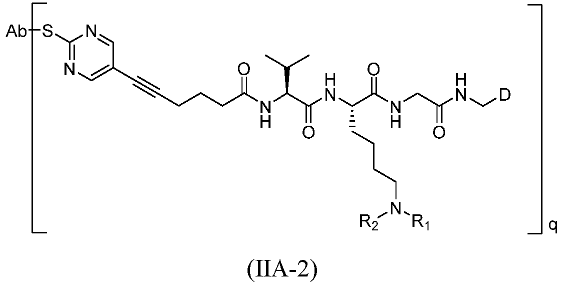 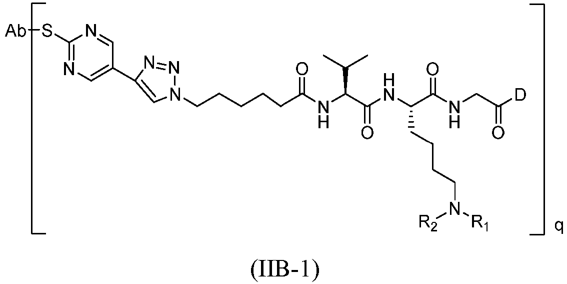 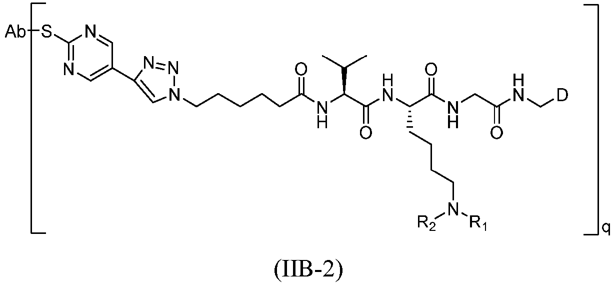 wherein,
Ab is the antibody or antigen-binding fragment thereof according to any one of claims 1-9, or the multispecific antibody according to claim 10;
R₁ and R₂ are independently selected from C₁₋₆ alkyl and H; and is preferably C₁₋₄ alkyl;
D is 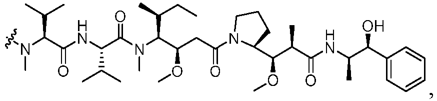 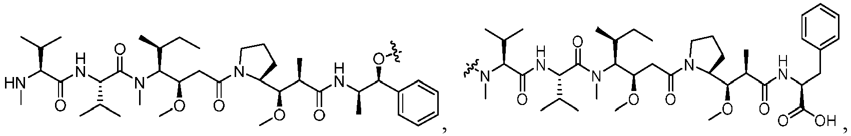 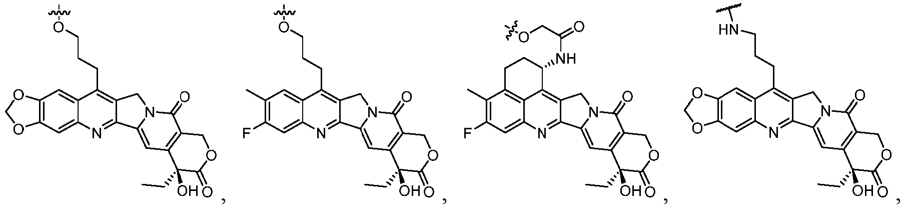 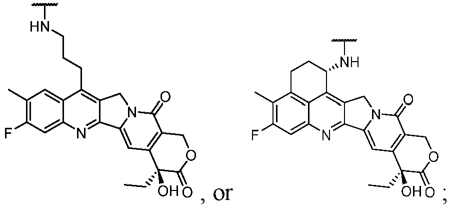 and
q is as defined in claim 16, and is preferably 2, 4, 6, or 8.

22. The antibody-drug conjugate, the stereoisomer thereof, the prodrug thereof, the pharmaceutically acceptable salt thereof, the tautomer thereof, or the pharmaceutically acceptable solvate thereof according to claim 16, wherein the antibody-drug conjugate has a structure as follows: 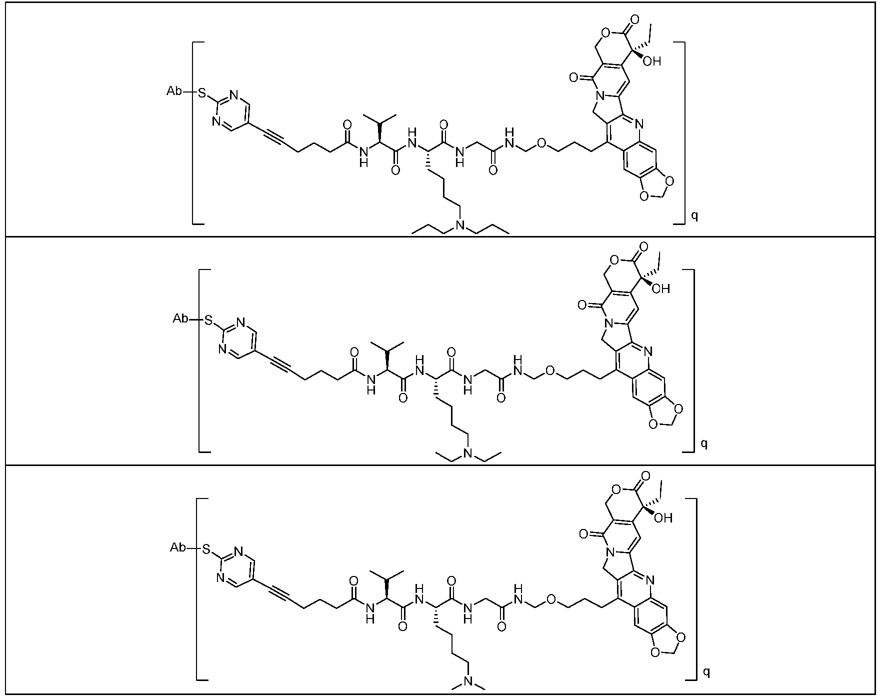 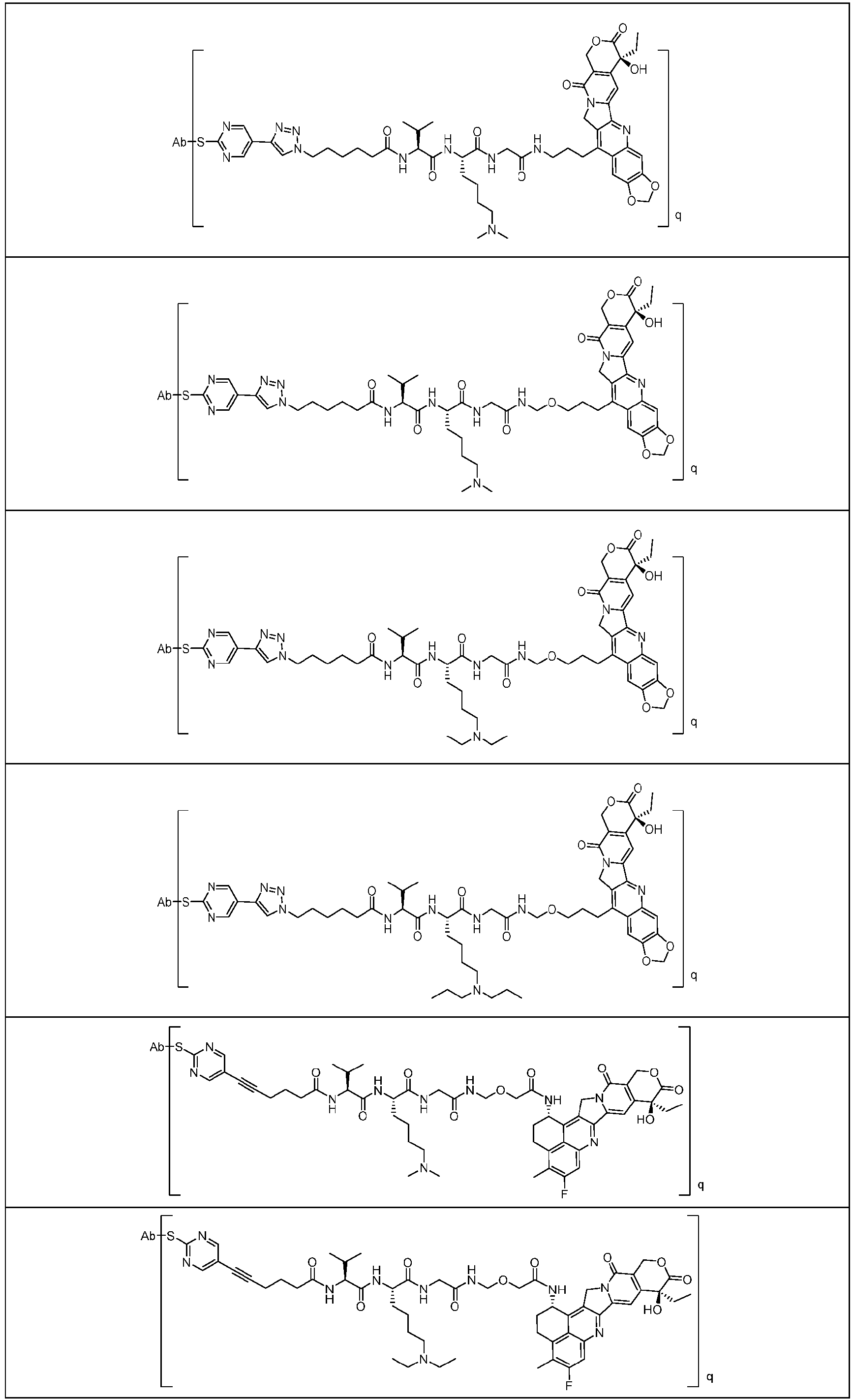 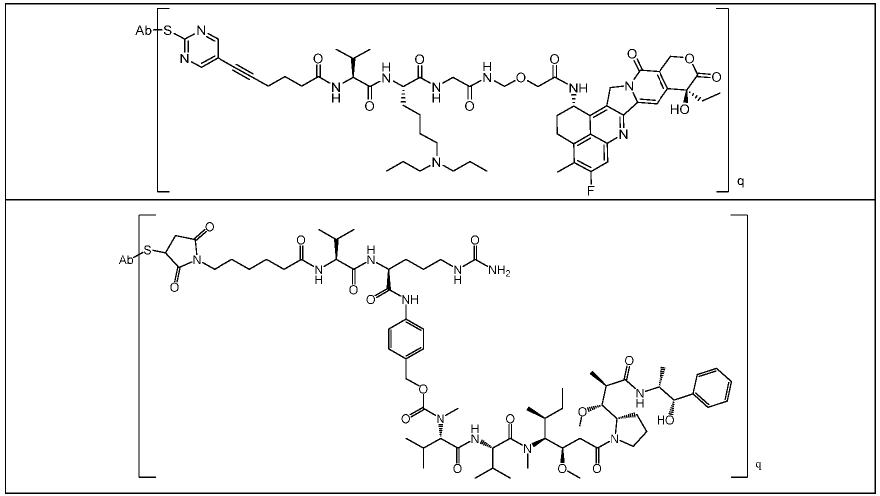 wherein,
q is as defined in claim 16; and is preferably 2, 4, 6 or, 8;
Ab is the antibody or antigen-binding fragment thereof according to any one of claims 1-9, or the multispecific antibody according to claim 10; and
preferably, Ab comprises: a heavy chain set forth in SEQ ID NO: 56 and a light chain set forth in SEQ ID NO: 57, or a heavy chain set forth in SEQ ID NO: 58 and a light chain set forth in SEQ ID NO: 59.

23. A population of antibody-drug conjugates, comprising or consisting of the antibody-drug conjugate, the stereoisomer thereof, the prodrug thereof, the pharmaceutically acceptable salt thereof, the tautomer thereof, or the pharmaceutically acceptable solvate thereof according to any one of claims 15-22, wherein the antibody-drug conjugate has one, two, or more q values;
preferably, an average DAR of the population of antibody-drug conjugates is selected from an integer or a decimal from 1 to 16, for example, selected from 1.5 to 2.5, 3.5 to 4.5, 5.5 to 6.5, or 7.5 to 8.5; and
more preferably, the average DAR of the population of antibody-drug conjugates is selected from about 2.0, 4.0, 6.0, or 8.0.

24. A pharmaceutical composition, comprising the antibody or antigen-binding fragment thereof according to any one of claims 1-9, the multispecific antibody according to claim 10, the antibody-drug conjugate, the stereoisomer thereof, the prodrug thereof, the pharmaceutically acceptable salt thereof, the tautomer thereof, or the pharmaceutically acceptable solvate thereof according to any one of claims 15-22, the nucleic acid molecule according to claim 11, the immunoconjugate according to claim 13 or the population of antibody-drug conjugates according to claim 23, and optionally one or more pharmaceutically acceptable excipients.

25. Use of the antibody-drug conjugate, the stereoisomer thereof, the prodrug thereof, the pharmaceutically acceptable salt thereof, the tautomer thereof, or the pharmaceutically acceptable solvate thereof according to any one of claims 15-22, the anti-c-MET antibody or antigen-binding fragment thereof according to any one of claims 1-9, the multispecific antibody according to claim 10, the nucleic acid molecule according to claim 11, the immunoconjugate according to claim 13 or the population of antibody-drug conjugates according to claim 23, or the pharmaceutical composition according to claim 24, in the preparation of a medicine for treatment or prophylaxis of a disease related to c-MET activity;
preferably, the disease related to the c-MET activity is a tumor related to the c-MET activity; and
preferably, the tumor is selected from: lung cancer (for example, non-small cell lung cancer, small cell lung cancer, or lung adenocarcinoma), colon cancer (for example, human colon adenocarcinoma), rectal cancer, gastric cancer, colorectal cancer (for example, colorectal adenocarcinoma).
